(19) Europäisches Patentamt
European Patent Office
Office européen des brevets

(11) Veröffentlichungsnummer: **0 292 808 B1**

(12) # EUROPÄISCHE PATENTSCHRIFT

(45) Veröffentlichungstag der Patentschrift: **02.03.94**

(51) Int. Cl.$^5$: **C07D 501/20**, A61K 31/545, A23K 1/17

(21) Anmeldenummer: **88107686.3**

(22) Anmeldetag: **13.05.88**

Die Akte enthält technische Angaben, die nach dem Eingang der Anmeldung eingereicht wurden und die nicht in dieser Patentschrift enthalten sind.

(54) **Substituierte Vinylcephalosporine, Verfahren zu ihrer Herstellung und ihre Verwendung als Arzneimittel.**

(30) Priorität: **26.05.87 DE 3717664**
**08.10.87 DE 3734004**

(43) Veröffentlichungstag der Anmeldung:
**30.11.88 Patentblatt 88/48**

(45) Bekanntmachung des Hinweises auf die Patenterteilung:
**02.03.94 Patentblatt 94/09**

(84) Benannte Vertragsstaaten:
**AT BE CH DE ES FR GB GR IT LI NL SE**

(56) Entgegenhaltungen:
**EP-A- 0 154 354**
**EP-A- 0 195 947**
**FR-A- 2 081 451**
**FR-A- 2 540 117**
**US-A- 4 255 423**

(73) Patentinhaber: **BAYER AG**

**D-51368 Leverkusen(DE)**

(72) Erfinder: **Schmidt, Gunter, Dr.**
**Pahlkestrasse 63**
**D-5600 Wuppertal 1(DE)**
Erfinder: **Metzger, Karl Georg, Dr.**
**Pahlkestrasse 75**
**D-5600 Wuppertal 1(DE)**
Erfinder: **Zeiler, Hans-Joachim, Dr.**
**Am Rohm 86**
**D-5600 Wuppertal 1(DE)**
Erfinder: **Endermann, Rainer, Dr.**
**In den Birken 152 a**
**D-5600 Wuppertal 1(DE)**
Erfinder: **Haller, Ingo, Dr.**
**Dornroeschenweg 4**
**D-5600 Wuppertal 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Es ist bekannt, daß verschiedene Vertreter der 7-α-Aminoacylcephalosporine mit unterschiedlichen Substituenten in der 3-Stellung des Moleküls, so z.B. Cephalexin [7-(D-α-Phenylglycylamido)-3-methyl-3-cephem-4-carbonsäure, vgl. DE-OS 24 32 485], Cefaclor [7-(D-α-Phenylglycylamido)-3-chlor-3-cephem-4-carbonsäure, vgl. DE-OS 24 08 698 und 27 28 578] und Cefadroxil [7-(D-α-p-Hydroxyphenylglycylamido)-3-methyl-3-cephem-4-carbonsäure vgl. DE-OS 27 18 741] gute antibiotische Wirksamkeit haben.

Weiterhin sind 3-Alkenyl-substituierte Cephalosporine als oral wirksame Verbindungen in DE-OS 34 02 642 den US-Patenten 4 619 925 und 4 255 423 sowie in EP-A-154 354 beschrieben.

3-Alkenyl-substituierte β-Lactam-Antibiotika mit verschiedenen benzokondensierten Substituenten in 7-Stellung werden auch in EP-A-0 195 947 beschrieben.

Die Erfindung betrifft nun β-Lactam-Verbindungen der allgemeinen Formel (I)

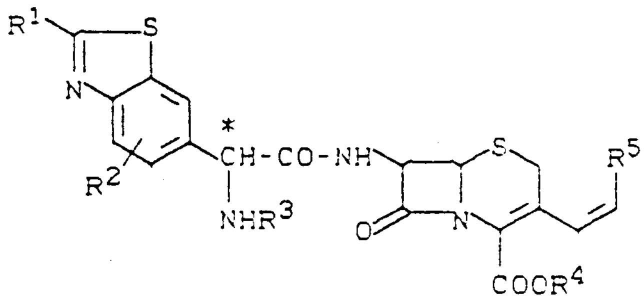

(I),

in welcher

$R^1$

- für Wasserstoff steht, oder
- für geradkettiges, verzweigtes oder cyclisches Alkyl oder Alkenyl mit bis zu 10 Kohlenstoffatomen steht, das substituiert sein kann durch Halogen, Hydroxy, Alkoxy mit bis zu 6 Kohlenstoffotomen, Cyano, Sulfo, Alkylsulfonyl mit bis zu 6 Kohlenstoffatomen, oder durch eine Gruppe der Formel $-NR^6R^7$,

worin

$R^6$, $R^7$

- gleich oder verschieden sind und
- Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, oder eine Aminoschutzgruppe bedeuten,
  oder
- für Aryl mit 6 bis 12 Kohlenstoffatomen steht, das substituiert sein kann durch Halogen, Cyano, Nitro, Alkyl, Alkoxy, Alkylthio mit jeweils bis zu 6 Kohlenstoffatomen, Trifluormethyl oder Trifluormethoxy, oder
- für Halogen, Alkoxy, Alkylthio, Alkylsulfonyl mit jeweils bis zu 8 Kohlenstoffatomen, Mercapto, Hydroxy, Phenylthio, Phenyloxy, Benzylthio, Benzyloxy, Sulfo, Sulfamoyl, $-PO(OH)_2$, $-NHNH_2$, -NHOH, Guanidino, Amidino, Heterocyclyl, Heterocyclylthio oder Heterocyclyloxy mit Heterocyclen aus der Reihe Pyrrolyl, Pyrrolidinyl, Pyrazolyl, Imidozolyl, Pyridyl, Pyridazinyl, Pyrimidyl, Pyrazinyl, Chinolyl, Isochinolyl, Indolyl, Chinoxazolyl, Chinazolyl, Piperidinyl, Morpholinyl, Piperazinyl, Thiomorpholinyl, Furyl, Thienyl, Oxazolyl, Thiazolyl, Isoxazolyl, Thiadiazolyl, Triazolyl oder Tetrazolyl, die wiederum substituiert sein können durch Methyl, Methoxy oder Halogen,
- oder für eine Gruppe der Formel $-NR^6R^7$ steht,

worin

$R^6$, $R^7$

- die oben angegebene Bedeutung haben,

$R^2$

- für Wasserstoff, Alkyl, Alkoxy, Alkylthio mit jeweils bis zu 8 Kohlenstoffatomen, Trifluormethyl, Trifluormethoxy, Hydroxy, Mercapto, Nitro, Cyano, Halogen oder Amino steht,

$R^3$

- für Wasserstoff oder
- für eine in der β-Lactamchemie übliche Aminoschutzgruppe steht,

$R^4$

- für Wasserstoff,
- für eine in der β-Lactamchemie übliche Carboxyschutzgruppe oder
- für einen in vivo spaltbaren Ester steht

und

$R^5$

- für Halogen, Cyano, Trifluormethyl, Carboxy, Alkoxycarbonyl mit bis zu 8 Kohlenstoffatomen, Alkylsulfonyl, Alkylsulfonyloxy mit jeweils bis zu 8 Kohlenstoffatomen, Phenylsulfonyl, Tolylsulfonyl, Phenylsulfonyloxy, Tolylsulfonyloxy, Sulfamoyl, Dialkylsulfamoyl mit jeweils bis zu 6 Kohlenstoffatomen je Alkylgruppe, oder
- für Cycloalkyl mit 3 bis 8 Kohlenstoffatomen steht, das substituiert sein kann durch Alkyl, Alkoxy, Alkoxycarbonyl mit jeweils bis zu 6 Kohlenstoffatomen, Carboxy, Carbamoyl, Halogen, Cyano oder Phenyl, oder
- für Cycloalkylalkyl mit 3 bis 6 Ringgliedern und 4 bis 10 Kohlenstoffatomen, oder
- für geradkettiges oder verzweigtes Alkinyl mit bis zu 8 Kohlenstoffatomen steht, das substituiert sein kann, durch Aryl mit 6 bis 12 Kohlenstoffatomen, Carboxy, Alkoxycarbonyl mit bis zu 8 Kohlenstoffatomen, oder durch Halogen, oder
- für Aryl mit 6 bis 12 Kohlenstoffatomen steht, das substituiert sein kann durch Halogen, Cyano, Nitro, Trifluormethyl, Trifluormethoxy, Trifluormethylthio, Alkyl, Alkoxy oder Alkylthio mit jeweils bis zu 6 Kohlenstoffatomen, oder
- für einen heterocyclischen Rest der Reihe Thienyl, Furyl, Pyridyl, Pyridyloxid, Pyrimidyl, Pyrazinyl, Pyridazinyl, Pyrazolyl, Imidazolyl, Triazolyl, Tetrazolyl, Oxazolyl, Thiazolyl oder Thiadiazolyl steht, wobei die heterocyclischen Reste durch Alkyl, Alkoxy mit jeweils bis zu 6 Kohlenstoffatomen, Halogen, Nitro oder Cyano substituiert sein können, oder
- für eine Gruppe der Formel $-NR^8R^9$, $-CH_2-R^{10}$ oder $-S-R^{11}$ steht,

worin

$R^8$, $R^9$

- gleich oder verschieden sind und
- Wasserstoff, Alkyl mit bis zu 6 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen oder eine Aminoschutzgruppe bedeuten, oder worin

$R^8$, $R^9$

- gemeinsam mit dem Stickstoffatom einen 5 bis 7-gliedrigen Ring bilden, der durch O, S, NH, N-Alkyl mit bis zu 4 Kohlenstoffatomen oder N-Phenyl unterbrochen sein kann,

$R^{10}$

- einen heterocyclischen Rest der Reihe Thienyl, Furyl, Pyridyl, Pyridyloxid, Pyrimidyl, Pyrazinyl, Pyridazinyl, Pyrazolyl, Imidazolyl, Triazolyl, Tetrazolyl, Oxazolyl, Thiazolyl oder Thiadiazolyl bedeutet, wobei die heterocyclischen Reste durch Alkyl, Alkoxy, Alkylthio mit jeweils bis zu 6 Kohlenstoffatomen, Halogen, Nitro, Cyano, Trifluormethyl, Trifluormethoxy substituiert sein können,

und

$R^{11}$

- einen heterocyclischen Rest der Reihe Thienyl, Furyl, Pyridyl, Pyridyloxid, Pyrimidyl, Imidazolyl, Triazolyl, Oxazolyl oder Thiadiazolyl bedeutet, wobei die heterocyclischen Reste durch Alkyl, Alkoxy mit jeweils bis zu 6 Kohlenstoffatomen, Halogen, Trifluormethyl, Trifluormethoxy, Nitro oder Cyano substituiert sein können,

und deren Salze.

Aminoschutzgruppe im Rahmen der oben angegebenen Definition steht im allgemeinen für eine in der β-Lactam-Chemie üblichen Schutzgruppe aus der Reihe: 4-Methoxyphenyl, 4-Methoxymethyloxyphenyl, 4-[-(2-Methoxyethoxy)methyloxy]phenyl, 3,4-Dimethoxyphenyl, Benzyl, 2-Nitrobenzyl, 4-Nitrobenzyl, 4-Methoxybenzyl, 2,4-Dimethoxybenzyl, 3,4-Dimethoxybenzyl, 2,4,6-Trimethoxybenzyl, tert-Butoxycarbonyl, Benzyloxycarbonyl, 2-Nitrobenzyloxycarbonyl,4-Nitrobenzyloxycarbonyl, Formyl, Acetyl, Chloracetyl, Trichloracetyl, Trifluoracetyl, Benzoyl, Methoxycarbonyl, Allyloxycarbonyl, 2,4-Dimethoxybenzyloxycarbonyl, Methoxycarbonylmethyl, tert-Butoxycarbonylmethyl, Allyloxymethyl, Bis-(4-methoxyphenyl)methyl, Methoxymethyl, Methylthiomethyl, Methoxyethoxymethyl, 2-(Methylthiomethoxy)ethoxycarbonyl, 2-Hydroxy-2-phenylmethyl, Methoxy-(4-methoxyphenyl)methyl, Trimethyl-, Triethyl-, Triphenylsilyl, tert-Butyl-dimethylsilyl, tert-Butyl-diphenylsilyl, [2-(Trimethylsilyl)ethoxy]methyl, 1-Methyl-2-benzyoyl-vinyl, 1-Methyl-2-ethoxy- oder -2-methoxyvinyl, Mesyl und Ethylsulfonyl.

Carboxyschutzgruppe im Rahmen der oben angegebenen Definition steht für die in der $\beta$-Lactam-Chemie üblichen Carboxyschutzgruppe. Bevorzugt sind leicht abspaltbare Gruppen zu nennen wie zum Beispiel: Methyl, Ethyl, tert.Butyl, Decyl, 2-Chlorethyl, 2,2,2-Trichlorethyl, Cyanoethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl.

Steht $R^4$ für einen in vivo leicht abspaltbaren Esterrest so sind hiermit pharmazeutisch verträgliche Esterreste gemeint, die in vivo leicht zu freien Carboxylgruppen ($R^4$ = H) hydrolysiert werden.

Solche Esterreste sind auf dem $\beta$-Lactam-Gebiet gut bekannt. In den meisten Fällen bessern sie die Absorptionseigenschaften der $\beta$-Lactam-Verbindungen. Außerdem sollte der Rest $R^4$ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 350 230 und DE-OS 2 228 255. Bevorzugte in vivo abspaltbare Estergruppen sind die der folgenden Formeln:

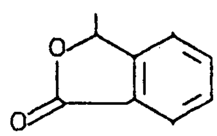 , 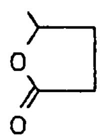 , 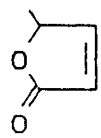 ,

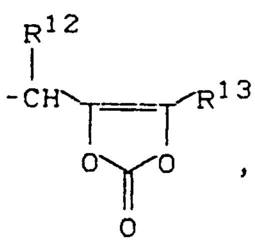 , 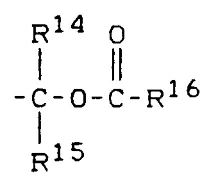 oder

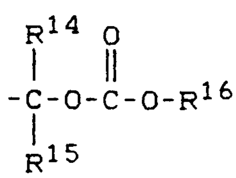

worin

$R^{12}$ und $R^{13}$ gleich oder verschieden sind und
- für Wasserstoff, Phenyl oder
- für $C_1$-$C_4$-Alkyl, bevorzugt für Methyl stehen,

$R^{14}$ und $R^{15}$ gleich oder verschieden sind und
- für Wasserstoff oder
- für $C_1$-$C_4$-Alkyl, bevorzugt Methyl stehen

und

$R^{16}$
- für $C_1$-$C_6$-Alkyl, bevorzugt für $C_1$-$C_4$-Alkyl steht.

Die erfindungsgemäßen Verbindungen der allgemeinen Formel (I) können als freie Säuren, als Ester, als innere Salze

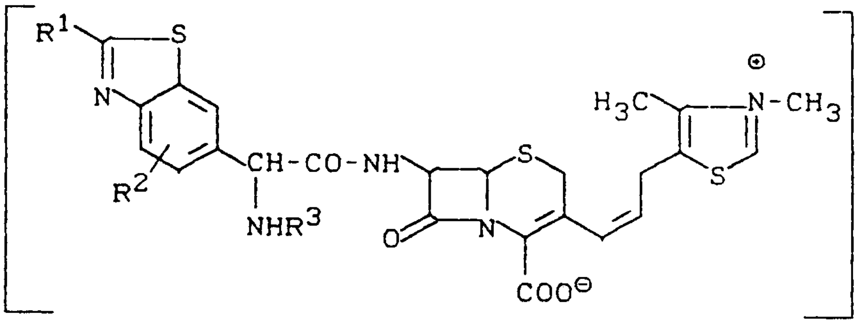

Beispiel für inneres Salz

oder als nicht-toxische, physiologisch verträgliche Salze mit einem Gegenkation

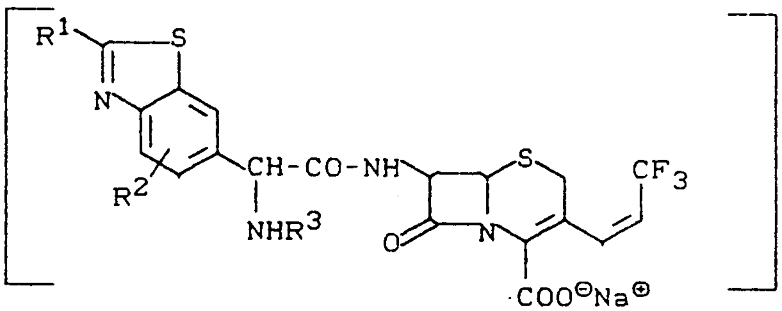

Beispiel für Salz mit Gegenkation

oder wenn $R^4$ ein positiv geladener Rest ist als nichttoxisch, physiologisch verträgliche Salze mit einem Gegenanion

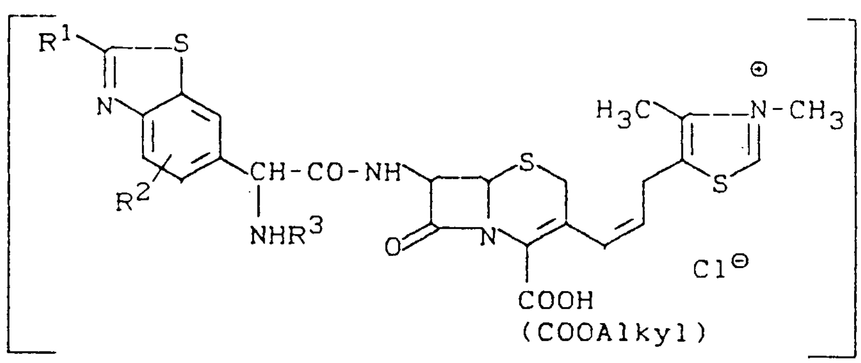

Beispiel Salz mit Gegenanion

vorliegen.

Als Gegenkationen sind bevorzugt Alkali- oder Erdalkalikationen wie beispielsweise Natrium-, Kalium-, Magnesium- oder Calciumionen zu nennen, oder Aluminium- oder Ammoniumionen, sowie nicht-toxische substituierte Ammoniumionen aus Aminen wie Diniedrigalkylamine, Triniedrigalkylamine, Prokain, Dibenzylamin, N,N'-Dibenzylethylendiamin, N-Benzyl-$\beta$-phenyl-ethylamin, N-Methylmorpholin, 1-Ephenamin, Dihydroabietylamin, N,N'-Bisdihydroabietylethylendiamin, N-Niedrigalkylpiperidine oder andere Amine, die zur Bildung von Salzen von $\beta$-Lactamverbindungen verwendet werden können.

Als Gegenanionen sind bevorzugt anorganische oder organische Säurereste zu nennen wie beispielsweise Chlorid, Bromid, Iodid, Sulfat, Hydrogensulfat, Phosphat, Hydrogenphosphat, Carbonat, Hydrogencarbonat, oder Sulfonate wie Methansulfonat, Ethansulfonat, Toluolsulfonat, Benzolsulfonat, Naphthalindisulfonat, oder Carboxylate wie Acetat, Formiat, Oxalat, Tartrat, Citrat, Maleat, Fumarat, Benzoat, Succinat, Lactat.

Die Verbindungen der allgemeinen Formel (I) existieren (bzgl. der Doppelbindung) in der Z-(cis)- sowie in der E-(trans)-Konfiguration. Bevorzugt sind die Verbindungen mit Z-(cis)-Konfiguration. Wegen der

Anwesenheit des mit * bezeichneten asymmetrischen Kohlenstoffatoms (siehe Formel I) schließen die erfindungsgemäßen β-Lactamantibiotika der allgemeinen Formel (I) die D-, L- und D,L-Formen ein. Sowohl die Diastereomerengemische als auch die D-Form und L-Form der erfindungsgemäßen Verbindungen können zur Behandlung bakterieller Infektionskrankheiten eingesetzt werden. Besonders bevorzugt sind die D-Formen der erfindungsgemäßen Verbindungen.

Bevorzugt seien Verbindungen der allgemeinen Formel (I) genannt,
in welcher

$R^1$

- für Wasserstoff oder
- für geradkettiges, verzweigtes oder cyclisches Alkyl steht, das substituiert sein kann durch Fluor, Chlor, Brom, Hydroxy, Alkoxy, Cyano, oder durch eine Gruppe der Formel

  $-NR^6R^7$,

  worin

  $R^6$, $R^7$

  - gleich oder verschieden sind und für
  - Wasserstoff, Alkyl mit bis zu 6 Kohlenstoffatomen, Phenyl, Benzyl oder eine Aminoschutzgruppe der Reihe 4-Methoxyphenyl, 3,4-Dimethoxyphenyl, 4-Methoxymethyloxyphenyl, 4-[(2-Methoxyethoxy)methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyl-dimethylsilyl, tert-Butyl-diphenylsilyl, Methoxycarbonylmethyl, tert-Butoxycarbonylmethyl oder 2-Hydroxy-2-phenylethyl steht, oder
- für gegebenenfalls durch Fluor, Chlor, Brom, Alkyl, Alkoxy mit bis zu 6 Kohlenstoffatomen, Trifluormethyl, Trifluormethoxy oder Cyano substituiertes Phenyl steht, oder
- für Fluor, Chlor, Brom, Alkoxy, Alkylthio, Alkylsulfonyl mit jeweils bis zu 6 Kohlenstoffatomen, Mercapto, Hydroxy, Phenylthio, Phenyloxy, Sulfamoyl oder
- für eine Gruppe der Formel $-NR^6R^7$ steht,

  worin

  $R^6$, $R^7$

  - die oben angegebene Bedeutung haben,

$R^2$

- für Wasserstoff, Methyl, Methoxy, Trifluormethyl, Trifluormethoxy, Cyano, Fluor, Chlor oder Brom steht,

$R^3$

- für Wasserstoff oder
- für eine Aminoschutzgruppe aus der Reihe: 4-Methoxyphenyl, 3,4-Dimethoxyphenyl, 4-Methoxymethyloxyphenyl, 4-[(2-Methoxyethoxy)methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyl-dimethylsilyl, tert-Butyl-diphenylsilyl, Methoxycarbonylmethyl, tert-Butoxycarbonylmethyl, 2-Hydroxy-2-phenylethyl, tert-Butyloxycarbonyl, 1-Methyl-2-benzoyl-vinyl oder 1-Methyl-2-methoxy-vinyl steht,

$R^4$

- für Wasserstoff steht, oder
- für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht, oder

- für einen Rest der Formel

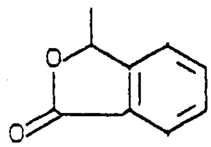 , 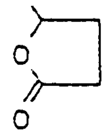 , 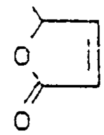 ,

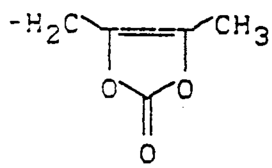 ,

$-CH_2-OCO-C(CH_3)_3$, $-CH(CH_3)-OCOOC_2H_5$ oder $-CH_2-OCOCH_3$ steht,

und

$R^5$

- für Fluor, Chlor, Brom, Cyano, Trifluormethyl, Carboxy, Alkoxycarbonyl mit bis zu 6 Kohlenstoffatomen, Alkylsulfonyl, Alkylsulfonyloxy mit jeweils bis zu 6 Kohlenstoffatomen, Phenylsulfonyl, Tolylsulfonyl, Phenylsulfonyloxy, Tolylsulfonyloxy, oder
- für Cyclopropyl, Cyclopentyl oder Cyclohexyl steht, die substituiert sein können durch Alkyl, Alkoxy, Alkoxycarbonyl mit jeweils bis zu 4 Kohlenstoffatomen, Fluor, Chlor, Brom oder Phenyl, oder
- für Cyclopropylmethyl, Cyclopentylmethyl oder Cyclohexylmethyl steht, oder
- für geradkettiges oder verzweigtes Alkinyl mit bis zu 6 Kohlenstoffatomen steht, das substituiert sein kann durch Phenyl, Carboxy, Alkoxycarbonyl mit bis zu 6 Kohlenstoffatomen oder durch Fluor, Chlor, Brom, oder
- für Phenyl steht, das substituiert sein kann durch Fluor, Chlor, Brom, Cyano, Nitro, Trifluormethyl, Trifluormethoxy, Alkyl oder Alkoxy mit bis zu 4 Kohlenstoffatomen, oder
- für einen heterocyclischen Rest der Reihe Thienyl, Furyl, Pyridyl, Pyridyloxid, Pyrimidyl, Pyrazolyl, Triazolyl, Tetrazolyl, Oxazolyl, Thiazolyl oder Thiadiazolyl steht, wobei die heterocyclischen Reste durch Alkyl, Alkoxy mit jeweils bis zu 4 Kohlenstoffatomen, Fluor, Chlor, Brom oder Nitro substituiert sein können, oder
- für eine Gruppe der Formel $-NR^8R^9$, $-CH_2-R^{10}$ oder $-S-R^{11}$ steht,

worin

$R^8$, $R^9$

- gleich oder verschieden sind und
- Wasserstoff, Alkyl mit bis zu 4 Kohlenstoffatomen, Phenyl, Benzyl oder eine Aminoschutzgruppe der Reihe 4-Methoxyphenyl, 3,4-Dimethoxyphenyl, 4-Methoxymethyloxyphenyl, 4-[(2-Methoxyethoxy)methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyldimethylsilyl, tert-Butyl-diphenylsilyl, Methoxycarbonylmethyl, tert-Butoxycarbonylmethyl oder 2-Hydroxy-2-phenylethyl bedeuten, oder worin

$R^8$, $R^9$

- gemeinsam mit dem Stickstoffatomen einen Pyrrolidin-, Piperidin-, Morpholin-, Thiomorpholin-, Piperazin-, 4-Methyl- oder 4-Phenylpiperazinring bilden,

$R^{10}$

- einen heterocyclischen Ring der Reihe Thienyl, Furyl, Pyridyl, Pyridyloxid, Pyrimidyl, Pyrazolyl, Triazolyl, Tetrazolyl, Oxazolyl, Thiazolyl oder Thiadiazolyl bedeutet, wobei die heterocyclischen Reste durch Alkyl, Alkoxy mit jeweils bis zu 4 Kohlenstoffatomen, Fluor, Chlor, Brom oder Nitro substituiert sein können,

und

$R^{11}$

- einen heterocyclischen Ring der Reihe Thienyl, Furyl, Pyridyl, Pyridyloxid, Pyrimidyl, Pyrazolyl, Oxazolyl oder Thiadiazolyl bedeutet, wobei die heterocyclischen Reste durch Alkyl, Alkoxy mit jeweils bis zu 4 Kohlenstoffatomen, Fluor, Chlor, Brom oder Nitro substituiert sein kann

und deren Salze.

Besonders bevorzugt seien Verbindungen der allgemeinen Formel (I) genannt, in welcher

$R^1$

- für Wasserstoff oder für Cyclopropyl steht, oder
- für geradkettiges oder verzweigtes Alkyl mit bis zu 6 Kohlenstoffatomen steht, das durch Fluor, Chlor, Methoxy, Cyano, Dimethylamino oder Diethylamino substituiert sein kann, oder
- für Phenyl steht, das durch Fluor, Chlor, Methyl, Methoxy, Trifluormethyl, Amino oder Trifluormethoxy substituiert sein kann, oder
- für Chlor, Alkoxy, Alkylsulfonyl mit jeweils bis zu 4 Kohlenstoffatomen, Mercapto, Hydroxy oder
- für eine Gruppe der Formel $-NHR^6$ steht,

worin

$R^6$

- Wasserstoff, Alkyl mit bis zu 4 Kohlenstoffatomen, Phenyl, Benzyl oder eine Aminoschutzgruppe der Reihe 4-Methoxyphenyl, 4-Methoxybenzyl, 2,4-Dimethoxybenzyl, 3,4-Dimethoxybenzyl oder tert-Butyl-dimethylsilyl bedeutet,

$R^2$

- für Wasserstoff steht,

$R^3$

- für Wasserstoff oder
- für 4-Methoxyphenyl, 4-Methoxybenzyl, 2,4-Dimethoxybenzyl, 3,4-Dimethoxybenzyl, tert-Butyl-dimethylsilyl, 1-Methyl-2-methoxy-vinyl (MMV) oder tert-Buyloxycarbonyl (Boc), steht, oder

$R^4$

- für Wasserstoff steht, oder
- für Methyl, Ethyl, tert.Butyl, Diphenylmethyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
- für einen Rest der Formel

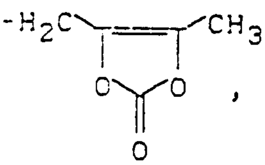

,

$-CH(CH_3)-OCOOC_2H_5$ oder $-CH_2-OCO-C(CH_3)_3$ steht,

und

$R^5$

- für Chlor, Brom, Trifluormethyl, Carboxy, Alkoxycarbonyl, Alkylsulfonyloxy mit jeweils bis zu 4 Kohlenstoffatomen, Phenylsulfonyloxy, Tolylsulfonyloxy, Cyclopropyl, Cyclopentyl, Cyclohexyl, Cyclopropylmethyl, Cyclopentylmethyl steht, oder
- für Alkinyl mit bis zu 4 Kohlenstoffatomen steht, das durch Phenyl, Carboxy oder Alkoxycarbonyl mit bis zu 4 Kohlenstoffatomen substituiert sein kann, oder
- für Phenyl steht, oder

- für einen Rest der Formel

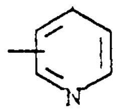 , 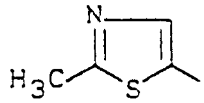 , 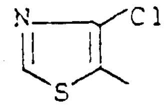 , 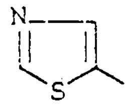 ,

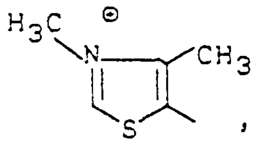 , 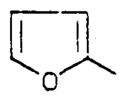 , 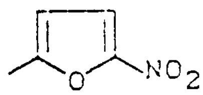 , 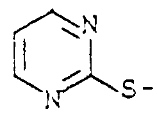 ,

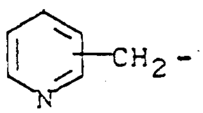 , 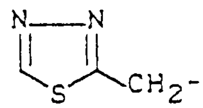 , 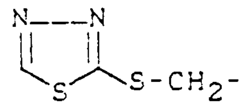 ,

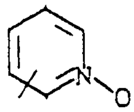 , 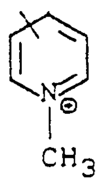 , 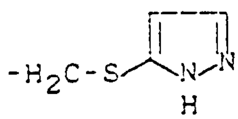

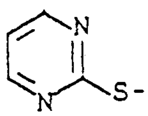 oder 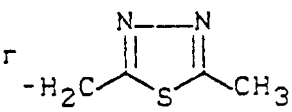

steht,
und deren Salze.

Ganz besonders bevorzugt sind darüber hinaus die in der folgenden Tabelle aufgeführten Verbindungen:

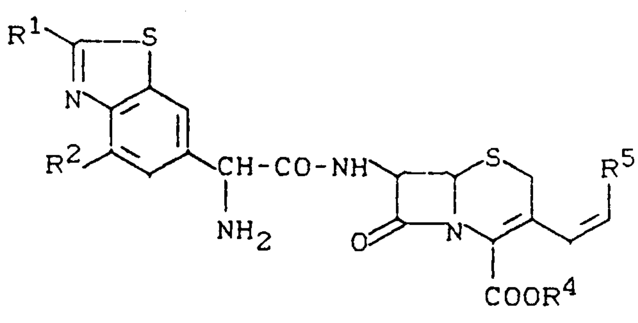

| $R^1$ | $R^2$ | $R^4$ | $R^5$ |
|---|---|---|---|
| $H_2N$ | H | H | 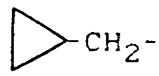 |
| $H_2N$ | H | H | $CH_3-C\equiv C-$ |
| $CH_3$ | H | $CH_2-OCOC(CH_3)_3$ | $CF_3-$ |
| $H_2N-$ | H | H | 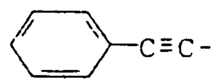 |

| $R^1$ | $R^2$ | $R^4$ | $R^5$ |
|---|---|---|---|
| $H_2N-$ | OH | $CH_2OCOCH_3$ | 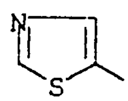 |
| $H_2N-$ | OH | H | $-N(CH_3)_2$ |
| $H_2N-$ | H | H | 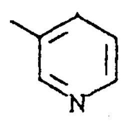 |
| 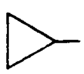 | H | H | $CF_3$ |
| $H_2N$ | H | H | Cl |
| $H_2N$ | H | H | $CH_2Cl$ |
| $H_2N$ | H | H | 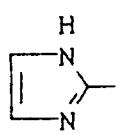 |
| $H_2N$ | H | H | 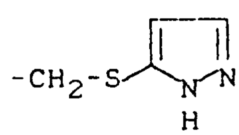 |
| $H_2N$ | $O_2N$ | H | 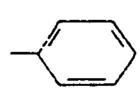 |
| $H_2N$ | HO- | H | 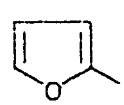 |

Darüber hinaus wurde ein Verfahren zur Herstellung der erfindungemäßen substituierten Vinylcephalosporinverbindungen der allgemeinen Formel (I) gefunden, das dadurch gekennzeichnet ist, daß man

[A] substituierte Cephalosporinverbindungen der allgemeinen Formel (II)

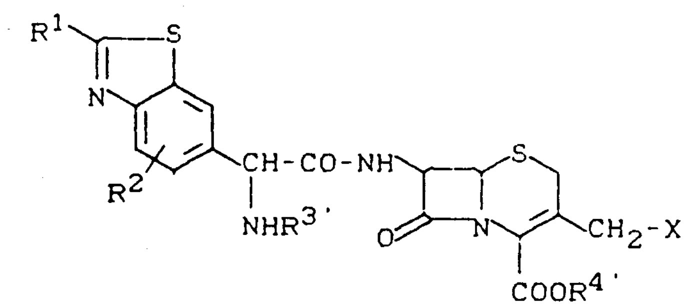

(II)

in welcher

$R^1$ und $R^2$ die oben angegebene Bedeutung hat,

$R^{3'}$ - für eine in der $\beta$-Lactam-Chemie übliche Aminoschutzgruppe steht,

$R^{4'}$ - für eine in der $\beta$-Lactam-Chemie übliche Carboxyschutzgruppe steht,

und

X - für eine Gruppe der Formel

$-\overset{\oplus}{P}(R^{17})_3Z^{\ominus}$ ,

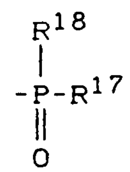

oder

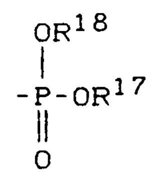

steht,

wobei

$R^{17}$ und $R^{18}$ gleich oder verschieden sind und Alkyl, Phenyl oder Tolyl bedeuten und

$Z^{\ominus}$ - ein Halogenidanion, bevorzugt Chlorid, Bromid oder Iodid bedeutet,

in inerten Lösemitteln in Anwesenheit von Basen, mit Aldenyden der allgemeinen Formel (III)

$R^5$-CHO (III)

in welcher

$R^5$ die oben angegebene Bedeutung hat

umsetzt,

oder daß man

[B] Phosphoniumverbindungen der allgemeinen Formel (IV)

$R^5$-$CH_2$-X (IV)

in welcher

$R^5$ die oben angegebene Bedeutung hat

und

X

- für eine Gruppe der Formel

$\overset{\oplus}{-P}(R^{17})_3Z^{\ominus}$ , 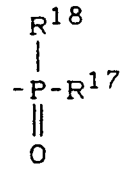 oder 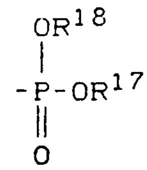

steht,
wobei

$R^{17}$ und $R^{18}$ gleich oder verschieden sind und Alkyl, Phenyl oder Tolyl bedeuten und

$Z^{\ominus}$ - ein Halogenidanion, bevorzugt Chlorid, Bromid oder Iodid bedeutet,

in inerten Lösemitteln in Anwesenheit von Basen, mit Cephalosporinaldehyden der allgemeinen Formel (V)

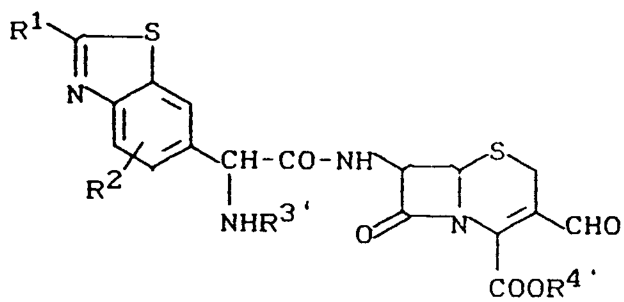

(V)

in welcher

$R^1$ und $R^2$ die oben angegebene Bedeutung haben,

$R^{3'}$ für eine in der $\beta$-Lactam-Chemie übliche Aminoschutzgruppe steht, und

$R^{4'}$ für eine in der $\beta$-Lactam-Chemie übliche Carboxyschutzgruppe steht,

umsetzt,
oder, daß man
[C] Carbonsäuren der allgemeinen Formel (VI)

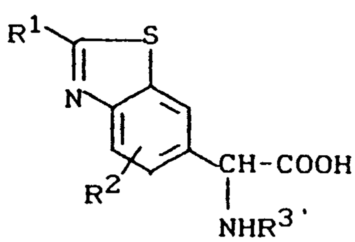

(VI)

in welcher

$R^1$ und $R^2$ die oben angegebene Bedeutung haben, und

$R^{3'}$ für eine in der $\beta$-Lactam-Chemie übliche Aminoschutzgruppe steht,

nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester beispielsweise mit Dicyclohexylcarbodiimid (DCC) gegebenenfalls in Anwesenheit von N-Hydroxybenztriazol,

mit den Vinylcephalosporinaminen der allgemeinen Formel (VII)

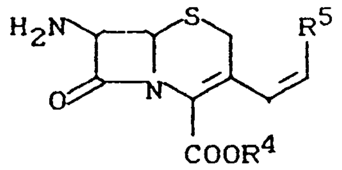

(VII),

in welcher
$R^4$ und $R^5$ die oben angegebene Bedeutung haben,
umsetzt,
dann gegebenenfalls Schutzgruppen abspaltet und die gewünschten Salze oder aus den Salzen die freien Säuren herstellt.

Das erfindungsgemäße Verfahren kann durch das folgende Formelschema erläutert werden:

Verfahrensvariante A:

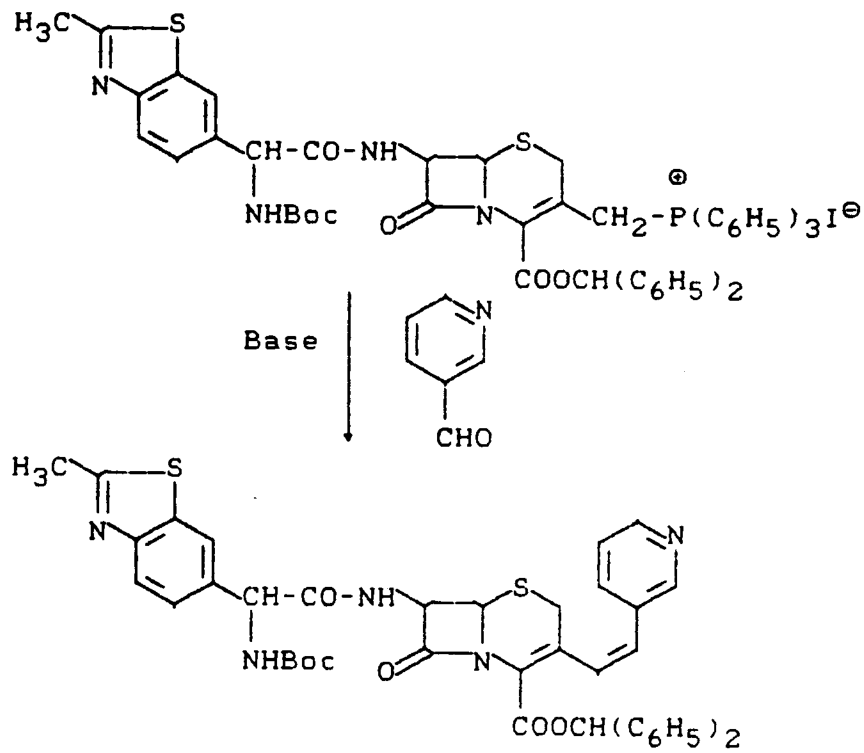


* Boc = $(H_3C)_3C-O-CO-$

Verfahrensvariante B:

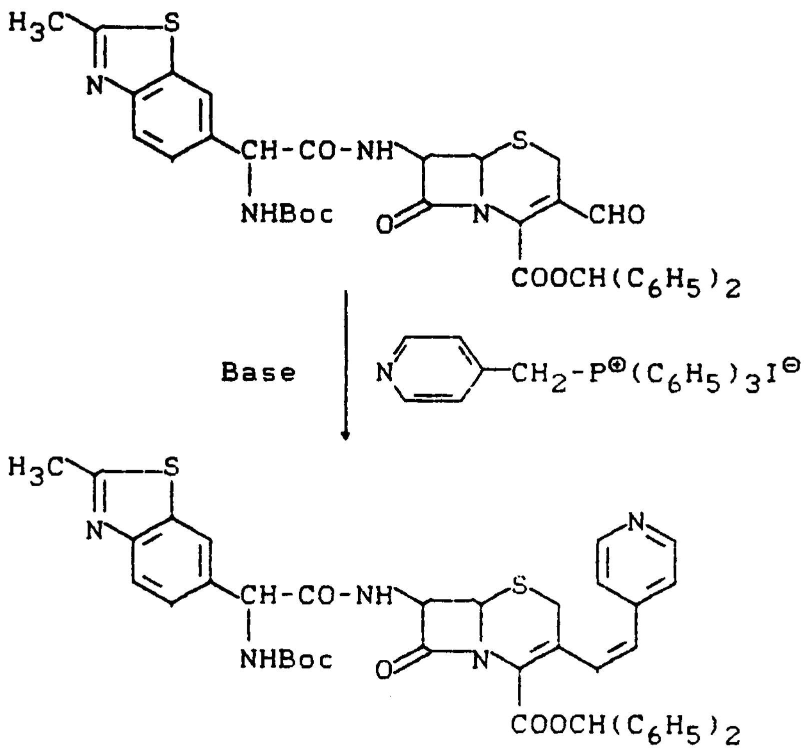


* Boc = $(H_3C)_3C-O-CO-$

Verfahrensvariante C:

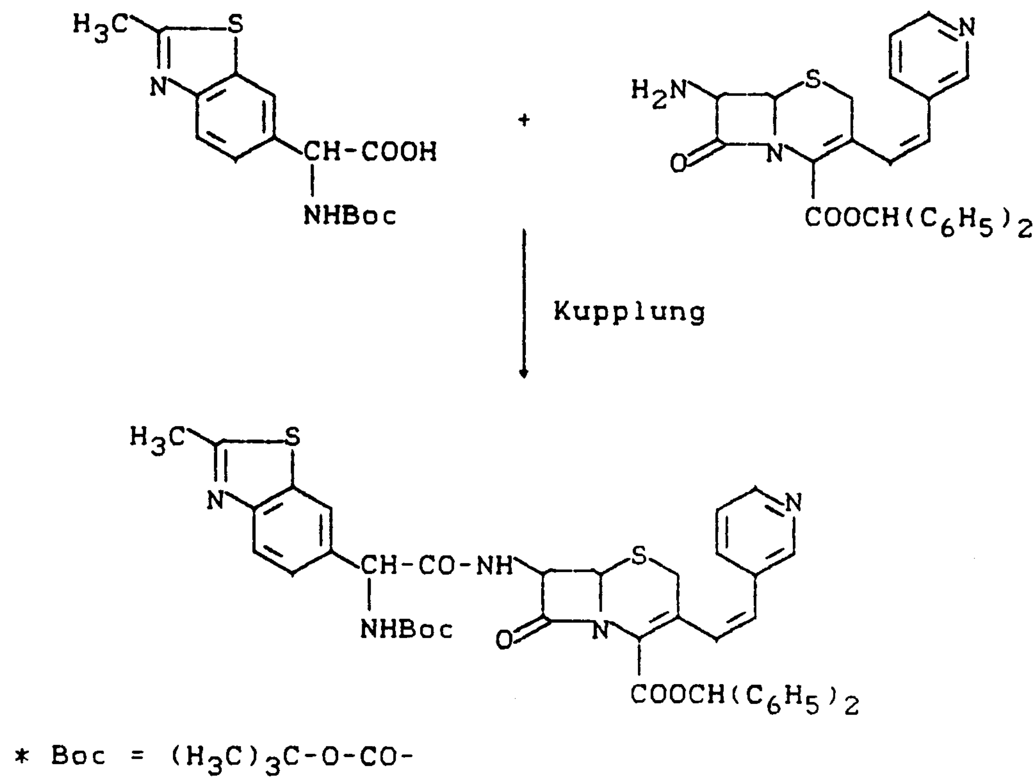

Erläuterungen zu den

Verfahrensvarianten A und B

Als inerte Lösemittel für die Verfahrensvarianten A und B eignen sich die üblichen organischen Lösemittel, die sich unter den Reaktionsbedingungen nicht verändern. Hierzu gehören bevorzugt Ether wie Diethylether, Butylmethylether, Dioxan oder Tetrahydrofuran, oder Kohlenwasserstoffe wie Benzol, Toluol, Xylol oder Cyclohexan, oder Amide wie Dimethylformamid oder Hexamethylphosphorsäuretriamid, oder Alkohole wie Methanol, Ethanol, Propanol oder Isopropanol, oder Chlorkohlenwasserstoffe wie Methylenchlorid, Chloroform oder Tetrachlorkohlenstoff, oder Aceton, Acetonitril oder Essigester. Ebenso ist es möglich, Gemische der genannten Lösemittel zu verwenden.

Als Basen für Verfahrensvarianten A und B eignen sich die üblichen basischen Verbindungen. Hierzu gehören bevorzugt Alkali- oder Erdalkalihydroxide wie beispielsweise Natriumhydroxid, Kaliumhydroxid oder Bariumhydroxid, oder Alkalicarbonate wie Natriumcarbonat, Natriumhydrogencarbonat oder Kaliumcarbonat, oder Alkalialkoholate wie Natriummethanolat, Natriumethanolat, Kaliummethanolat, Kaliumethanolat oder Kalium-tert.butylat.

Die Wahl des Lösemittels bzw. der Base richtet sich nach Stabilität, Hydrolyseempfindlichkeit bzw. CH-Azidität der entsprechenden Phosphorverbindung. Besonders bevorzugt verwendet man als Lösemittel Chlorkohlenwasserstoffe wie beispielsweise Methylenchlorid, Chloroform oder Tetrachlorkohlenstoff in Anwesenheit von Dimethylformamid als Co-Solvens. Als Basen verwendet man besonders bevorzugt Alkalicarbonate wie Natriumcarbonat, Natriumhydrogencarbonat oder Kaliumcarbonat, oder Alkali- oder Erdalkalihydroxide wie beispielsweise Natriumhydroxid, Kaliumhydroxid oder Bariumhydroxid, besonders bevorzugt in Form ihrer wäßrigen Lösungen.

Die Umsetzung wird im allgemeinen in einem Temperaturbereich von -30 °C bis +80 °C, bevorzugt von 0 °C bis +30 °C durchgeführt.

Die Umsetzung kann bei normalen, erhöhtem oder bei erniedrigtem Druck durchgeführt werden (z.B. in einem Bereich von 0,5 bis 5 bar). Im allgemeinen arbeitet man bei Normaldruck.

Bei der Durchführung der Verfahrensvarianten A und B werden im allgemeinen die Phosphorverbindungen (II) bzw. (IV) in einer Menge von 1 bis 3 mol, bevorzugt in molaren Mengen, bezogen auf 1 mol der

Aldehyde (III) bzw. (V) eingesetzt. Die Basen werden im allgemeinen in einer Menge von 1 bis 5 mol, bevorzugt von 1 bis 2 mol, bezogen auf 1 mol der Phsophorverbindungen eingesetzt.

Besonders bevorzugt werden die Verfahrensvarianten A und B als Wittig-Reaktion durchgeführt. Bei der Durchführung des erfindungsgemäßen Verfahrens ist es ebenso möglich, anstelle der Phosphoniumsalze [X = $-P(R^{17})_3^{\oplus}Z^{\ominus}$] die entsprechenden Phosphorane

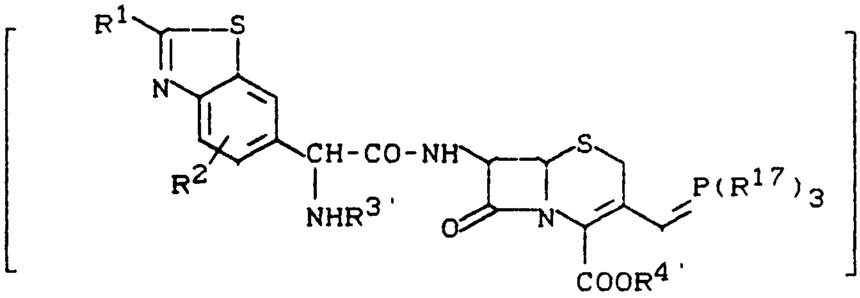

direkt einzusetzten, die man zuvor aus den entsprechenden Phosphoniumsalzen und Base in einer gesonderten Umsetzung hergestellt hat. Es hat sich jedoch als günstig erwiesen, die Umsetzung mit den Triphenylphosphoniumsalzen ( X = $P^{\oplus}(C_6H_5)_3Z^{\ominus}$) in Anwesenheit von Basen als Eintopfverfahren durchzuführen. Als besondere Variante eines Eintopfverfahrens kann die Umsetzung je nach der Stabilität der Phosphorverbindungen auch in Form einer phasentransferkatalysierten Reaktion durchgeführt werden, wobei als Lösemittel Ether, Kohlenwasserstoffe sowie Halogenkohlenwasserstoffe verwendet werden und als Basen wäßrige Natriumhydroxid-oder Kaliumhydroxidlösungen eingesetzt werden.

Wird die Reaktion alternativ derart durchgeführt, daß das entsprechende Phosphoran als Zwischenverbindung isoliert wird und in einem zweiten Schritt mit dem Aldehyd umgesetzt wird, wurde darüberhinaus gefunden, daß durch Zugabe eines geeigneten Lithiumhalogenids wie beispielsweise Lithiumchlorid, Lithiumbromid oder Lithiumiodid die Ausbeute und das Verhältnis von Z-/E-Isomer der Endprodukte der allgemeinen Formel (I) verbessert wird. Die Umsetzung wird hierbei vorzugsweise mit 10 bis 15 Äquivalenten Lithiumhalogenid durchgeführt.

Besonders bevorzugt ist jedoch die Durchführung der Verfahrensvarianten A und B als Eintopfreaktion ohne Isolierung des Zwischenproduktes. Die erfindungsgemäßen Verfahrensvarianten können beispielsweise durchgeführt werden, indem man zu den Phosphoniumverbindungen, in einem geeigneten Lösemittel gelöst oder suspendiert, die Base und dann einen entsprechenden Aldehyd, gegebenfalls in einem geeigneten Lösemittel, zugibt und gegebenenfalls erwärmt. Die Aufarbeitung erfolgt in üblicher Art und Weise durch Extraktion, Chromatographie und/oder Kristallisation.

Weitere spezielle Verfahrenvarianten für die Wittig-Reaktion sind u.a. an den folgenden Stellen beschrieben: J. Fuhrhop und G. Penzlin: Organis Synthesis, Verlag Chemie, 1983, Seite 26 - 35; R.K. Mackie und D.M. Smith: Guidebook to Organic Synthesis, Longman Group Limited, 1982, Seite 93 - 99; H.O. House: Stereochemistry of the Wittig-Reaction with stabilized Ylides:J. Org. Chem. 29, 3327 - 3333 (1964).

Verfahrensvariante C

Es hat sich als vorteilhaft erwiesen, die Aminosäuren zu aktivieren und dann mit den β-Lactamen, die als Salze mit Aminen in Lösung gebracht werden, zu kuppeln.

Besonders vorteilhaft ist die Aktivierung der Carbonsäuren der allgemeinen Formel (VI) mit (a) Sulfonsäurederivaten der allgemeinen Formel (VIII) oder mit (b) Chlorameisensäureestern, bevorzugt Chlorameisensäureethylester, zu Anhydriden der allgemeinen Formel (IX), wie im folgenden Reaktionsschema verdeutlicht wird:

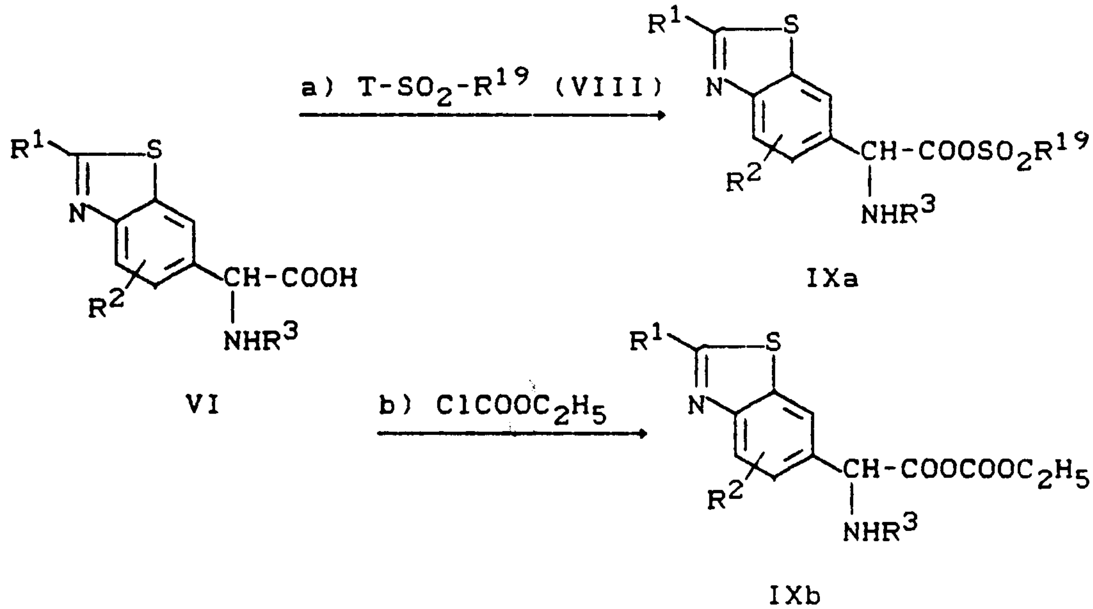


VI

IXa

IXb

Hierbei steht in der Formel (VIII) bzw. (IXa)

T

- für den Rest $R^{19}$-$SO_2$-O- oder Halogen

und

$R^{19}$

- für Alkyl mit bis zu 10 Kohlenstoffatomen, das gegebenenfalls substituiert ist durch Fluor, Chlor, Cyano, Alkyl, Alkoxycarbonyl, Alkoxy oder Alkyl mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl mit jeweils bis zu 4 Kohlenstoffatomen, Nitro, Trifluormethyl oder Phenyl.

Wenn $R^{19}$ substituiert ist, sind bevorzugt 1 bis 3 Substituenten, besonders bevorzugt die oben genannten vorhanden.

Ganz besonders bevorzugt stellt $R^{19}$ einen Methyl- oder p-Tolylrest dar.

Die gemischten Anhydride der allgemeinen Formel (IXa,b) werden hergestellt, indem man die Carbonsäuren der allgemeinen Formel (VI) und 1 bis 1,4 Äquivalente eines Amins in einem Lösemittel löst und mit 1 bis 1,2 Äquivalenten eines Sulfonsäurederivates der Formel (VIII) oder eines Chlorameisensäureesters reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen.

Als Amine eignen sich tertiäre Amine, wie beispielsweise Triethylamin, Ethyl-diisopropylamin oder Tributylamin, aber auch sterisch gehinderte sekundäre Amine, wie beispielsweise Diisopropylamin. Ebenso können Gemische der genannten Amine eingesetzt werden.

Die Umsetzungen können bei Temperaturen zwischen -80 °C und Raumtemperatur durchgeführt werden. Vorteilhaft wird die Aktivierung mit Methansulfonsäurechlorid in Dimethylformamid bei -40 °C bis -60 °C innerhalb von 0,2 bis 24 Stunden, vorzugsweise 0,5 bis 5 Stunden durchgeführt.

Zum Lösen der Vinylcephalosporinamine der Formel (VII) können die bei der Herstellung der Verbindungen der Formel (IX) genannten Lösemittel oder Wasser, sowie als Base die dort genannten Amine verwendet werden.

Besonders vorteilhaft ist auch eine Aktivierung der Carbonsäuren der allgemeinen Formel (VI) durch Überführung in einen aktivierten Ester mit beispielsweise Dicyclohexylcarbodiimid gegebenenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (IX) eignen und dort bereits aufgeführt sind.

Die Umsetzungen können bei Temperaturen zwischen -30 °C und +100 °C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit den Vinylcephalosporinaminen der Formel (VII) in Form einer Lösung ihres Aminsalzes innerhalb von 2 bis 24 Stunden umsetzt. Zum Lösen der Vinylcephalosporinamine der Formel (VII) können die bei der Herstellung der Verbindungen der Formel (IX) genannten Lösemittel sowie als Base die dort genannten Amine verwendet werden.

Die als Ausgangsstoffe eingesetzten Aldehyde der allgemeinen Formel (III) sind bekannt oder können nach bekannten Methoden hergestellt werden [Houben-Weyl's "Methoden der organischen Chemie" Bd. VII/1; E2].

Die als Ausgangsverbindungen eingesetzten Cephalosporinaldehyde der allgemeinen Formel (V) sind bekannt oder können nach bekannten Methoden durch Oxidation der entsprechenden 3-Hydroxymethyl-cephalosporinverbindungen mit Chromtrioxid in Aceton (Jones-Reagenz) hergestellt werden, wie es beispielsweise von J.A. Webber, J. L. Ott and R.T. Vasileff in J. Med. Chemistry 18, 986 (1987) beschrieben wird.

Die als Ausgangsstoffe eingesetzten Phosphoniumverbindungen der allgemeinen Formel (IV) sind bekannt oder können nach bekannten Methoden hergestellt werden [Houben-Weyl's "Methoden der organischen Chemie" Bd. V/1b, 383 bzw. 872, Bd. XII/1, 33, 167].

Die als Ausgangsstoffe eingesetzten substituierten Cephalosporinverbindungen der allgemeinen Formel (II) sind teilweise neu.

Es wurde ein Verfahren zur Herstellung der substituierten Cephalosporinverbindungen der allgemeinen Formel (II) gefunden,

das dadurch gekennzeichnet ist, daß man

Halogenmethylcephalosporinverbindungen der allgemeinen Formel (X)

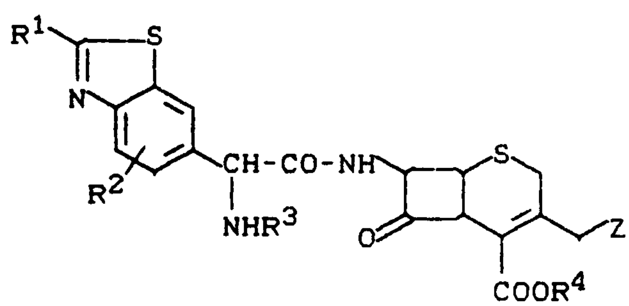

(X)

in welcher

| | |
|---|---|
| $R^1$, $R^2$, $R^3$ und $R^4$ | die oben angegebene Bedeutung haben, und |
| Z | - für Halogen, bevorzugt für Chlor, Brom oder Iod steht, |

ohne Lösemittel oder in inerten Lösemitteln mit Phosphorverbindungen der allgemeinen Formel (XI)

X| (XI)

wobei

X|

- für eine Phosphorverbindung der Formel XIa, XIb, XIc

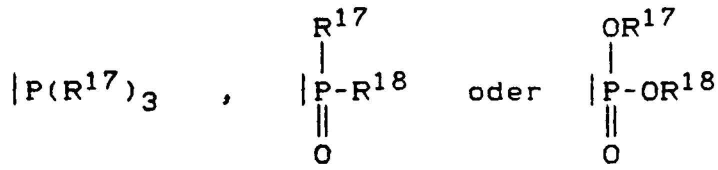
, oder

XIa XIb XIc

steht,
wobei
R[17] und R[18] gleich oder verschieden sind und
- für Alkyl, Phenyl oder gegebenenfalls substituiertes Phenyl stehen,

umsetzt.

Das erfindungsgemäße Verfahren kann durch folgendes Schema verdeutlicht werden:

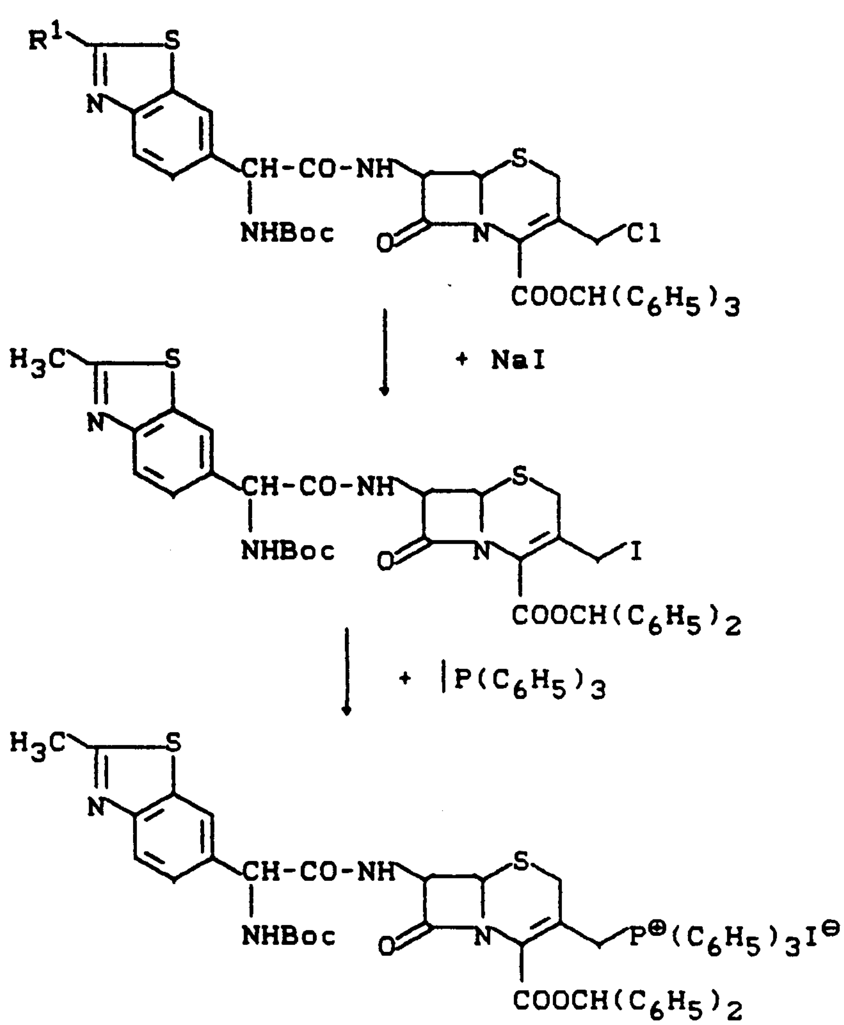


Als inerte Lösemittel eignen sich die üblichen organischen Lösemittel, die unter den Reaktionsbedingungen nicht verändert werden. Hierzu gehören bevorzugt Ether wie Diethylether, Butylmethylether, Dioxan, Tetrahydrofuran oder Glykoldimethylether, oder Kohlenwasserstoffe wie Benzol, Toluol, Xylol, Hexan, Cyclohexan, oder Erdölfraktionen, oder Halogenkohlenwasserstoffe wie Methylenchlorid, Chloroform, Tetrachlorkohlenstoff oder Chlorbenzol, oder Essigester, Aceton, Dimethylformamid, Hexamethylphosphorsäuretriamid oder Dimethylacetamid. Ebenso ist es möglich, Gemische der genannten Lösemittel einzusetzen.

Die Umsetzung wird im allgemeinen in einem Temperaturbereich von 0°C bis +150°C, bevorzugt von +20°C bis +180°C, durchgeführt.

Die Umsetzung kann bei normalem, erhöhtem oder erniedrigtem Druck durchgeführt werden. Im allgemeinen arbeitet man bei Normaldruck.

Die Umsetzung erfolgt im allgemeinen derart, daß man die Halogenmethylcephalosporinverbindung und die Phosphorverbindung in einem inertem Lösemittel mischt und gegebenenfalls erwärmt. Hierbei wird die Phosphorverbindung im allgemeinen in einer Menge von 1 bis 5, bevorzugt von 1 bis 2 mol, bezogen auf 1 mol der Chlormethylcephalosporinverbindung eingesetzt.

Bei der Durchführung des erfindungsgemäßen Verfahrens hat es sich als besonders günstig erwiesen, als Halogenmethylcephalosporinverbindungen die entsprechenden Iodverbindungen einzusetzen (X = I), die man aus den entsprechenden Chlormethyl- bzw. Brommethylverbindungen durch Behandeln mit Natriumiodid in Dimethylformamid oder Aceton erhält. Darüberhinaus ist es möglich, bei der Verwendung der Chlormethyl- bzw. Brommethylverbindungen die Umwandlung zur Iodverbindung und die Umsetzung mit der Phosphorverbindung als Eintopfreaktion durchzuführen. Hierzu werden die entsprechenden Brommethyl- bzw. Chlormethylcephalosporinverbindungen in einem geeigneten Lösemittel wie beispielsweise Ether, Essigester, Kohlenwasserstoffe, Chlorkohlenwasserstoffe, bevorzugt Aceton, mit Natriumiodid und der entsprechenden Phosphorverbindungen umgesetzt.

Die als Ausgangsstoffe eingesetzten Halogenmethylcephalosporinverbindungen der allgemeinen Formel (X) sind neu.

Es wurde ein Verfahren zur Herstellung der Halogenmethylcephalosporine der allgemeinen Formel (X) gefunden, das dadurch gekennzeichnet ist, daß man

Carbonsäuren der allgemeinen Formel (VI)

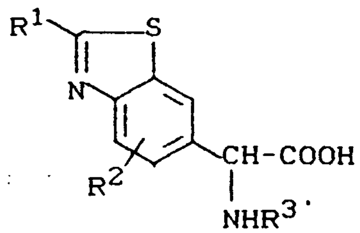

(VI)

in welcher

$R^1$ und $R^2$ die angegebene Bedeutung haben, und

$R^{3'}$ für eine in der $\beta$-Lactam-Chemie übliche Aminoschutzgruppe steht,

nach Aktivierung der Carboxylgruppe durch Überführen in ein gemischtes Anhydrid, beispielsweise mit Chlorameisensäureethylester, Chlorameisensäureisobutylester oder Methansulfonsäurechlorid, oder durch Überführen in das Säurehalogenid, oder durch Überführen in einen aktivierten Ester mit beispielsweise N-Hydroxybenztriazol und Dicyclohexylcarbodiimid

mit einer $\beta$-Lactam-Verbindung der allgemeinen Formel (XII)

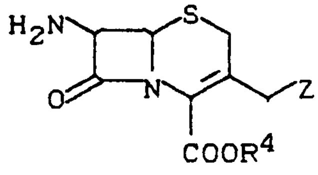

(XII)

in welcher

$R^4$ und Z die oben angegebene Bedeutung haben

umgesetzt werden, dann gegebenenfalls Schutzgruppen abgespaltet werden und die gewünschten Salze

oder aus den Salzen die freien Säuren hergestellt werden.

Für die Kupplung von Carbonsäuren (VI) an die β-Lactam-Verbindung (XII) kann eine große Zahl von aus der Cephalosporin- bzw. Penicillinchemie bekannten Methoden verwendet werden. Es hat sich als vorteilhaft erwiesen, die Carbonsäuren der allgemeinen Formel (VI) ohne Aminschutzgruppe zu aktivieren und dann mit der β-Lactam-Verbindung der Formel (XII), die als Salze mit einem Amin in Lösung gebracht wurden, zu kuppeln.

Besonders vorteilhaft ist die Aktivierung der Carbonsäuren der allgemeinen Formel (VI) mit (a) Sulfonsäurederivaten der allgemeinen Formel (VIII) oder mit (b) Chlorameisensäureestern, bevorzugt Chlorameisenäureethylester, zu Anhydriden der allgemeinen Formel (IXa, b), wie im folgenden Reaktionsschema verdeutlicht wird:

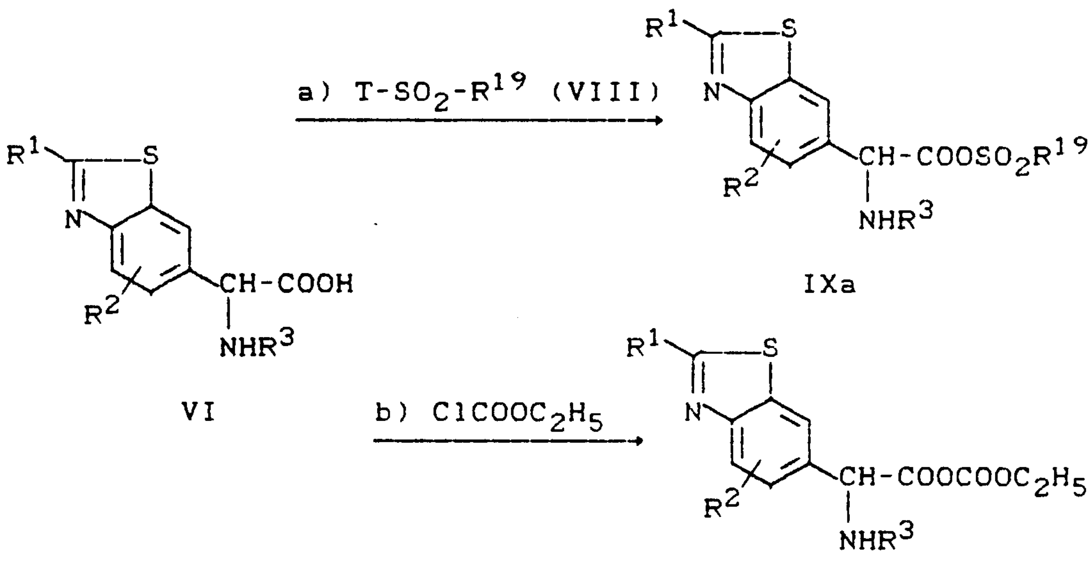


VI

IXa

IXb

Hierbei steht in der Formel (VIII) bzw. (IXa)

T
- für den Rest $R^{19}$-$SO_2$-O- oder Halogen

und

$R^{19}$
- Alkyl mit bis zu 10 Kohlenstoffatomen, das gegebenenfalls substituiert ist durch Fluor, Chlor, Cyano, Alkyl, Alkoxycarbonyl, Alkoxy oder Alkyl mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl mit jeweils bis zu 4 Kohlenstoffatomen, Nitro, Trifluormethyl oder Phenyl.

Wenn $R^{19}$ substituiert ist, sind bevorzugt 1 bis 3 Substituenten, besonders bevorzugt die oben genannten vorhanden.

Ganz besonders bevorzugt stellt $R^{19}$ einen Methyl- oder p-Tolylrest dar.

Die gemischten Anhydride der allgemeinen Formel (IXa,b) werden hergestellt, indem man die Carbonsäuren der allgemeinen Formel (VI) und 1 bis 1,4 Äquivalente eines Amins in einem Lösemittel löst und mit 1 bis 1,2 Äquivalenten eines Sulfonsäurederivatives der Formel (VIII) oder eines Chlorameisensäureesters reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen.

Als Amine eignen sich tertiäre Amine, wie beispielsweise Triethylamin, Ethyl-diisopropylamin oder Tributylamin, aber auch sterisch gehinderte sekundäre Amine, wie beispielsweise Diisopropylamin. Ebenso

können Gemische der gennanten Amine eingesetzt werden.

Die Umsetzungen können bei Temperaturen zwischen -80°C und Raumtemperatur durchgeführt werden. Vorteilhaft wird die Aktivierung mit Methansulfonsäurechlorid in Dimethylformamid bei -40°C bis -60°C innerhalb von 0,2 bis 24 Stunden, vorzugsweise 0,5 bis 5 Stunden durchgeführt.

Zum Lösen der β-Lactam-Verbindung der Formel (XII) können die bei der Herstellung der Verbindungen der Formel (IX) genannten Lösemittel oder Wasser, sowie als Base die dort genannten Amine verwendet werden.

Besonders vorteilhaft ist auch eine Aktivierung der Carbonsäuren der allgemeinen Formel (VI) durch Überführung in einen aktivierten Ester mit beispielsweise Dicyclohexylcarbodiimid gegebenenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (IX) eignen und dort bereits aufgeführt sind.

Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit der β-Lactam-Verbindung der Formel (XII) in Form einer Lösung ihres Aminsalzes innerhalb von 2 bis 24 Stunden umsetzt. Zum Lösen der β-Lactam-Verbindung der Formel (XII) können die bei der Herstellung der Verbindungen der Formel (IX) genannten Lösemittel sowie als Base die dort genannten Amine verwendet werden.

Die als Ausgangsstoffe eingesetzten Carbonsäuren der allgemeinen Formel (VI) sind bekannt oder können nach bekannten Methoden hergestellt werden [DE-OS 35 08 258].

Die als Ausgangsstoffe eingesetzten Amino-β-Lactame der allgemeinen Formel (VII + XII) sind nur zum Teil bekannt oder können nach bekannten Methoden hergestellt werden [DE-OS 34 02 642; US-Patentschrift 4 639 448].

Die erfindungsgemäßen Verbindungen der allgemeinen Formel I weisen bei geringer Toxizität ein breites antibakterielles Spektrum gegen gram-positive und gram-negative Keime auf. Diese Eigenschaften ermöglichen ihre Verwendung als chemotherapeutische Wirkstoffe in der Human-und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strep. faecalis, Strep. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella and Shigella; ferner Klebsiellen (Klebs, pneumoniae, Klebs, oxytoca), Enterobacter (Ent. aerogenes, Ent, agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis. Insbesondere wirken die erfindungsgemäßen Stoffe gegen Staphylokokken, Streptokokken,Enterokokken und Haemophilus influenzae. Bei parenteraler oder insbesondere oraler Verabreichung sind die neuen Verbindungen gegen Mikroorganismen wie Staphylokokken, Streptokokken, Enterobakteriaceen, Escherichia coli, Klebsiella, Salmonella, Shigella, anaerobe Bakterien (z.B. Bacteroides) und Proteus sehr gut wirksam.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis,

Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen.

Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die erfindungsgemäßen Verbindungen können zum Zwecke der Erweiterung des Wirkungsspektrums und um eine Wirkungssteigerung speziell bei $\beta$-lactamasebildenden Bakterien zu erzielen mit anderen

antimikrobiellen Wirkstoffen und Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

Herstellungsbeispiele

Beispiel 1

7-Amino-3-chlormethyl-3-cephem-4-carbonsäurebenzhydrylester

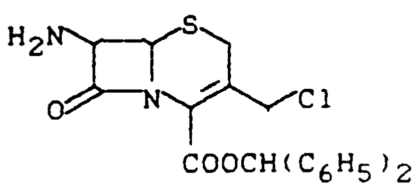

Zu einer Suspension von 50 g (0,0972 mol) 7-Phenylacetamido-3-hydroxymethyl-3-cephem-4-carbonsäurebenzhydrylester in 500 ml Methylenchlorid gibt man bei Raumtemperatur 19,64 ml (0,242 mol) Pyridin. Nach Abkühlung auf -20 °C werden 40,48 g (0,0972 mol) Phosphorpentachlorid zugegeben und 5 Minuten bei -20 °C gerührt. Mit einem Eisbad wird auf 0 °C erwärmt und 10 Minuten gerührt, anschließend wird mit einem Wasserbad auf 15 °C erwärmt und 1 Stunde gerührt. Danach wird der Ansatz auf -70 °C gekühlt und 720 ml kaltes Methanol schnell zugegeben. Dann wird 5 Minuten bei -70 °C, 10 Minuten bei 0 °C und 25 Minuten bei +15 °C gerührt. Anschließend wird die Lösung im Vakuum stark eingeengt und mit 1400 ml gesättigte Natriumhydrogencarbonat-Lösung versetzt. Man extrahiert diese Lösung dreimal mit Methylenchlorid, trocknet die organische Phase mit Natriumsulfat und engt im Vakuum ein. Das Rohprodukt wird an 500 g Kieselgel 60 (0,04 - 0,063 mm) mit Methylenchlorid chromatographiert.
Ausbeute: 29,0 g (72% der Theorie)
$C_{21}H_{19}ClN_2O_3S$ (414,9)
NMR ($CDCl_3$) : $\delta$ = 2,06 (s, 2H); 3,45 (d, 1H); 3,62 (d, 1H); 4,25 - 4,41 (q, 2H); 4,75 (d, 1H); 4,93 (d, 1H); 6,97 (s, 1H); 7,25 - 7,46 (m, 10H) ppm.

Beispiel 2

D-7-[2-(t-Butoxycarbonylamino)-2-(2-aminobenzothiazol-6-yl)-glycylamido]-3-chlormethyl-3-cepham-4-carbonsäurebenzhydrylester

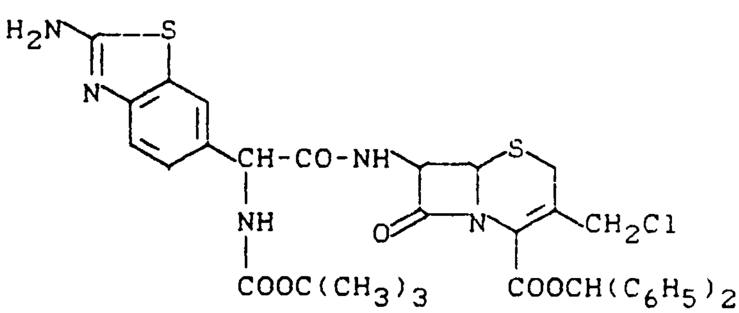

Zu einer Mischung von 28,3 g (0,0875 mol) D-α-t-Butoxycarbonylamino-α-(2-aminobenzothiazol-6-yl)-essigsäure und 24,1 g (0,058 mol) 7-Amino-3-chlormethyl-3-cephem-4-carbonsäurebenzhydrylester (Beispiel 1) in 136 ml Tetrahydrofuran und 77 ml Dimethylformamid gibt man 18,07 g (0,0875 mol) N,N'-Dicyclohexylkcarbodiimid (DCC) gelöst in 150 ml Tetrahydrofuran bei 0 °C hinzu, rührt anschließend 2

Stunden bei Raumtemperatur und engt zur Trockne ein. Der Rückstand wird in 1200 ml Essigester suspendiert, 10 Minuten gerührt und danach ungelöste Bestandteile durch Absaugen abgetrennt. Nach Abdestillieren von Essigester wird der Rückstand an Kieselgel 60 (0,04 -0,063 mm) mit Toluol / Essigester (1 : 1) chromatographiert.
Ausbeute: 17,6 g (42% der Theorie)
$C_{35}H_{34}ClN_5S_2O_6$ (720,3)
NMR (DMSO) : $\delta$ = 1,37 (s, 9H); 3,46 (d, 1H); 3,64 (d, 1H); 4,32 - 4,43 (q, 2H); 5,11 (d, 5 Hz, 1H); 5,31 (d, 1H); 5,8 - 5,86 (q, 1H); 6,98 (s, 1H); 7,15 - 7,5 (mm, 14H); 7,67 (s, 1H) ppm

Beispiel 3

D-7-[2-(t-Butoxycarbonylamino)-2-(2-aminobenzothiazol-6-yl)glycylamido]-3-iodmethyl-3-cephem-4-carbonsäurebenzhydrylester

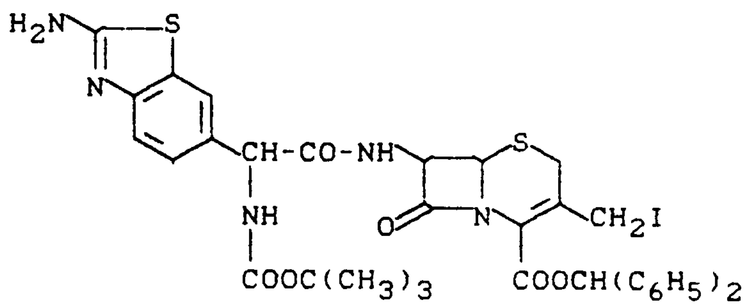

Eine Mischung von 20,3 g (0,0282 mol) D-7-[2-(t-Butoxycarbonylamino)-2-(2-aminobenzothiazol-6-yl)-glycylamido]-3-chlormethyl-3-cephem-4-carbonsäurebenzhydrylester (Beispiel 2) und 12,68 g (0,0846 mol) Natriumiodid in 300 ml Aceton wird 2 Stunden bei Raumtemperatur gerührt und zur Trockne eingedampft. Der Rückstand wird in 500 ml Essigester aufgenommen, mit einer wäßrigen Natriumthiosulfat-Lösung, Wasser und Natriumchlorid-Lösung gewaschen. Nach Trocknen über Natriumsulfat wird vom Lösemittel abdestilliert und der Rückstand in Ether digeriert.
Ausbeute: 22 g
Die Verbindung wird direkt in der nächsten Stufe eingesetzt.

Beispiel 4

D-7-[2-(t-Butoxycarbonylamino)-2-(2-aminobenzothiazol-6-yl)glycylamido]-3-(triphenylphosphonio)methyl-3-cephem-4-carbonsäurebenzhydrylester-iodid

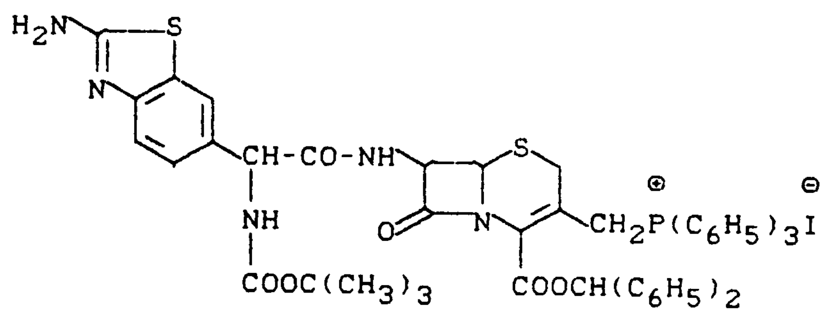

Eine Mischung von 22 g (0,0271 mol) D-7-[2-(t-Butoxycarbonylamino)-2-(2-aminobenzothiazol-6-yl)-glycylamido]-3-iodmethyl-3-cephem-4-carbonsäure-benzhydrylester (Beispiel 3) und 21,32 g (0,0813 mol) Triphenylphosphin in 500 ml Essigester rührt man 1 Stunde bei Raumtemperatur. Das Produkt fällt nach 30 Minuten aus. Man engt die Mischung bei vermindertem Druck auf etwa 150 ml ein und versetzt das Konzentrat mit 500 ml Ether. Der erhaltene Niederschlag wird abgesaugt und mit Ether nachgewaschen.
Ausbeute: 19,6 g (67% der Theorie)

$C_{53}H_{49}IN_5O_6PS_2$ (1074,1)
NMR (DMSO): δ = 1,35 (s, 9H); 3,32 - 3,42 (dd, 2H); 4,81 - 4,93 (t, 2H); 5,2 (d, 1H); 5,33 (d, 1H); 5,72 - 5,79 (q, 1H); 6,24 (s, 1H); 7,2 - 7,49 (mm, 15H); 7,6 - 7,79 (m, 15H) ppm.

Beispiel 5

D-7-[2-(t-Butoxycarbonylamino)-2-(2-aminobenzothiazol-6-yl)glycylamido]-3-[(Z)-2-cyclopropyl-vinyl]-3-cephem-4-carbonsäurebenzhydrylester

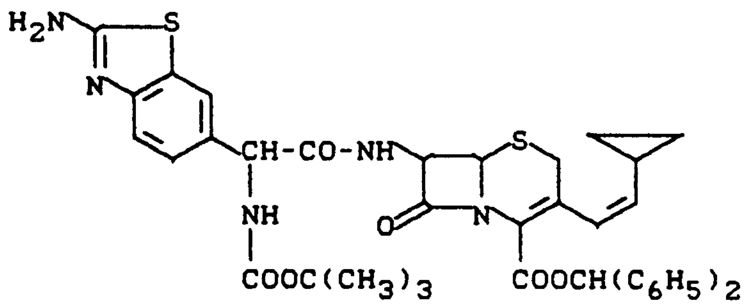

Zu einer kalten Lösung von 6,08 g (0,07 mol) Lithiumbromid in 50 ml Dimethylformamid und 150 ml Methylenchlorid gibt man bei -5 ° C 6,34 g (0,091 mol) Cyclopropancarboxaldehyd und 7,6 g (0,007 mol) D-7-[2-(t-Butoxycarbonylamino)-2-(2-aminobenzothiazol-6-yl)glycylamido]-3-(triphenylphosphonio)methyl-3-cephem-4-carbonsäurebenzhydrylester-iodid (Beispiel 4). Man rührt die Mischung 20 Stunden bei -5 ° C und danach 5 Stunden bei Raumtemperatur. Die Lösung wird im Vakuum auf ca. 50 ml eingeengt und in einem Lösemittelgemisch aus 200 ml Essigester und 200 ml Wasser verteilt. Die obere Schicht wird abgetrennt und einmal mit wäßriger Kochsalzlösung gewaschen. Nach Trocknen über Natriumsulfat und Abdestillieren des Lösemittels wird der Rückstand in Toluol aufgenommen und auf eine Säule, die mit Kieselgel (0,04 - 0,063 mm) gepackt ist, gegeben. Zunächst wird mit Toluol und danach mit dem Lösemittelgemisch Toluol / Essigester (5:1) und Toluol / Essigester (1:1) eluiert.
Ausbeute: 3,0 g (57,5% der Theorie)
$C_{39}H_{37}N_5O_6S_2$ (737,9)

| | | | | |
|---|---|---|---|---|
| Ber.: | C 63,48 | H 5,33 | N 9,49 | S 8,69 |
| Gef.: | C 62,8 | H 5,01 | N 9,18 | S 7,93 |

Beispiel 6

D-7-[(2-Aminobenzothiazol-6-yl)glycylamido]-3-[(Z)-2-cyclopropyl-vinyl]-3-cephem-4-carbonsäure, cis-Isomer

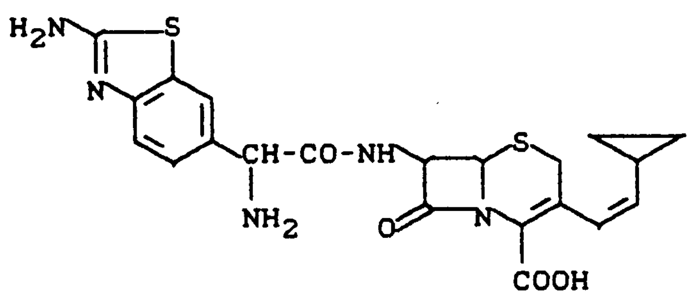

cis

2,9 g (3,9 mmol) D-7-[2-(t-Butoxycarbonylamino)-2-(2-aminobenzothiazol-6-yl)glycylamido]-3-[(Z)-2-cyclopropyl-vinyl]-3-cephem-4-carbonsäurebenzhydrylester (Beispiel 5) werden in 20 ml Methylenchlorid gelöst, mit 40 ml Trifluoressigsäure (TFA) versetzt und 60 Minuten magnetisch bei Raumtemperatur gerührt. Methylenchlorid und Trifluoressigsäure werden im Vakuum entfernt, das zurückbleibende, halbfeste rote Öl

in Ether verrieben, abgesaugt und mit Ether gewaschen. Das hellgelbe Trifluoracetat wird im Vakuum getrocknet, anschließend in 100 ml Wasser suspendiert, von unlöslichen gelben Flocken über Kieselgur abgesaugt und mit 30 ml Wasser nachgewaschen. Die noch leicht trübe Lösung wird nochmals über ein Membranfilter (Millipore, 0,45 μm) filtriert. Das Filtrat wird auf eine RP 18-Säule (Hibar 250-25, Merck) gepumpt. Die Säule wird zunächst mit 200 ml Wasser (Fraktion 1), danach mit 400 ml 5%igem Methanol (Fraktion 2) und schließlich mit 10%igem Methanol eluiert, wobei jeweils 300 ml Fraktionen gesammelt werden (Fraktion 3 bis 12).
Die Fraktionen werden mittels analytischer HPLC untersucht und die Fraktionen 6 bis 10, die den gewünschten Peak enthalten, vereinigt, Methanol im Vakuum abdestilliert und lyophilisiert.
Ausbeute: 480 mg (25,9% der Theorie)
$C_{21}H_{21}N_5O_4S_2$ (471,5)
NMR (DCOOD): δ = 0,48 (m, 2H); 0,81 (m, 2H); 1,37-1,48 (m, 1H); 3,48-3,68 (q, 2H); 5,1-5,18 (t, 1H); 5,28 (d, 1H); 5,72 (s, 1H); 5,82 (d, 1H); 6,15-6,2 (d, 1H); 7,8 (q, 2H); 8,12 (s, 1H) ppm.

Beispiel 7

7-Phenylacetamido-3-(triphenylphosphonio)methyl-3-cephem-4-carbonsäurebenzhydrylester-iodid

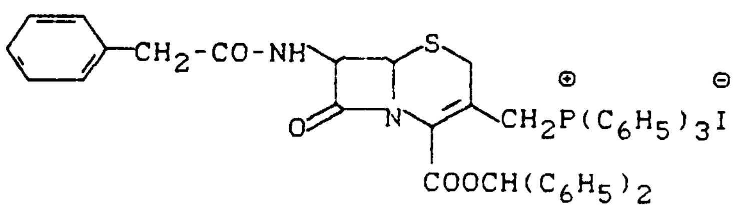

32,5 g (0,609 mol) 7-Phenylacetamido-3-chlormethyl-3-cephem-4-carbonsäure-benzhydrylester werden in 330 ml Aceton gelöst und unter Rühren mit 10,1 g (0,0674 mol) NaI und 17,6 g (0,0671 mol) Triphenylphosphin nacheinander versetzt. Nach 1,5 Stunden Rühren bei Raumtemperatur wird das unlösliche Material durch Absaugen abgetrennt und die klare Mutterlauge in 1000 ml Ether eingerührt. Das ausfallende weiße flockige Material wird abgesaugt, mit 300 ml Ether gewaschen und im Vakuum getrocknet.
Ausbeute: 51 g (94% der Theorie)
$C_{47}H_{40}INO_2O_4PS$ (886,8)
NMR (DMSO): δ = 3,51 - 3,61 (q, 4H); 4,93 - 5,05 (t, 1H); 5,22 - 5,33 (d und t, 2H); 5,7 - 5,76 (q, 1H); 6,26 (s, 1H); 7,21 - 7,46 (mm, 15H); 7,68 - 7,79 (m, 15H); 9,14 (d, 1H) ppm.

Beispiel 8

7-Phenylacetamido-3-[(Z)-2-cyclopropyl-vinyl]-3-cephem-4-carbonsäurebenzhydrylester

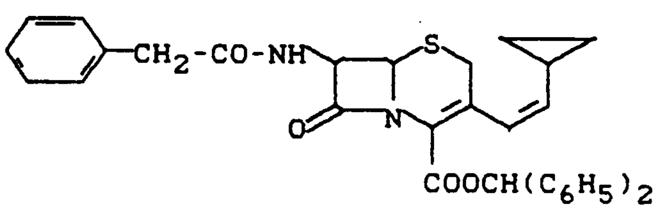

15,9 g (17,9 mmol) 7-Phenylacetamido-3-(triphenylphosphonio)methyl-3-cephem-4-carbonsäurebenzhydrylester-iodid (Beispiel 7) werden in einem 250 ml Dreihalskolben in 100 ml Methylenchlorid und 17,56 g (250,6 mmol) Cyclopropancarboxaldehyd vorgelegt. Man kühlt auf 0 °C und gibt 100 ml Wasser hinzu. Anschließend läßt man unter Konstanthaltung des pH-Wertes bei 8,6 16,3 ml 1N NaOH innerhalb von 4 Stunden zutropfen. Die Reaktionslösung wird mit Methylenchlorid verdünnt, die organische Phase abgetrennt, einmal mit Wasser gewaschen und dann über Natriumsulfat getrocknet. Nach Abtrennen des Trockenmittels wird die Methylenchlorid-Lösung nochmals mit 13 ml (233 mmol) Cyclopropancarboxaldehyd versetzt und über Nacht gerührt. Anschließend wird die Reaktionslösung bis zur Trockene eingeengt,

erneut in wenig Methylenchlorid gelöst und auf eine Säule gegeben, die mit 500 ml Kieselgel (0,04 -0,063 mm) gefüllt ist. Es werden 400 ml Fraktionen gesammelt und mittels analytischer HPLC alle Fraktionen auf die cis-isomere Verbindung untersucht.
Ausbeute: 4,5 g (45,6% der Theorie)
$C_{33}H_{30}N_2O_4S$ (550,7)

| | | | | |
|---|---|---|---|---|
| Ber.: | C 71,97 | H 5,49 | N 5,09 | S 4,58 |
| Gef.: | C 70,5 | H 5,11 | N 4,81 | S 4,05 |

NMR ($CDCl_3$): δ = 0,19-0,25 (m, 1H); 0,34-0,42 (m, 1H); 0,63-0,78 (mm, 2H); 1,24-1,35 (m, 1H); 3,4-3,6 (dd, 2H); 3,66 (q, 2H); 4,87-4,93 (t, 1H); 5,01 (d, 1H); 5,77-5,81 (q, 1H); 6,08-6,12 (d, 1H); 6,15 (d, 1H); 6,93 (s, 1H); 7,27-7,41 (mm, 15H) ppm.

Beispiel 9

7-Phenylacetamido-3-[(Z)-2-cyclopropylvinyl]-3-cephem-4-carbonsäure

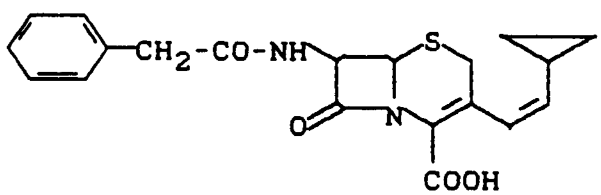

12,5 g (0,0227 mmol) 7-Phenylacetamido-3-[(Z)-2-cyclopropyl-vinyl]-3-cephem-4-carbonsäurebenzhydrylester werden in 23 ml Methylenchlorid unter Zusatz von 7,2 ml Anisol gelöst, auf 0 °C gekühlt und mit 23 ml Trifluoressigsäure versetzt. Man rührt 30 Min. bei 0 °C und gibt danach 46 ml Wasser hinzu. Anschließend versetzt man die Reaktionslösung mit 400 ml Diisopropylether und rührt 30 Min. bei 0 °C. Das auskristallisierte Produkt wird abgesaugt und mit Diisopropylether gewaschen.
Ausbeute: 6,9 g (79,1 % der Theorie)
$C_{22}H_{20}N_2O_4S$ (384,45)
NMR (DMSO): δ = 0,39-0,47 (m, 2H); 0,75-0,83 (m, 2H); 1,44-1,60 (m, 1H); 3,55 (d, 2H); 3,61 (d, 1H); 3,77 (d, 1H); 4,93-5,04 (m, 1H); 5,16 (d, 1H); 5,59-5,69 (q, 1H); 6,08-6,15 (d, 1H); 7,20-7,35 (m, 5H) ppm.

Beispiel 10

7-Amino-3-[(Z)-2-cyclopropyl-vinyl]-3-cephem-4-carbonsäure

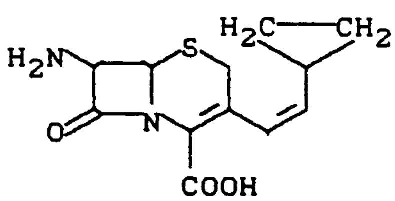

6 g (0,0156 mol) 7-Phenylacetamido-3-[(Z)-2-cyclopropyl-vinyl]-3-cephem-4-carbonsäure werden in 320 ml Wasser suspendiert und mit halbkonzentrierter Ammoniaklösung auf pH 7,8 gestellt, wobei eine fast klare Lösung entsteht. Man erwärmt die Lösung auf 37 °C und gibt 35 g Penicillin-Acylase-Harz hinzu. Die Reaktionslösung wird weiterhin mit Ammoniak auf pH 7,8 titriert und 5 Stunden bei 37 °C gehalten. Anschließend saugt man das Harz ab und engt das Filtrat bis auf Volumen von 50 ml ein. Die eingeengte Lösung wird mit 2 N HCl auf pH 4,2 gestellt, wobei das Produkt ausfällt, das abgesaugt, mit Wasser und Aceton gewaschen wird.
Ausbeute: 3,6 g (86,7 % der Theorie)
$C_{12}H_{14}N_2O_3S$ (266,32)

NMR (DCOOD): δ = 0,53-0,61 (m, 2H); 0,88-0,98 (m, 2H); 1,45-2,0 (m, 1H); 3,58 (d, 1H); 3,72 (d, 1H); 5,25-5,38 (t, 1H); 5,39 (d, 1H); 5,48 (d, 1H); 6,44-6,50 (d, 1H) ppm.

Beispiel 11

D-7-[(2-Aminobenzothiazol-6-yl)glycylamido]-3-[(Z)-2-cyclopropyl-vinyl]-3-cephem-4-carbonsäure, cis-Isomer

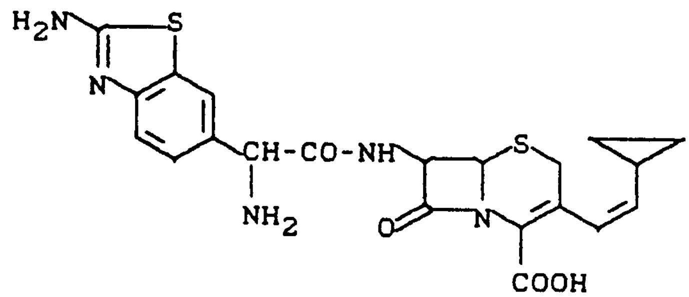

a) Aktivierung der Precursorsäure

4,12 g [entspricht 3,54 g (0,0103 mol) Reinsubstanz] Natrium-D-α-[(1-methyl-2-methoxycarbonyl-vinyl)-amino]-(2-amino-benzothiazol-6-yl)acetat (Gehalt: 86 %) werden in 30 ml Dimethylformamid und 10 ml Acetonitril klar gelöst. Man kühlt die Lösung auf -70 °C und gibt 5 Tropfen 3-Dimethylaminopropanol und 1,0 ml (0,0104 mol) Chlorameisensäureethylester hinzu und rührt 30 Minuten bei -60 °C.

b) Zubereitung der Cephalosporinkomponente

2,5 g (9,39 mmol) 7-Amino-3-[(Z)-2-cyclopropyl-vinyl]-3-cephem-4-carbonsäure werden in 20 ml Dimethylformamid und 7 ml Acetonitril suspendiert, auf 0 °C gekühlt und durch Zugabe von 1 N Natronlauge (ca. 9 ml) bis pH 8,5 in eine klare Lösung verwandelt. Man kühlt die Lösung -50 °C ab.

c) Kupplung, Deblockierung und Isolierung des Rohbetains:

Die gekühlte Cephalosporin-Lösung (b) wird zur Lösung des gemischten Anhydrids der Precursorsäure (a) bei -60 °C gegeben. Danach läßt man die Temperatur innerhalb von 90 Minuten auf -10 °C ansteigen (ohne Kühlbad) und rührt die Lösung mit 500 mg Aktivkohle und 500 mg Kieselgur zusätzlich noch 10 Minuten, wobei die Temperatur bis auf +10 °C ansteigt. Man filtriert die Reaktionsmischung über ein Seitz-Filter, wäscht mit wenig DMF nach und versetzt das Filtrat mit 2 ml konz. Salzsäure bei 0 °C. Nach 15 Minuten wird die Lösung in Vakuum aufkonzentriert und ausgefallenes Salz abgesaugt und mit wenig Dimethylformamid nachgewaschen. Das Filtrat wird mit 25 %iger Ammoniak-Lösung auf pH 4,6 gestellt und dann in 500 ml Aceton eingerührt, wobei das Rohbetain ausfällt. Man saugt den Niederschlag ab, wäscht mit Aceton nach und trocknet das Material im Vakuum. Ausbeute: 6,1 g

d) Chromatographie:

Das Rohbetain wird in 80 ml Wasser suspendiert und mit 2 N HCl ein pH-Wert von 1,4 eingestellt (klare Lösung). Die Lösung gibt man auf eine Säule, die mit 800 ml Adsorber-Harz LPG 4429 (Lewatit® OC 1062, Korngröße 0,1 bis 0,5 mm, BAYER) gefüllt ist. Zunächst eluiert man die Säule mit 1300 ml Wasser. Anschließend wäscht man die Säule mit Wasser, das kontinuierlich mit einem steigenden Gehalt von Aceton von 0 % bis 20 % versetzt wird. Man sammelt insgesamt 18 Fraktionen à 200 ml Eluat, wobei die Fraktionen 9-15 nach analytischer HPLC-Kontrolle die gewünschte Verbindung enthalten. Die Fraktionen mit dem Z-Isomer werden vereinigt, Aceton im Vakuum abdestilliert und das wäßrige Filtrat lyophilisiert.
Ausbeute: 1,42 g (29 % der Theorie)
$C_{21}H_{21}N_5O_4S_2 \cdot 3H_2O$ (525,61)

| | | | | |
|---|---|---|---|---|
| Ber.: | C 48,0 | H 5,18 | N 13,32 | S 12,20 |
| Gef.: | C 47,9 | H 5,0 | N 12,5 | S 11,7 |

NMR (DMSO): δ = 0.48 (schmales m, 2H); 0,81 (schmales m, 2H); 1,37-1,48 (m, 1H); 3,48-3,68 (q, 2H); 5,1-5,18 (t, 1H); 5,28 (d, 1H); 5,72 (s, 1H); 5,82 (d, 1H); 6,15-6,2 (d, 1H); 7,8 (q, 2H); 8,12 (s, 1H) ppm.

Beispiel 12

7-Phenylacetamido-3-[(triphenylphosphoran-yliden)-methyl]-3-cephem-4-carbonsäurebenzhydrylester

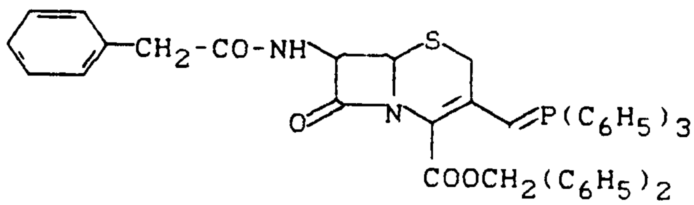

Zu einer Suspension von 78,0 g (0,088 mol) 7-Phenylacetamido-3-(triphenylphosphonio)methyl-3-cephem-4-carbonsäure-benzhydrylester-iodid (Beispiel 7) in 400 ml Methylenchlorid gibt man 14,0 g (0,132 mol) $Na_2CO_3$ gelöst in 150 ml Wasser und rührt heftig 15 Minuten bei Raumtemperatur. Die $CH_2Cl_2$-Schicht wird abgetrennt, die wäßrige Phase nochmals mit 200 ml $CH_2Cl_2$ extrahiert und die vereinigten organischen Phasen über Natriumsulfat getrocknet. Die Methylenchlorid-Phase wird bis zur Trockene eingeengt und der Rückstand in ca. 600 ml Aceton 1 h verrührt. Das Produkt wird abgesaugt und mit Aceton gründlich nachgewaschen.
Ausbeute: 58,9 g (88,3% der Theorie)
$C_{47}H_{39}N_2O_4SP$ (758,8)
NMR (DMSO); δ = 2,4 (d, 1H); 3,2 (d, 1H); 3,49 (s, 2H); 5,1 (d, 1H); 5,19 - 5,24 (q, 1H); 5,43 (d, 1H); 6,75 (s, 1H); 7,2 - 7,49 (breites m, 15H); 7,62 - 7,78 (m, 15H); 8,84 (d, 1H) ppm.

Beispiel 13

7-Phenylacetamido-3-[(Z)-3-(3,3,3-trifluoro-propenyl]-3-cephem-4-carbonsäure-benzhydrylester

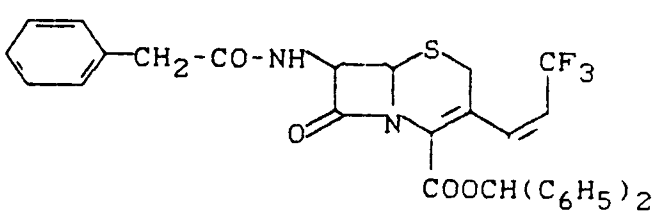

Eine Lösung von 385 ml Methylenchlorid und 38,5 ml Methanol, die mit 257 mg (1,13 mmol) p-Benzoylbenzoesäure vesetzt wird, kühlt man auf +8°C. Man gibt 32,5 g (0,332 mol) Trifluoroacetaldehyd innerhalb von 10 Minuten hinzu, wobei die Temperatur der Lösung auf +15°C ansteigen darf. 5 Minuten nach der Zugabe gibt man 12,6 g (0,0166 mol) 7-Phenylacetamido-3-[(triphenylphosphoranyliden)-methyl]-3-cephem-4-carbonsäurebenzhydrylester (Beispiel 12) hinzu, entfernt das Kältebad und rührt die Mischung 4 Stunden bei +35°C unter Lichtausschluß und Stickstoff. Die dunkelrote Lösung wird danach bis zur Trockene eingeengt und das zurückbleibende Öl mit Ether versetzt, wobei allmählich Kristallisation einsetzt.
Ausbeute: 6,2 g (64,6% der Theorie)
$C_{31}H_{25}F_3N_2O_4S$ (578,6)

| | | | | | |
|---|---|---|---|---|---|
| Ber.: | C 64,35 | H 4,36 | N 4,84 | S 5,54 | F 9,85 |
| Gef.: | C 63,72 | H 4,10 | N 4,27 | S 5,02 | F 9,27 |

Beispiel 14

7-Amino-3-[(Z)-3-(3,3,3-trifluoro-propenyl]-3-cephem-4-carbonsäure-benzhydrylester

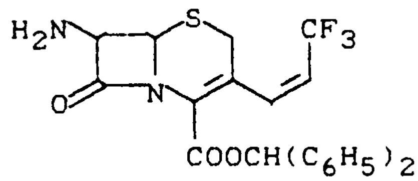

6,0 g (0,0104 mol) 7-Phenylacetamido-3-[(Z)-3-(3,3,3-trifluoropropenyl]-3-cephem-4-carbonsäure-benzhydryl ester (Beispiel 13) werden in 64 ml Methylenchlorid gelöst, mit einem Trockeneisbad auf -40 °C gekühlt und nacheinander 2,1 ml (0,026 mol) Pyridin und 2,17 g (0,0104 mol) Phosphorpentachlorid zugesetzt. Nach 5 Minuten läßt man auf -20 °C erwärmen, danach soll die Temperatur innerhalb von 20 Minuten auf -10 °C und schließlich auf +10 °C ansteigen. Man rührt nunmehr die Lösung 1 Stunde bei +10 °C bis +15 °C. Anschließend kühlt man den Ansatz auf -40 °C, gibt 70 ml Methanol (-30 °C) hinzu und rührt noch 30 Minuten bei +10 °C. Die Reaktionslösung wird schonend eingeengt, das anfallende Öl in 600 ml Methylenchlorid gelöst, in 800 ml Natriumhydrogencarbonat-Lösung eingerührt und 10 Minuten gerührt. Die Methylenchlorid-Phase wird abgetrennt, einmal mit Wasser gewaschen und über Natriumsulfat getrocknet. Das Methylenchlorid-Filtrat wird an 400 ml Kieselgel (0,04 - 0,063 mm) chromatographiert, wobei zunächst mit Methylenchlorid und danach mit Methylenchlorid unter Zusatz von Methanol (Gradient bis 10%) eluiert wird. Das Eluat wird mittels analytischer HPLC und Dünnschichtchromatographie (DC: Methylenchlorid / Methanol = 100:1) untersucht.
Ausbeute: 3,4 g (71,1% der Theorie)
$C_{23}H_{19}F_3N_2O_3S$ (460,5)
NMR ($CDCl_3$): $\delta$ = 3,3 (d, 1H); 3,48 (d, 1H); 4,75 (d, 1H); 4,98 (d, 1H); 5,45 - 5,55 (d - q, 1H); 6,07 (d, 1H); 6,96 (s, 1H); 7,23 - 7,42 (m, 10H); 8,6 (d, 2H) ppm.

Beispiel 15

7-Amino-3-[(Z)-3-(3,3,3-trifluoro-propenyl]-3-cephem-4-carbonsäure

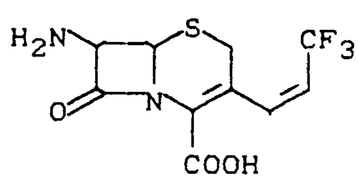

Zu einer gerührten Lösung von 30 ml Trifluoressigsäure (TFA) und 1,5 ml Anisol, die auf 0 °C gekühlt wird, gibt man 3,1 g (6,73 mmol) 7-Amino-3-[(Z)-3-(3,3,3-trifluoro-propenyl]-3-cephem-4-carbonsäurebenzhydrylester (Beispiel 14). Die Mischung wird 1 Stunde bei Raumtemperatur gerührt, anschließend bei 30 °C im Vakuum eingeengt und der ölige Rückstand mit 100 ml Ether 1 Stunde verrührt. Der Niederschlag wird abgesaugt, mit 50 ml Ether gewaschen und der Filterrückstand 3 Stunden im Vakuum getrocknet. Das Trifluoracetat wird in 20 ml Wasser suspendiert, die Aufschlämmung auf +5 °C gekühlt und mit 12 N HCl der pH-Wert auf 0,2 - 0,4 gestellt. Die entstandene klare Lösung wird auf +5 °C gekühlt und mit 300 mg Aktivkohle 10 Min. gerührt. Man saugt über Kieselgur ab und wäscht mit ca. 20 ml 0,1 N HCl nach. Das Filtrat wird bei +5 °C mit 20%iger NaOH bis pH 2,1 eingestellt und das ausgefallene Produkt 1 Stunde im

Kühlschrank stehen gelassen, um die Kristallisation zu vervollständigen. Der Kristallbrei wird abgesaugt, mit 20 ml Wasser und 80 ml Aceton gewaschen und im Vakuum getrocknet.
Ausbeute: 1,45 g (73,2% der Theorie)
$C_{10}H_9F_3N_2O_3S$ (294,3)

| | | | | | |
|---|---|---|---|---|---|
| Ber.: | C 40,81 | H 3,08 | N 9,52 | S 10,89 | F 19,37 |
| Gef.: | C 39,60 | H 2,91 | N 9,02 | S 10,18 | F 18,52 |

Beispiel 16

D-7-[(2-Aminobenzothiazol-6-yl)glycyl-amino]-3-[(Z)-3-(3,3,3-trifluoro-propenyl]-3-cephem-4-carbonsäure, cis-Isomer

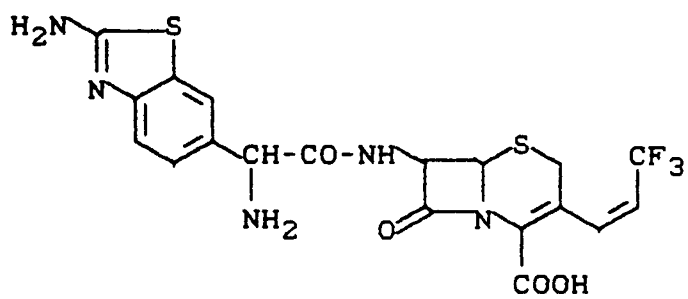

a) Activierung der Precursorsäure:

1,72 g [entspricht 1,63 g (4,75 mmol)] Natrium-D-α-[(1-methyl-2-methoxycarbonyl-vinyl)-amino]-(2-aminobenzothiazol-6-yl)acetat (Gehalt = 95%) werden in 15 ml Dimethylformamid gelöst und danach mit 7 ml Acetonitril verdünnt. Man kühlt die Lösung auf -70 °C und gibt hintereinander 40 µl 3-Dimelthylaminopropanol und 0,456 ml (4,75 mmol) Chlorameisensäureethylester hinzu und rührt 20 Min. bei -70 °C.

b) Zubereitung der Cephalosporinkomponente

1,4 g (4,75 mmol) 7-Amino-3-[(Z)-3-(3,3,3-trifluoropropenyl]-3-cephem-4-carbonsäure (Beispiel 15) werden in 15 ml Dimethylformamid und 7 ml Acetonitril suspendiert und durch Zugabe von 1 N Natronlauge (4,2 ml) bei Raumtemperatur bis pH 8,5 in eine klare Lösung verwandelt. Man kühlt die Lösung auf -20 °C bis -30 °C ab.

c) Kupplung, Deblockierung und Isolierung des Rohbetains

Die gekühlte Lösung des 3-Trifluoropropenylcephalosporins b) (-20 °C) läßt man langsam zur Lösung des gemischten Anhydrids der Precursorsäure nach a) bei -70 °C zutropfen und rührt 10 Min. bei -70 °C nach. Anschließend läßt man die Temperatur der Lösung innerhalb von 45 Min. auf 0 °C kommen (ohne Kühlung) und rührt mit 150 mg Aktivkohle und 500 mg Kieselgur noch weitere 10 Min. Man filtriert die Reaktionsmischung über ein Seitz-Filter, wäscht mit wenig Dimethylformamid nach und versetzt das Filtrat mit 1 ml konzentrierter Salzsäure. Das Volumen der Lösung wird bis auf 25 ml konzentriert, wobei ausgefallene Salze abgetrennt werden. Das Filtrat wird unter magnetischem Rühren mit 25% $NH_3$-Lösung auf pH 4,0 gestellt und mit 100 ml Aceton versetzt, wobei das Rohbetain ausfällt. Man rührt die Ausfällung 10 Min, saugt ab und wäscht mit Aceton nach und trocknet das Material im Vakuum.
Ausbeute: 1,9 g (74,5% der Theorie)
Das Rohbetain wird in Wasser suspendiert, mit halbkonzentrierter Salzsäure bei pH 1,2 in Lösung gebracht und 15 Min. mit 190 mg A-Kohle gerührt. Die Mischung wird über ein Kieselgur-Bett abgesaugt, mit 15 ml 0,1 N Salzsäure nachgewaschen und das Filtrat auf eine RP 18-Säule (Hibar 250-25, Merck) gepumpt. Die Säule wird zunächst mit Wasser, danach mit 5%igem Methanol eluiert. Die Fraktionen werden mittels

analytischer HPLC untersucht und die Fraktionen, die das Z-isomere Derivat enthalten, vereinigt, Methanol im Vakuum abdestilliert und die wäßrige Lösung lyophilisiert.
Ausbeute: 520 mg (31,7% der Theorie)
$C_{19}H_{16}F_3N_5O_4S_2 \cdot 2H_2O$ (535,5)
NMR (DCOOD): $\delta$ = 3,21 (d, 1H); 3,52 (d, 1H); 5,19 (d, 1H); 5,78 - 5,91 (m, 3H); 6,25 (d, 1); 7,81 - 7,9 (q, 2H); 8,18 (s, 1H) ppm.

Beispiel 17

D-7-[(2-Aminobenzothiazol-6-yl)glycylamido]-3-[(E)-3-(3,3,3-trifluoropropenyl]-3-cephem-4-carbonsäure, trans-Isomer

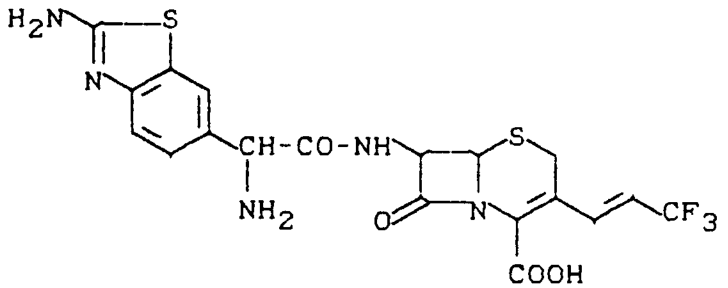

trans

Die trans-isomere-Verbindung wird als Nebenprodukt durch präparative HPLC-Trennung an einer Hibar-Säule, RP-18 aus den methanolhaltigen Eluaten des Beispiels 16 erhalten.
IR: Nujol 1780, 1690, 1620, 1520,
$\nu$ max. 1470, 1380, 1350, 1280 $cm^{-1}$

Beispiel 18

D-7-[(2-Aminobenzothiazol-6-yl)glycylamido]-3-[2-(3-pyridyl)vinyl]-3-cephem-4-carbonsäure, cis-Isomer

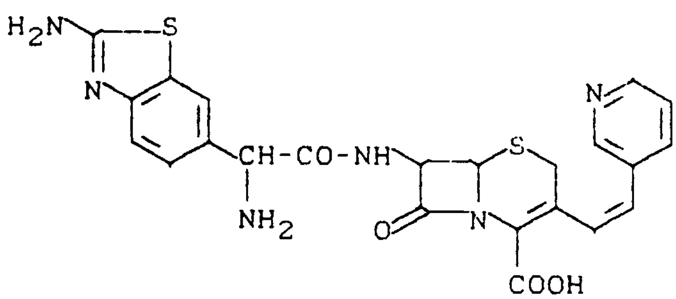

1,6 g entspricht 1,52 g (4,43 mmol) Natrium-D-$\alpha$-[(1-Methyl-2-methoxycarbonyl-vinyl)-amino]-(2-aminobenzothiazol-6-yl)acetat (Gehalt: 95%) werden analog Beispiel 16 in Dimethylformamid / Acetonitril mit 0,425 ml (4,43 mmol) Chlorameisensäureethylester mit katalytischen Mengen 3-Dimethylaminopropanol aktiviert und mit einer Lösung von 1,34 g (4,43 mmol) 7-Amino-3-[2-(3-pyridyl)vinyl]-3-cephem-4-carbonsäure in Dimethylformamid / Acetonitril und Natronlauge (1N) umgesetzt.
Nach Isolierung des Rohbetains analog Beispiel 16 erfolgt mit Hilfe der präparativen HPLC die Aufreinigung der cis-isomeren Verbindung.
Ausbeute: 735 mg (30,5% der Theorie)
$C_{23}H_{20}N_6O_4S_2 \cdot 2H_2O$ (544,6)
IR (Nujol), max.: 1775, 1690, 1620, 1530 $cm^{-1}$.

**Patentansprüche**
**Patentansprüche für folgende Vertragsstaaten : AT, BE, CH, DE, FR, GB, IT, LI, NL, SE**

1. β-Lactam-Verbindungen der allgemeinen Formel (I)

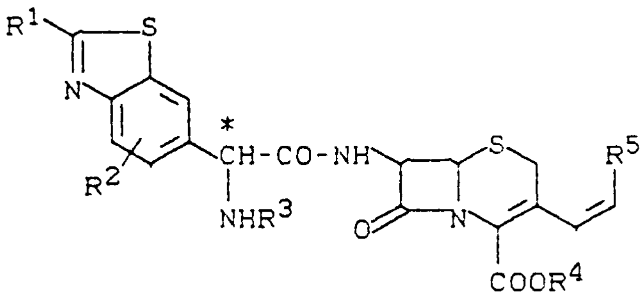

(I),

in welcher

$R^1$

- für Wasserstoff steht, oder
- für geradkettiges, verzweigtes oder cyclisches Alkyl oder Alkenyl mit bis zu 10 Kohlenstoffatomen steht, das substituiert sein kann durch Halogen, Hydroxy, Alkoxy mit bis zu 6 Kohlenstoffatomen, Cyano, Sulfo, Alkylsulfonyl mit bis zu 6 Kohlenstoffatomen, oder durch eine Gruppe der Formel $-NR^6R^7$,

worin

$R^6$, $R^7$

- gleich oder verschieden sind und
- Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, oder eine Aminoschutzgruppe bedeuten,

  oder
- für Aryl mit 6 bis 12 Kohlenstoffatomen steht, das substituiert sein kann durch Halogen, Cyano, Nitro, Alkyl, Alkoxy, Alkylthio mit jeweils bis zu 6 Kohlenstoffatomen, Trifluormethyl oder Trifluormethoxy, oder
- für Halogen, Alkoxy, Alkylthio, Alkylsulfonyl mit jeweils bis zu 8 Kohlenstoffatomen, Mercapto, Hydroxy, Phenylthio, Phenyloxy, Benzylthio, Benzyloxy, Sulfo, Sulfamoyl, - $PO(OH)_2$, $-NHNH_2$, -NHOH, Guanidino, Amidino, Heterocyclyl, Heterocyclylthio oder Heterocyclyloxy mit Heterocyclen aus der Reihe Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxazolyl, Chinazolyl, Piperidinyl, Morpholinyl, Piperazinyl, Thiomorpholinyl, Furyl, Thienyl, Oxazolyl, Thiazolyl, Isoxazolyl, Thiadiazolyl, Triazolyl oder Tetrazolyl, die wiederum substituiert sein können durch Methyl, Methoxy oder Halogen,
- oder für eine Gruppe der Formel $-NR^6R^7$ steht,

worin

$R^6$, $R^7$

- die oben angegebene Bedeutung haben,

$R^2$

- für Wasserstoff, Alkyl, Alkoxy, Alkylthio mit jeweils bis zu 8 Kohlenstoffatomen, Trifluormethyl, Trifluormethoxy, Hydroxy, Mercapto, Nitro, Cyano, Halogen oder Amino steht,

$R^3$

- für Wasserstoff oder
- für eine in der β-Lactamchemie übliche Aminoschutzgruppe steht,

$R^4$

- für Wasserstoff,
- für eine in der β-Lactamchemie übliche Carboxyschutzgruppe oder
- für einen in vivo spaltbaren Ester steht

und

$R^5$

- für Halogen, Cyano, Trifluormethyl, Carboxy, Alkoxycarbonyl mit bis zu 8 Kohlenstoffatomen, Alkylsulfonyl, Alkylsulfonyloxy mit jeweils bis zu 8 Kohlenstoffatomen, Phenylsulfonyl, Tolylsulfonyl, Phenylsulfonyloxy, Tolylsulfonyloxy, Sulfamoyl, Dialkylsulfamoyl mit jeweils bis zu 6 Kohlenstoffatomen je Alkylgruppe, oder
- für Cycloalkyl mit 3 bis 8 Kohlenstoffatomen steht, das substituiert sein kann durch Alkyl, Alkoxy, Alkoxycarbonyl mit jeweils bis zu 6 Kohlenstoffatomen, Carboxy, Carbamoyl, Halogen, Cyano oder Phenyl, oder
- für Cycloalkylalkyl mit 3 bis 6 Ringgliedern und 4 bis 10 Kohlenstoffatomen, oder
- für geradkettiges oder verzweigtes Alkinyl mit bis zu 8 Kohlenstoffatomen steht, das substituiert sein kann, durch Aryl mit 6 bis 12 Kohlenstoffatomen, Carboxy, Alkoxycarbonyl mit bis zu 8 Kohlenstoffatomen, oder durch Halogen, oder
- für Aryl mit 6 bis 12 Kohlenstoffatomen steht, das substituiert sein kann durch Halogen, Cyano, Nitro, Trifluormethyl, Trifluormethoxy, Trifluormethylthio, Alkyl, Alkoxy oder Alkylthio mit jeweils bis zu 6 Kohlenstoffatomen, oder
- für einen heterocyclischen Rest der Reihe Thienyl, Furyl, Pyridyl, Pyridyloxid, Pyrimidyl, Pyrazinyl, Pyridazinyl, Pyrazolyl, Imidazolyl, Triazolyl, Tetrazolyl, Oxazolyl, Thiazolyl oder Thiadiazolyl steht, wobei die heterocyclischen Reste durch Alkyl, Alkoxy mit jeweils bis zu 6 Kohlenstoffatomen, Halogen, Nitro oder Cyano substituiert sein können, oder
- für eine Gruppe der Formel $-NR^8R^9$, $-CH_2-R^{10}$ oder $-S-R^{11}$ steht,

worin

$R^8$, $R^9$

- gleich oder verschieden sind und
- Wasserstoff, Alkyl mit bis zu 6 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen oder eine Aminoschutzgruppe bedeuten, oder

worin

$R^8$, $R^9$

- gemeinsam mit dem Stickstoffatom einen 5 bis 7-gliedrigen Ring bilden, der durch O, S, NH, N-Alkyl mit bis zu 4 Kohlenstoffatomen oder N-Phenyl unterbrochen sein kann,

$R^{10}$

- einen heterocyclischen Rest der Reihe Thienyl, Furyl, Pyridyl, Pyridyloxid, Pyrimidyl, Pyrazinyl, Pyridazinyl, Pyrazolyl, Imidazolyl, Triazolyl, Tetrazolyl, Oxazolyl, Thiazolyl oder Thiadiazolyl bedeutet, wobei die heterocyclischen Reste durch Alkyl, Alkoxy, Alkylthio mit jeweils bis zu 6 Kohlenstoffatomen, Halogen, Nitro, Cyano, Trifluormethyl, Trifluormethoxy substituiert sein können,

und

$R^{11}$

- einen heterocyclischen Rest der Reihe Thienyl, Furyl, Pyridyl, Pyridyloxid, Pyrimidyl, Imidazolyl, Triazolyl, Oxazolyl oder Thiadiazolyl bedeutet, wobei die heterocyclischen Reste durch Alkyl, Alkoxy mit jeweils bis zu 6 Kohlenstoffatomen, Halogen, Trifluormethyl, Trifluormethoxy, Nitro oder Cyano substituiert sein können,

und deren Salze.

**2.** Verbindungen der allgemeinen Formel (I) nach Anspruch 1, in welcher

$R^1$

- für Wasserstoff oder
- für geradkettiges, verzweigtes oder cyclisches Alkyl steht, das substituiert sein kann durch Fluor, Chlor, Brom, Hydroxy, Alkoxy, Cyano, oder durch eine Gruppe der Formel

$-NR^6R^7$,

worin

$R^6$, $R^7$

- gleich oder verschieden sind und für
- Wasserstoff, Alkyl mit bis zu 6 Kohlenstoffatomen, Phenyl, Benzyl oder eine Aminoschutzgruppe der Reihe 4-Methoxyphenyl, 3,4-Dimethoxyphenyl, 4-Methoxymethyloxyphenyl, 4-[(2-Methoxyethoxy)methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyl-dimethylsilyl, tert-Butyl-diphenylsilyl, Methoxycarbonylmethyl, tert-Butoxycarbonylmethyl oder 2-Hydroxy-2-phenylethyl steht,
  oder
- für gegebenenfalls durch Fluor, Chlor, Brom, Alkyl, Alkoxy mit bis zu 6 Kohlenstoffatomen, Trifluormethyl, Trifluormethoxy oder Cyano substituiertes Phenyl steht, oder
- für Fluor, Chlor, Brom, Alkoxy, Alkylthio, Alkylsulfonyl mit jeweils bis zu 6 Kohlenstoffatomen, Mercapto, Hydroxy, Phenylthio, Phenyloxy, Sulfamoyl oder
- für eine Gruppe der Formel $-NR^6R^7$ steht,

worin

$R^6$, $R^7$

- die oben angegebene Bedeutung haben,

$R^2$

- für Wasserstoff, Methyl, Methoxy, Trifluormethyl, Trifluormethoxy, Cyano, Fluor, Chlor oder Brom steht,

$R^3$

- für Wasserstoff oder
- für eine Aminoschutzgruppe aus der Reihe: 4-Methoxyphenyl, 3,4-Dimethoxyphenyl, 4-Methoxymethyloxyphenyl, 4-[(2-Methoxyethoxy)methyloxylphenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyl-dimethylsilyl, tert-Butyl-diphenylsilyl, Methoxycarbonylmethyl, tert-Butoxycarbonylmethyl, 2-Hydroxy-2-phenylethyl, tert-Butyloxycarbonyl, 1-Methyl-2-benzoyl-vinyl oder 1-Methyl-2-methoxy-vinyl steht,

$R^4$

- für Wasserstoff steht, oder
- für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht, oder
- für einen Rest der Formel

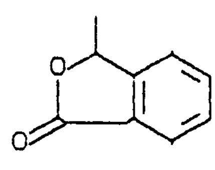 , 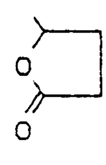 , 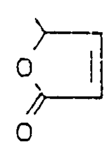 ,

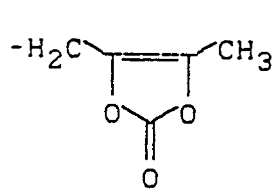 ,

$-CH_2-OCO-C(CH_3)_3$, $-CH(CH_3)-OCOOC_2H_5$ oder $-CH_2-OCOCH_3$ steht,

und

$R^5$

- für Fluor, Chlor, Brom, Cyano, Trifluormethyl, Carboxy, Alkoxycarbonyl mit bis zu 6 Kohlenstoffatomen, Alkylsulfonyl, Alkylsulfonyloxy mit jeweils bis zu 6 Kohlenstoffatomen, Phenylsulfonyl, Tolylsulfonyl, Phenylsulfonyloxy, Tolylsulfonyloxy, oder
- für Cyclopropyl, Cyclopentyl oder Cyclohexyl steht, die substituiert sein können durch Alkyl, Alkoxy, Alkoxycarbonyl mit jeweils bis zu 4 Kohlenstoffatomen, Fluor, Chlor, Brom oder Phenyl, oder
- für Cyclopropylmethyl, Cyclopentylmethyl oder Cyclohexylmethyl steht, oder
- für geradkettiges oder verzweigtes Alkinyl mit bis zu 6 Kohlenstoffatomen steht, das substituiert sein kann durch Phenyl, Carboxy, Alkoxycarbonyl mit bis zu 6 Kohlenstoffatomen oder durch Fluor, Chlor, Brom, oder
- für Phenyl steht, das substituiert sein kann durch Fluor, Chlor, Brom, Cyano, Nitro, Trifluormethyl, Trifluormethoxy, Alkyl oder Alkoxy mit bis zu 4 Kohlenstoffatomen, oder
- für einen heterocyclischen Rest der Reihe Thienyl, Furyl, Pyridyl, Pyridyloxid, Pyrimidyl, Pyrazolyl, Triazolyl, Tetrazolyl, Oxazolyl, Thiazolyl oder Thiadiazolyl steht, wobei die heterocyclischen Reste durch Alkyl, Alkoxy mit jeweils bis zu 4 Kohlenstoffatomen, Fluor, Chlor, Brom oder Nitro substituiert sein können, oder
- für eine Gruppe der Formel $-NR^8R^9$, $-CH_2-R^{10}$ oder $-S-R^{11}$ steht,

worin

$R^8$, $R^9$

- gleich oder verschieden sind und
- Wasserstoff, Alkyl mit bis zu 4 Kohlenstoffatomen, Phenyl, Benzyl oder eine Aminoschutzgruppe der Reihe 4-Methoxyphenyl, 3,4-Dimethoxyphenyl, 4-Methoxymethyloxyphenyl, 4-[(2-Methoxyethoxy)methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyldimethylsilyl, tert-Butyl-diphenylsilyl, Methoxycarbonylmethyl, tert-Butoxycarbonylmethyl oder 2-Hydroxy-2-phenylethyl bedeuten, oder worin

$R^8$, $R^9$

- gemeinsam mit dem Stickstoffatomen einen Pyrrolidin-, Piperidin-, Morpholin-, Thiomorpholin-, Piperazin-, 4-Methyl- oder 4-Phenylpiperazinring bilden,

$R^{10}$

- einen heterocyclischen Ring der Reihe Thienyl, Furyl, Pyridyl, Pyridyloxid, Pyrimidyl, Pyrazolyl, Triazolyl, Tetrazolyl, Oxazolyl, Thiazolyl oder Thiadiazolyl bedeutet, wobei die heterocyclischen Reste durch Alkyl, Alkoxy mit jeweils bis zu 4 Kohlenstoffatomen, Fluor, Chlor, Brom oder Nitro substituiert sein können,

und

$R^{11}$

- einen heterocyclischen Ring der Reihe Thienyl, Furyl, Pyridyl, Pyridyloxid, Pyrimidyl, Pyrazolyl, Oxazolyl oder Thiadiazolyl bedeutet, wobei die heterocyclischen Reste durch Alkyl, Alkoxy mit jeweils bis zu 4 Kohlenstoffatomen, Fluor, Chlor, Brom oder Nitro substituiert sein kann

und deren Salze.

**3.** Verbindungen der allgemeinen Formel (I) nach Anspruch 1,
in welcher

$R^1$

- für Wasserstoff oder für Cyclopropyl steht, oder
- für geradkettiges oder verzweigtes Alkyl mit bis zu 6 Kohlenstoffatomen steht, das durch Fluor, Chlor, Methoxy, Cyano, Dimethylamino oder Diethylamino substituiert sein kann, oder
- für Phenyl steht, das durch Fluor, Chlor, Methyl, Methoxy, Trifluormethyl, Amino oder Trifluormethoxy substituiert sein kann, oder
- für Chlor, Alkoxy, Alkylsulfonyl mit jeweils bis zu 4 Kohlenstoffatomen, Mercapto, Hydroxy oder

- für eine Gruppe der Formel $-NHR^6$ steht,

worin

$R^6$

- Wasserstoff, Alkyl mit bis zu 4 Kohlenstoffatomen, Phenyl, Benzyl oder eine Aminoschutzgruppe der Reihe 4-Methoxyphenyl, 4-Methoxybenzyl, 2,4-Dimethoxybenzyl, 3,4-Dimethoxybenzyl oder tert-Butyl-dimethylsilyl bedeutet,

$R^2$

- für Wasserstoff steht,

$R^3$

- für Wasserstoff oder
- für 4-Methoxyphenyl, 4-Methoxybenzyl, 2,4-Dimethoxybenzyl, 3,4-Dimethoxybenzyl, tert-Butyl-dimethylsilyl, 1-Methyl-2-methoxy-vinyl (MMV) oder tert-Buyloxycarbonyl (Boc), steht, oder

$R^4$

- für Wasserstoff steht, oder
- für Methyl, Ethyl, tert.Butyl, Diphenylmethyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
- für einen Rest der Formel

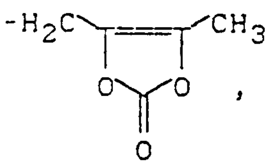

,

$-CH(CH_3)-OCOOC_2H_5$ oder $-CH_2-OCO-C(CH_3)_3$ steht,

und

$R^5$

- für Chlor, Brom, Trifluormethyl, Carboxy, Alkoxycarbonyl, Alkylsulfonyloxy mit jeweils bis zu 4 Kohlenstoffatomen, Phenylsulfonyloxy, Tolylsulfonyloxy, Cyclopropyl, Cyclopentyl, Cyclohexyl, Cyclopropylmethyl, Cyclopentylmethyl steht, oder
- für Alkinyl mit bis zu 4 Kohlenstoffatomen steht, das durch Phenyl, Carboxy oder Alkoxycarbonyl mit bis zu 4 Kohlenstoffatomen substituiert sein kann, oder
- für Phenyl steht, oder

- für einen Rest der Formel

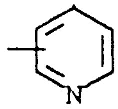 , 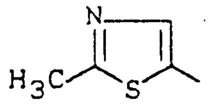 , 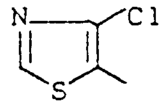 , 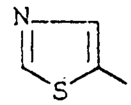 ,

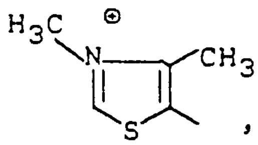 , 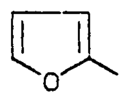 , 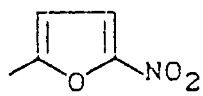 , 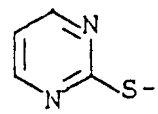 ,

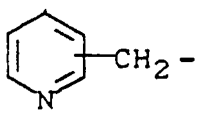 , 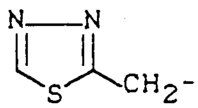 , 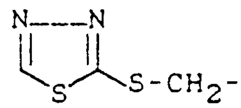 ,

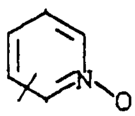 , 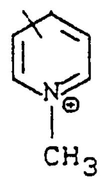 , 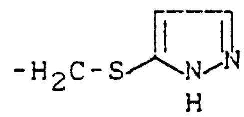

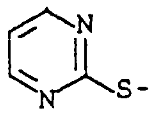 oder 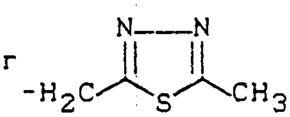

steht,

und deren Salze.

**4.** Verfahren zur Herstellung von $\beta$-Lactam-Verbindungen der allgemeinen Formel (I)

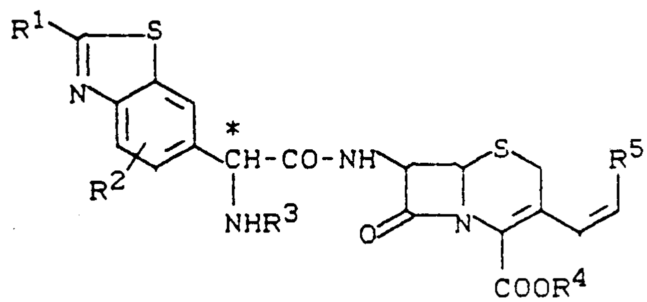 (I),

gemäß Anspruch 1, und deren Salzen, dadurch gekennzeichnet, daß man

[A] substituierte Cephalosporinverbindungen der allgemeinen Formel (II)

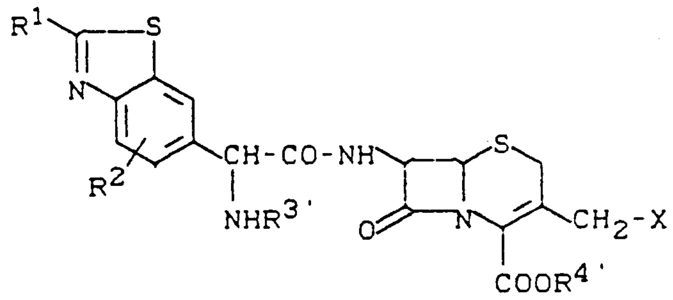

(II)

in welcher

$R^1$ und $R^2$ die oben angegebene Bedeutung hat,

$R^{3'}$ für eine in der $\beta$-Lactamchemie übliche Aminoschutzgruppe steht,

$R^4$ für eine in der $\beta$-Lactamchemie übliche Carboxyschutzgruppe steht und

X

- für eine Gruppe der Formel

$-\overset{\oplus}{P}(R^{17})_3Z^{\ominus}$ , 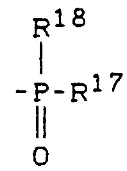 oder 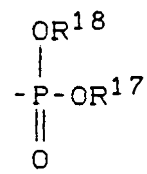

steht,

wobei

$R^{17}$ und $R^{18}$ gleich oder verschieden sind und Alkyl, Phenyl oder Tolyl bedeuten und

$Z^{\ominus}$

- ein Halogenidanion, bevorzugt Chlorid, Bromid oder Iodid bedeutet,

in inerten Lösemitteln in Anwesenheit von Basen, mit Aldehyden der allgemeinen Formel (III)

$R^5$-CHO (III)

in welcher

$R^5$ die oben angegebene Bedeutung hat

umsetzt,

oder daß man

[B] Phosphoniumverbindungen der allgemeinen Formel (IV)

$R^5-CH_2-X$ (IV)

in welcher

$R^5$ die oben angegebene Bedeutung hat

und

X

- für eine Gruppe der Formel

$-\overset{\oplus}{P}(R^{17})_3Z^{\ominus}$ , 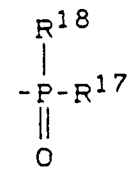 oder 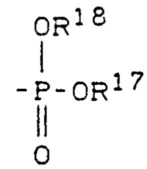

steht,

wobei

$R^{17}$ und $R^{18}$ gleich oder verschieden sind und Alkyl, Phenyl oder Tolyl bedeuten

und

$Z^{\ominus}$

- ein Halogenidanion, bevorzugt Chlorid, Bromid oder Iodid bedeutet,

in inerten Lösemitteln in Anwesenheit von Basen, mit Cephalosporinaldehyden der allgemeinen Formel (V)

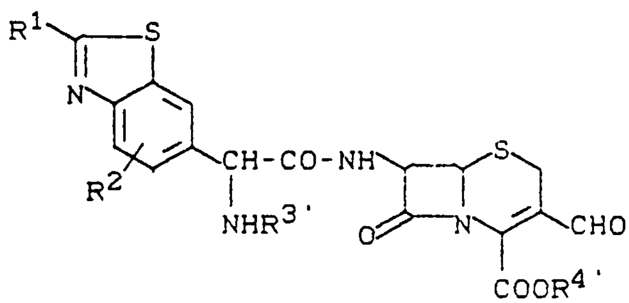

(V)

in welcher

$R^1$ und $R^2$ die oben angegebene Bedeutung haben,

$R^{3'}$ für eine in der $\beta$-Lactamchemie übliche Aminoschutzgruppe steht,

$R^4$ für eine in der $\beta$-Lactamchemie übliche Carboxyschutzgruppe steht

umsetzt,

oder, daß man

[C] Carbonsäuren der allgemeinen Formel (VI)

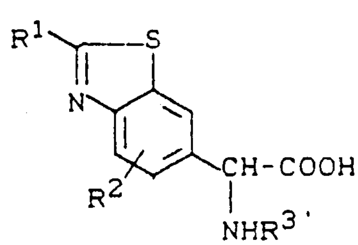

(VI)

in welcher

$R^1$ und $R^2$ die oben angegebene Bedeutung haben,

und

$R^{3'}$ für eine in der $\beta$-Lactamchemie übliche Aminoschutzgruppe steht,

nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester beispielsweise mit Dicyclohexylcarbodiimid (DCC) gegebenenfalls in Anwesenheit von N-Hydroxybenztriazol,
mit den Vinylcephalosporinaminen der allgemeinen Formel (VII)

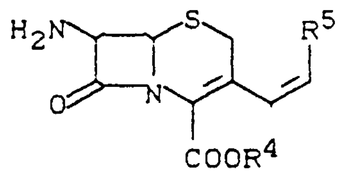

(VII),

in welcher
$R^4$ und $R^5$ die oben angegebene Bedeutung haben,
umsetzt,
dann gegebenenfalls Schutzgruppen abspaltet und die gewünschten Salze oder aus den Salzen die freien Säuren herstellt.

**5.** β-Lactam-Verbindungen der allgemeinen Formel (I) gemäß Anspruch 1 und deren Salze zur Verwendung in einem Verfahren zur therapeutischen Behandlung des menschlichen oder tierischen Körpers.

**6.** Verwendung von Verbindungen der Formel (I) nach Anspruch 1 zur Herstellung von Arzneimitteln.

**7.** Arzneimittel enthaltend β-Lactam-Verbindungen der allgemeinen Formel (I) nach Anspruch 1 und deren Salze.

**8.** Tierfutter und Tierfutterzusatzmittel enthaltend Verbindungen der Formel (I) nach Anspruch 1.

**Patentansprüche für folgende Vertragsstaaten : ES, GR**

**1.** Verfahren zur Herstellung von β-Lactam-Verbindungen der allgemeinen Formel (I)

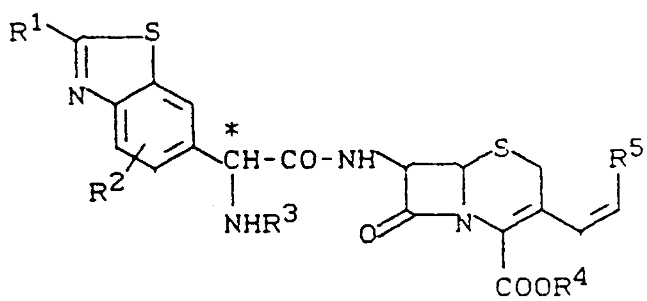

(I),

in welcher
$R^1$
- für Wasserstoff steht, oder
- für geradkettiges, verzweigtes oder cyclisches Alkyl oder Alkenyl mit bis zu 10 Kohlenstoffatomen steht, das substituiert sein kann durch Halogen, Hydroxy, Alkoxy mit bis zu 6 Kohlenstoffatomen, Cyano, Sulfo, Alkylsulfonyl mit bis zu 6 Kohlenstoffatomen, oder durch eine Gruppe der Formel $-NR^6R^7$,

worin
$R^6$, $R^7$
- gleich oder verschieden sind und
- Wasserstoff, Alkyl mit bis zu 8 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen, oder eine Aminoschutzgruppe bedeuten,

oder

- für Aryl mit 6 bis 12 Kohlenstoffatomen steht, das substituiert sein kann durch Halogen, Cyano, Nitro, Alkyl, Alkoxy, Alkylthio mit jeweils bis zu 6 Kohlenstoffatomen, Trifluormethyl oder Trifluormethoxy, oder
- für Halogen, Alkoxy, Alkylthio, Alkylsulfonyl mit jeweils bis zu 8 Kohlenstoffatomen, Mercapto, Hydroxy, Phenylthio, Phenyloxy, Benzylthio, Benzyloxy, Sulfo, Sulfamoyl, - $PO(OH)_2$, -$NHNH_2$, -NHOH, Guanidino, Amidino, Heterocyclyl, Heterocyclylthio oder Heterocyclyloxy mit Heterocyclen aus der Reihe Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxazolyl, Chinazolyl, Piperidinyl, Morpholinyl, Piperazinyl, Thiomorpholinyl, Furyl, Thienyl, Oxazolyl, Thiazolyl, Isoxazolyl, Thiadiazolyl, Triazolyl oder Tetrazolyl, die wiederum substituiert sein können durch Methyl, Methoxy oder Halogen,
- oder für eine Gruppe der Formel -$NR^6R^7$ steht,

worin

$R^6$, $R^7$
- die oben angegebene Bedeutung haben,

$R^2$
- für Wasserstoff, Alkyl, Alkoxy, Alkylthio mit jeweils bis zu 8 Kohlenstoffatomen, Trifluormethyl, Trifluormethoxy, Hydroxy, Mercapto, Nitro, Cyano, Halogen oder Amino steht,

$R^3$
- für Wasserstoff oder
- für eine in der $\beta$-Lactamchemie übliche Aminoschutzgruppe steht,

$R^4$
- für Wasserstoff,
- für eine in der $\beta$-Lactamchemie übliche Carboxyschutzgruppe oder
- für einen in vivo spaltbaren Ester steht

und

$R^5$
- für Halogen, Cyano, Trifluormethyl, Carboxy, Alkoxycarbonyl mit bis zu 8 Kohlenstoffatomen, Alkylsulfonyl, Alkylsulfonyloxy mit jeweils bis zu 8 Kohlenstoffatomen, Phenylsulfonyl, Tolylsulfonyl, Phenylsulfonyloxy, Tolylsulfonyloxy, Sulfamoyl, Dialkylsulfamoyl mit jeweils bis zu 6 Kohlenstoffatomen je Alkylgruppe, oder
- für Cycloalkyl mit 3 bis 8 Kohlenstoffatomen steht, das substituiert sein kann durch Alkyl, Alkoxy, Alkoxycarbonyl mit jeweils bis zu 6 Kohlenstoffatomen, Carboxy, Carbamoyl, Halogen, Cyano oder Phenyl, oder
- für Cycloalkylalkyl mit 3 bis 6 Ringgliedern und 4 bis 10 Kohlenstoffatomen, oder
- für geradkettiges oder verzweigtes Alkinyl mit bis zu 8 Kohlenstoffatomen steht, das substituiert sein kann, durch Aryl mit 6 bis 12 Kohlenstoffatomen, Carboxy, Alkoxycarbonyl mit bis zu 8 Kohlenstoffatomen, oder durch Halogen, oder
- für Aryl mit 6 bis 12 Kohlenstoffatomen steht, das substituiert sein kann durch Halogen, Cyano, Nitro, Trifluormethyl, Trifluormethoxy, Trifluormethylthio, Alkyl, Alkoxy oder Alkylthio mit jeweils bis zu 6 Kohlenstoffatomen, oder
- für einen heterocyclischen Rest der Reihe Thienyl, Furyl, Pyridyl, Pyridyloxid, Pyrimidyl, Pyrazinyl, Pyridazinyl, Pyrazolyl, Imidazolyl, Triazolyl, Tetrazolyl, Oxazolyl, Thiazolyl oder Thiadiazolyl steht, wobei die heterocyclischen Reste durch Alkyl, Alkoxy mit jeweils bis zu 6 Kohlenstoffatomen, Halogen, Nitro oder Cyano substituiert sein können, oder
- für eine Gruppe der Formel -$NR^8R^9$, -$CH_2$-$R^{10}$ oder -S-$R^{11}$ steht,

worin

$R^8$, $R^9$
- gleich oder verschieden sind und
- Wasserstoff, Alkyl mit bis zu 6 Kohlenstoffatomen, Aryl mit 6 bis 12 Kohlenstoffatomen, Aralkyl mit 7 bis 14 Kohlenstoffatomen, Acyl mit bis zu 7 Kohlenstoffatomen oder eine Aminoschutzgruppe bedeuten, oder worin

$R^8$, $R^9$

- gemeinsam mit dem Stickstoffatom einen 5 bis 7-gliedrigen Ring bilden, der durch O, S, NH, N-Alkyl mit bis zu 4 Kohlenstoffatomen oder N-Phenyl unterbrochen sein kann,

$R^{10}$

- einen heterocyclischen Rest der Reihe Thienyl, Furyl, Pyridyl, Pyridyloxid, Pyrimidyl, Pyrazinyl, Pyridazinyl, Pyrazolyl, Imidazolyl, Triazolyl, Tetrazolyl, Oxazolyl, Thiazolyl oder Thiadiazolyl bedeutet, wobei die heterocyclischen Reste durch Alkyl, Alkoxy, Alkylthio mit jeweils bis zu 6 Kohlenstoffatomen, Halogen, Nitro, Cyano, Trifluormethyl, Trifluormethoxy substituiert sein können,

und

$R^{11}$

- einen heterocyclischen Rest der Reihe Thienyl, Furyl, Pyridyl, Pyridyloxid, Pyrimidyl, Imidazolyl, Triazolyl, Oxazolyl oder Thiadiazolyl bedeutet, wobei die heterocyclischen Reste durch Alkyl, Alkoxy mit jeweils bis zu 6 Kohlenstoffatomen, Halogen, Trifluormethyl, Trifluormethoxy, Nitro oder Cyano substituiert sein können,

und deren Salze,
dadurch gekennzeichnet, daß man
[A] substituierte Cephalosporinverbindungen der allgemeinen Formel (II)

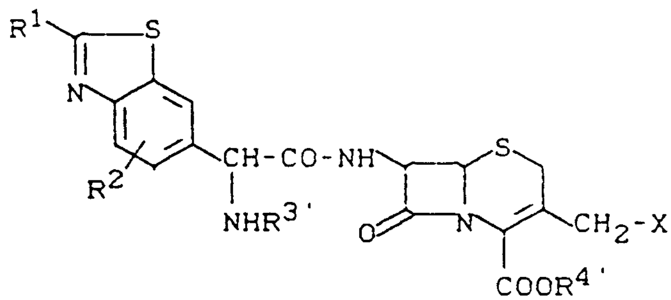

(II)

in welcher

$R^1$ und $R^2$ die oben angegebene Bedeutung hat,
$R^{3'}$ für eine in der $\beta$-Lactamchemie übliche Aminoschutzgruppe steht,
$R^4$ für eine in der $\beta$-Lactamchemie übliche Carboxyschutzgruppe steht und

X

- für eine Gruppe der Formel

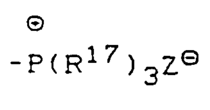 , 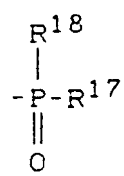 oder 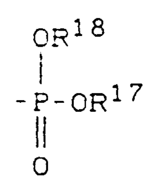

steht,
wobei

$R^{17}$ und $R^{18}$ gleich oder verschieden sind und Alkyl, Phenyl oder Tolyl bedeuten und

$Z^{\ominus}$

- ein Halogenidanion, bevorzugt Chlorid, Bromid oder Iodid bedeutet,

in inerten Lösemitteln in Anwesenheit von Basen, mit Aldehyden der allgemeinen Formel (III)

$R^5$-CHO (III)

in welcher

$R^5$ die oben angegebene Bedeutung hat

umsetzt,

oder daß man

[B] Phosphoniumverbindungen der allgemeinen Formel (IV)

$R^5$-$CH_2$-X (IV)

in welcher

$R^5$ die oben angegebene Bedeutung hat und

X - für eine Gruppe der Formel

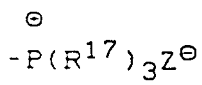 , 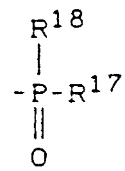 oder 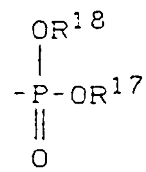

steht,

wobei

$R^{17}$ und $R^{18}$ gleich oder verschieden sind und Alkyl, Phenyl oder Tolyl bedeuten und

$Z^{\ominus}$ - ein Halogenidanion, bevorzugt Chlorid, Bromid oder Iodid bedeutet,

in inerten Lösemitteln in Anwesenheit von Basen, mit Cephalosporinaldehyden der allgemeinen Formel (V)

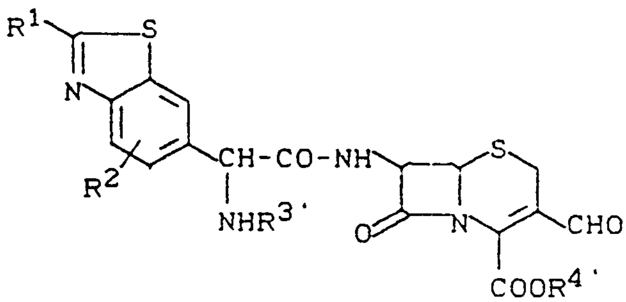

(V)

in welcher

$R^1$ und $R^2$ die oben angegebene Bedeutung haben,

$R^{3'}$ für eine in der $\beta$-Lactamchemie übliche Aminoschutzgruppe steht,

$R^{4'}$ für eine in der $\beta$-Lactamchemie übliche Carboxyschutzgruppe steht

umsetzt,

oder, daß man

[C] Carbonsäuren der allgemeinen Formel (VI)

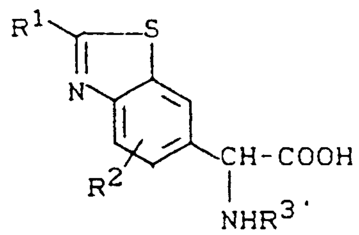

(VI)

in welcher

$R^1$ und $R^2$ die oben angegebene Bedeutung haben, und

$R^{3'}$ für eine in der β-Lactamchemie übliche Aminoschutzgruppe steht,

nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester beispielsweise mit Dicyclohexylcarbodiimid (DCC) gegebenenfalls in Anwesenheit von N-Hydroxybenztriazol,

mit den Vinylcephalosporinaminen der allgemeinen Formel (VII)

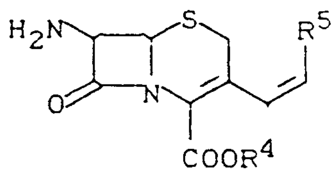

(VII),

in welcher

$R^4$ und $R^5$ die oben angegebene Bedeutung haben,

umsetzt,

dann gegebenenfalls Schutzgruppen abspaltet und die gewünschten Salze oder aus den Salzen die freien Säuren herstellt.

**2.** Verwendung von Verbindungen der Formel (I) nach Anspruch 1 zur Herstellung von Arzneimitteln.

**Claims**

**Claims for the following Contracting States : AT, BE, CH, DE, FR, GB, IT, LI, NL, SE**

**1.** β-Lactam compounds of the general formula (I)

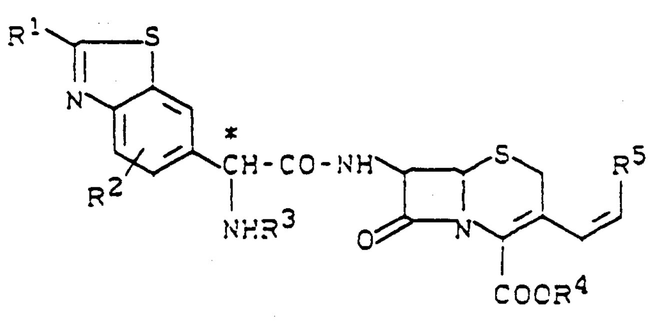

(I),

in which

$R^1$ represents hydrogen, or
represents straight-chain, branched or cyclic alkyl or alkenyl which has up to 10 carbon atoms and can be substituted by halogen, hydroxyl, alkoxy with up to 6 carbon atoms, cyano, sulpho, alkylsulphonyl with up to 6 carbon atoms, or by a group of the formula $-NR^6R^7$,
wherein
$R^6$ and $R^7$ are identical or different and denote hydrogen, alkyl with up to 8 carbon atoms, aryl with 6 to 12 carbon atoms, aralkyl with 7 to 14 carbon atoms, acyl with up to 7 carbon atoms or an amino-protective group,
or
$R^1$ represents aryl which has 6 to 12 carbon atoms and can be substituted by halogen, cyano, nitro, alkyl, alkoxy or alkylthio with in each case up to 6 carbon atoms, trifluoromethyl or trifluoromethoxy, or
represents halogen, alkoxy, alkylthio or alkylsulphonyl with in each case up to 8 carbon atoms, mercapto, hydroxyl, phenylthio, phenyloxy, benzylthio, benzyloxy, sulpho, sulphamoyl, $-PO(OH)_2$, $-NHNH_2$, -NHOH, guanidino, amidino, heterocyclyl, heterocyclylthio or heterocyclyloxy, with heterocyclic radicals from the series comprising pyrrolyl, pyrrolidinyl, pyrazolyl, imidazolyl, pyridyl, pyridazinyl, pyrimidyl, pyrazinyl, quinolyl, isoquinolyl, indolyl, quinoxazolyl, quinazolyl, piperidinyl, morpholinyl, piperazinyl, thiomorpholinyl, furyl, thienyl, oxazolyl, thiazolyl, isoxazolyl, thiadiazolyl, triazolyl and tetrazolyl, which can in turn be substituted by methyl, methoxy or halogen,
or represents a group of the formula $-NR^6R^7$,
wherein
$R^6$ and $R^7$ have the abovementioned meaning,
$R^2$ represents hydrogen, alkyl, alkoxy or alkylthio with in each case up to 8 carbon atoms, trifluoromethyl, trifluoromethoxy, hydroxyl, mercapto, nitro, cyano, halogen or amino,
$R^3$ represents hydrogen, or
represents an amino-protective group customary in $\beta$-lactam chemistry,
$R^4$ represents hydrogen, or
represents a carboxyl-protective group customary in $\beta$-lactam chemistry, or
represents an ester which can be split off in vivo,
and
$R^5$ represents halogen, cyano, trifluoromethyl, carboxyl, alkoxycarbonyl with up to 8 carbon atoms, alkylsulphonyl or alkylsulphonyloxy with in each case up to 8 carbon atoms, phenylsulphonyl, tolylsulphonyl, phenylsulphonyloxy, tolylsulphonyloxy, sulphamoyl or dialkylsulphamoyl with in each case up to 6 carbon atoms per alkyl group, or
represents cycloalkyl which has 3 to 8 carbon atoms and can be substituted by alkyl, alkoxy or alkoxycarbonyl with in each case up to 6 carbon atoms, carboxyl, carbamoyl, halogen, cyano or phenyl, or
represents cycloalkylalkyl with 3 to 6 ring members and 4 to 10 carbon atoms, or
represents straight-chain or branched alkinyl which has up to 8 carbon atoms and can be substituted by aryl with 6 to 12 carbon atoms, carboxyl or alkoxycarbonyl with up to 8 carbon atoms, or by halogen, or
represents aryl which has 6 to 12 carbon atoms and can be substituted by halogen, cyano, nitro, trifluoromethyl, trifluoromethoxy, trifluoromethylthio or alkyl, alkoxy or alkylthio with in each case up to 6 carbon atoms, or
represents a heterocyclic radical from the series comprising thienyl, furyl, pyridyl, pyridyl oxide, pyrimidyl, pyrazinyl, pyridazinyl, pyrazolyl, imidazolyl, triazolyl, tetrazolyl, oxazolyl, thiazolyl or thiadiazolyl, it being possible for the heterocyclic radicals to be substituted by alkyl or alkoxy with in each case up to 6 carbon atoms, halogen, nitro or cyano, or represents a group of the formula $-NR^8R^9$, $-CH_2-R^{10}$ or $-S-R^{11}$,
wherein
$R^8$ and $R^9$ are identical or different and denote hydrogen, alkyl with up to 6 carbon atoms, aryl with 6 to 12 carbon atoms, aralkyl with 7 to 14 carbon atoms, acyl with up to 7

carbon atoms or an amino-protective group, or wherein

$R^8$ and $R^9$, together with the nitrogen atom, form a 5- to 7-membered ring, which can be interrupted by O, S, NH, N-alkyl with up to 4 carbon atoms or N-phenyl,

$R^{10}$ denotes a heterocyclic radical from the series comprising thienyl, furyl, pyridyl, pyridyl oxide, pyrimidyl, pyrazinyl, pyridazinyl, pyrazolyl, imidazolyl, triazolyl, tetrazolyl, oxazolyl, thiazolyl or thiadiazolyl, it being possible for the heterocyclic radicals to be substituted by alkyl, alkoxy or alkylthio with in each case up to 6 carbon atoms, halogen, nitro, cyano, trifluoromethyl or trifluoromethoxy,

and

$R^{11}$ denotes a heterocyclic radical from the series comprising thienyl, furyl, pyridyl, pyridyl oxide, pyrimidyl, imidazolyl, triazolyl, oxazolyl and thiadiazolyl, it being possible for the heterocyclic radicals to be substituted by alkyl or alkoxy with in each case up to 6 carbon atoms, halogen, trifluoromethyl, trifluoromethoxy, nitro or cyano,

and salts thereof.

**2.** Compounds of the general formula (I) according to Claim 1, in which

$R^1$ represents hydrogen, or

represents straight-chain, branched or cyclic alkyl, which can be substituted by fluorine, chlorine, bromine, hydroxyl, alkoxy or cyano, or by a group of the formula $-NR^6R^7$,

wherein

$R^6$ and $R^7$ are identical or different and represent hydrogen, alkyl with up to 6 carbon atoms, phenyl, benzyl or an amino-protective group from the series comprising 4-methoxyphenyl, 3,4-dimethoxyphenyl, 4-methoxymethoxyphenyl, 4-[(2-methoxyethoxy)-methoxy]phenyl, benzyl, 4-methoxybenzyl, 2,4-dimethoxybenzyl, 3,4-dimethoxybenzyl, 2,4,6-trimethoxybenzyl, methoxy-(4-methoxyphenyl)methyl, trimethylsilyl, triethylsilyl, tert-butyl-dimethylsilyl, tert-butyl-diphenylsilyl, methoxycarbonylmethyl, tert-butoxycarbonylmethyl and 2-hydroxy-2-phenylethyl,

or

$R^1$ represents phenyl which is optionally substituted by fluorine, chlorine, bromine, alkyl or alkoxy with up to 6 carbon atoms, trifluoromethyl, trifluoromethoxy or cyano, or represents fluorine, chlorine, bromine, alkoxy, alkylthio or alkylsulphonyl with in each case up to 6 carbon atoms, mercapto, hydroxyl, phenylthio, phenyloxy or sulphamoyl, or represents a group of the formula $-NR^6R^7$,

wherein

$R^6$ and $R^7$ have the abovementioned meaning,

$R^2$ represents hydrogen, methyl, methoxy, trifluoromethyl, trifluoromethoxy, cyano, fluorine, chlorine or bromine,

$R^3$ represents hydrogen, or

represents an amino-protective group from the series comprising: 4-methoxyphenyl, 3,4-dimethoxyphenyl, 4-methoxymethoxyphenyl, 4-[(2-methoxyethoxy)methoxy]-phenyl, benzyl, 4-methoxybenzyl, 2,4-dimethoxybenzyl, 3,4-dimethoxybenzyl, 2,4,6-trimethoxybenzyl, methoxy-(4-methoxyphenyl)methyl, trimethylsilyl, triethylsilyl, tert-butyl-dimethylsilyl, tert-butyl-diphenylsilyl, methoxycarbonylmethyl, tert-butoxycarbonylmethyl, 2-hydroxy-2-phenylethyl, tert-butoxycarbonyl, 1-methyl-2-benzoyl-vinyl and 1-methyl-2-methoxy-vinyl,

$R^4$ represents hydrogen, or

represents methyl, ethyl, tert-butyl, 2-chloroethyl, 2,2,2-trichloroethyl, cyanoethyl, diphenylmethyl, triphenylmethyl, acetoxymethyl, allyl, benzyl, 4-methoxybenzyl, 2,4-dimethoxybenzyl, 1-phenoxyethyl, 2-methyl-2-propenyl, 4-nitrobenzyl, 2-nitrobenzyl, trimethylsilylethyl or tert-butyl-dimethylsilylethyl, or represents a radical of the formula

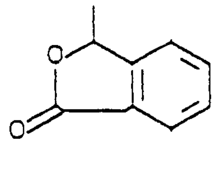 , 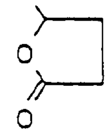 , 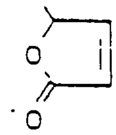 ,

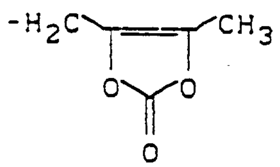 ,

$-CH_2-OCO-C(CH_3)_3$, $-CH(CH_3)-OCOOC_2H_5$ or $-CH_2-OCOCH_3$
and

$R^5$ represents fluorine, chlorine, bromine, cyano, trifluoromethyl, carboxyl, alkoxycarbonyl with up to 6 carbon atoms, alkylsulphonyl or alkylsulphonyloxy with in each case up to 6 carbon atoms, phenylsulphonyl, tolylsulphonyl, phenylsulphonyl or tolylsulphonyloxy, or
represents cyclopropyl, cyclopentyl or cyclohexyl, which can be substituted by alkyl, alkoxy or alkoxycarbonyl with in each case up to 4 carbon atoms, fluorine, chlorine, bromine or phenyl, or
represents cyclopropylmethyl, cyclopentylmethyl or cyclohexylmethyl, or
represents straight-chain or branched alkinyl which has up to 6 carbon atoms and can be substituted by phenyl, carboxyl or alkoxycarbonyl with up to 6 carbon atoms, or by fluorine, chlorine or bromine, or
represents phenyl, which can be substituted by fluorine, chlorine, bromine, cyano, nitro, trifluoromethyl, trifluoromethoxy or alkyl or alkoxy with up to 4 carbon atoms, or
represents a heterocyclic radical from the series comprising thienyl, furyl, pyridyl, pyridyl oxide, pyrimidyl, pyrazolyl, triazolyl, tetrazolyl, oxazolyl, thiazolyl and thiadiazolyl, it being possible for the heterocyclic radicals to be substituted by alkyl or alkoxy with in each case up to 4 carbon atoms, fluorine, chlorine, bromine or nitro, or
represents a group of the formula $-NR^8R^9$, $-CH_2-R^{10}$ or $-S-R^{11}$,
wherein

$R^8$ and $R^9$ are identical or different and denote hydrogen, alkyl with up to 4 carbon atoms, phenyl, benzyl or an amino-protective group from the series comprising 4-methoxyphenyl, 3,4-dimethoxyphenyl, 4-methoxymethoxyphenyl, 4-[(2-methoxyethoxy)-methoxy]phenyl, benzyl, 4-methoxybenzyl, 2,4-dimethoxybenzyl, 3,4-dimethoxybenzyl, 2,4,6-trimethoxybenzyl, methoxy-(4-methoxyphenyl)methyl, trimethylsilyl, triethylsilyl, tert-butyl-dimethylsilyl, tert-butyl-diphenylsilyl, methoxycarbonylmethyl, tert-butoxycarbonylmethyl and 2-hydroxy-2-phenylethyl or wherein

$R^8$ and $R^9$, together with the nitrogen atom, form a pyrrolidine, piperidine, morpholine, thiomorpholine, piperazine or 4-methyl- or 4-phenyl-piperazine ring,

$R^{10}$ denotes a heterocyclic ring from the series comprising thienyl, furyl, pyridyl, pyridyl oxide, pyrimidyl, pyrazolyl, triazolyl, tetrazolyl, oxazolyl, thiazolyl and thiadiazolyl, it being possible for the heterocyclic radicals to be substituted by alkyl or alkoxy with in each case up to 4 carbon atoms, fluorine, chlorine, bromine or nitro,
and

$R^{11}$ denotes a heterocyclic ring from the series comprising thienyl, furyl, pyridyl, pyridyl oxide, pyrimidyl, pyrazolyl, oxazolyl and thiadiazolyl, it being possible for the heterocyclic radicals to be substituted by alkyl or alkoxy with in each case up to 4 carbon atoms, fluorine, chlorine, bromine or nitro,

and salts thereof.

**3.** Compounds of the general formula (I) according to Claim 1, in which

$R^1$ represents hydrogen or cyclopropyl, or represents straight-chain or branched alkyl which has up to 6 carbon atoms and can be substituted by fluorine, chlorine, methoxy, cyano, dimethylamino or diethylamino, or
represents phenyl, which can be substituted by fluorine, chlorine, methyl, methoxy, trifluoromethyl, amino or trifluoromethoxy, or
represents chlorine, alkoxy or alkylsulphonyl with in each case up to 4 carbon atoms, mercapto, hydroxyl or
represents a group of the formula $-NHR^6$,
wherein

$R^6$ denotes hydrogen, alkyl with up to 4 carbon atoms, phenyl, benzyl or an amino-protective group from the series comprising 4-methoxyphenyl, 4-methoxybenzyl, 2,4-dimethoxybenzyl, 3,4-dimethoxybenzyl and tert-butyl-dimethylsilyl,

$R^2$ represents hydrogen,

$R^3$ represents hydrogen, or
represents 4-methoxyphenyl, 4-methoxybenzyl, 2,4-dimethoxybenzyl, 3,4-dimethoxybenzyl, tert-butyl-dimethylsilyl, 1-methyl-2-methoxy-vinyl (MMV) or tert-butoxy-carbonyl (Boc), or

$R^4$ represents hydrogen, or
represents methyl, ethyl, tert-butyl, diphenylmethyl, 2,2,2-trichloroethyl, allyl, acetoxymethyl, 4-nitrobenzyl, 2-nitrobenzyl, 4-methoxybenzyl, benzyl or trimethylsilylethyl, or represents a radical of the formula

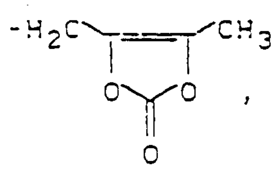 ,

$-CH(CH_3)-OCOOC_2H_5$
or $-CH_2-OCO-C(CH_3)_3$
and

$R^5$ represents chlorine, bromine, trifluoromethyl, carboxyl, alkoxycarbonyl or alkylsulphonyloxy with in each case up to 4 carbon atoms, phenylsulphonyloxy, tolylsulphonyloxy, cyclopropyl, cyclopentyl, cyclohexyl, cyclopropylmethyl or cyclopentylmethyl, or
represents alkinyl which has up to 4 carbon atoms and can be substituted by phenyl, carboxyl or alkoxycarbonyl with up to 4 carbon atoms, or represents phenyl, or
represents a radical of the formula

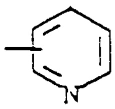 , 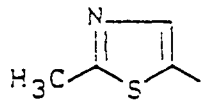 , 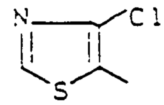 , 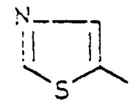 ,

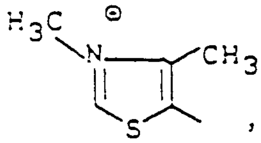 , 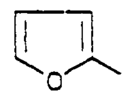 , 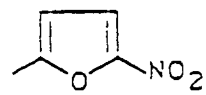 , 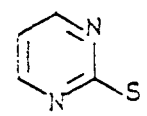 ,

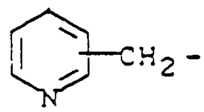 , 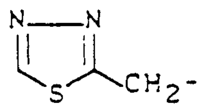 , 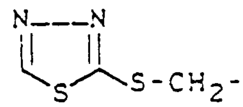 ,

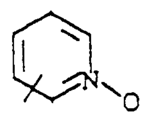 , 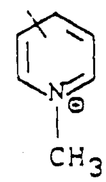 , 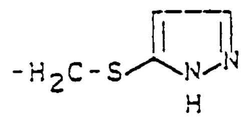

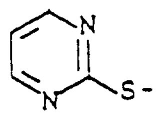 or 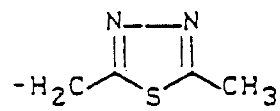

and salts thereof.

**4.** Process for the preparation of $\beta$-lactam compounds of the general formula (I)

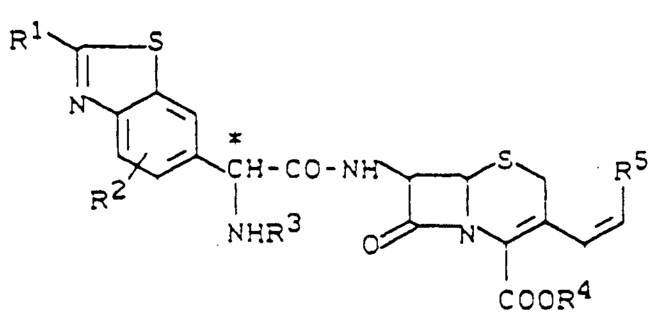 (I),

according to Claim 1, and salts thereof, characterized in that

[A] substituted cephalosporin compounds of the general formula (II)

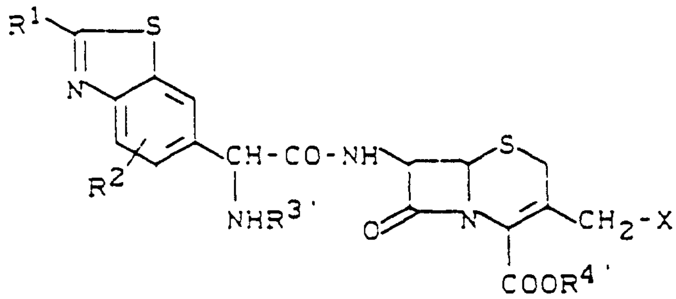

(II)

in which

| | |
|---|---|
| $R^1$ and $R^2$ | have the abovementioned meaning, |
| $R^{3'}$ | represents an amino-protective group customary in $\beta$-lactam chemistry, |
| $R^{4'}$ | represents a carboxyl-protective group customary in $\beta$-lactam chemistry and |
| X | represents a group of the formula |

$-\overset{\ominus}{P}(R^{17})_3Z^{\ominus}$ , 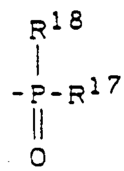 or 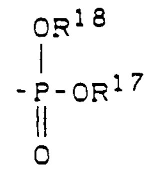

wherein

| | |
|---|---|
| $R^{17}$ and $R^{18}$ | are identical or different and denote alkyl, phenyl or tolyl and |
| $Z^{\ominus}$ | denotes a halide anion, preferably chloride, bromide or iodide, |

are reacted with aldehydes of the general formula (III)

$R^5$-CHO (III)

in which

$R^5$ has the abovementioned meaning,

in inert solvents in the presence of bases,

or in that

[B] phosphonium compounds of the general formula (IV)

$R^5$-$CH_2$-X (IV)

in which

| | |
|---|---|
| $R^5$ | has the abovementioned meaning and |
| X | represents a group of the formula |

$-\overset{\ominus}{P}(R^{17})_3Z^{\ominus}$ , 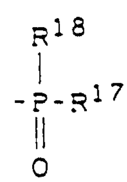 or 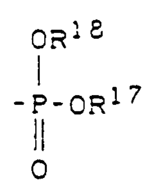

wherein

$R^{17}$ and $R^{18}$ are identical or different and denote alkyl, phenyl or tolyl and

$Z^{\ominus}$ denotes a halide anion, preferably chloride, bromide or iodide,

are reacted with cephalosporin aldehydes of the general formula (V)

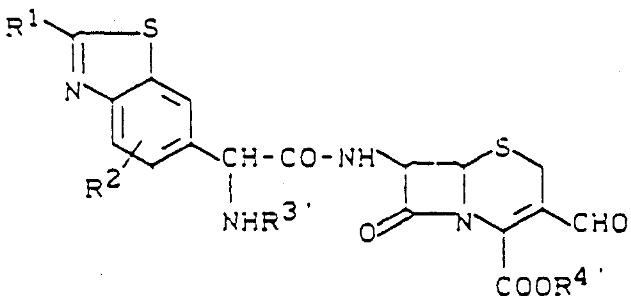

(V)

in which

$R^1$ and $R^2$ have the abovementioned meaning,

$R^{3'}$ represents an amino-protective group customary in $\beta$-lactam chemistry and

$R^{4'}$ represents a carboxyl-protective group customary in $\beta$-lactam chemistry,

in inert solvents in the presence of bases,

or in that

[C] carboxylic acids of the general formula (VI)

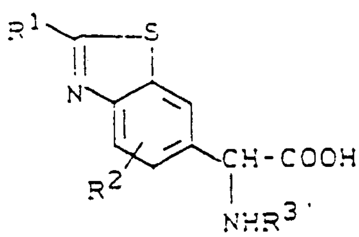

(VI)

in which

$R^1$ and $R^2$ have the abovementioned meaning and

$R^3$ represents an amino-protective group customary in $\beta$-lactam chemistry, after activation of the carboxyl group by conversion into a mixed anhydride, for example with ethyl chloroformate or isobutyl chloroformate or methanesulphonyl chloride, or by conversion into the acid halide, or by conversion into an activated ester, for example with dicyclohexylcarbodiimide (DCC), if appropriate in the presence of N-hydroxybenzotriazole,

are reacted with the vinylcephalosporinamines of the general formula (VII)

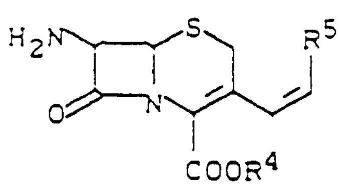

(VII),

in which

$R^4$ and $R^5$ have the abovementioned meaning,

and, if appropriate, the protective groups are split off and the desired salts are prepared or the free

acids are prepared from the salts.

**5.** $\beta$-Lactam compounds of the general formula (I) according to Claim 1 and salts thereof, for use in a process for the therapeutic treatment of the human or animal body.

**6.** Use of compounds of the formula (I) according to Claim 1 for the preparation of medicaments.

**7.** Medicaments containing $\beta$-lactam compounds of the general formula (I) according to Claim 1 and salts thereof.

**8.** Animal feeds and animal feed additives containing compounds of the formula (I) according to Claim 1.

**Claims for the following Contracting States : ES, GR**

**1.** Process for the preparation of $\beta$-lactam compounds of the general formula (I)

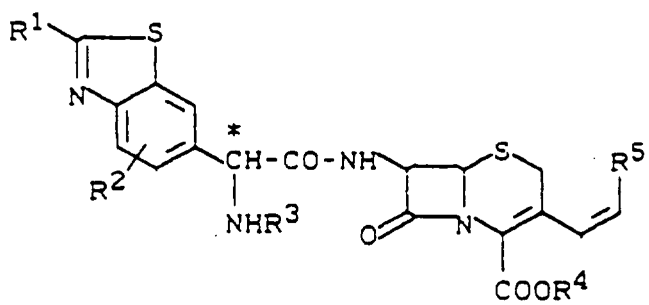

(I),

in which

| | |
|---|---|
| $R^1$ | represents hydrogen, or represents straight-chain, branched or cyclic alkyl or alkenyl which has up to 10 carbon atoms and can be substituted by halogen, hydroxyl, alkoxy with up to 6 carbon atoms, cyano, sulpho, alkylsulphonyl with up to 6 carbon atoms or by a group of the formula $-NR^6R^7$, wherein |
| $R^6$ and $R^7$ | are identical or different and denote hydrogen, alkyl with up to 8 carbon atoms, aryl with 6 to 12 carbon atoms, aralkyl with 7 to 14 carbon atoms, acyl with up to 7 carbon atoms or an amino-protective group, or |
| $R^1$ | represents aryl which has 6 to 12 carbon atoms and can be substituted by halogen, cyano, nitro, alkyl, alkoxy or alkylthio with in each case up to 6 carbon atoms, trifluoromethyl or trifluoromethoxy, or represents halogen, alkoxy, alkylthio or alkylsulphonyl with in each case up to 8 carbon atoms, mercapto, hydroxyl, phenylthio, phenyloxy, benzylthio, benzyloxy, sulpho, sulphamoyl, $-PO(OH)_2$, $-NHNH_2$, -NHOH, guanidino, amidino, heterocyclyl, heterocyclylthio or heterocyclyloxy, with heterocyclic radicals from the series comprising pyrrolyl, pyrrolidinyl, pyrazolyl, imidazolyl, pyridyl, pyridazinyl, pyrimidyl, pyrazinyl, quinolyl, isoquinolyl, indolyl, quinoxazolyl, quinazolyl, piperidinyl, morpholinyl, piperazinyl, thiomorpholinyl, furyl, thienyl, oxazolyl, thiazolyl, isoxazolyl, thiadiazolyl, triazolyl and tetrazolyl, which can in turn be substituted by methyl, methoxy or halogen, or represents a group of the formula $-NR^6R^7$, wherein |
| $R^6$ and $R^7$ | have the abovementioned meaning, |
| $R^2$ | represents hydrogen, alkyl, alkoxy or alkylthio with in each case up to 8 carbon atoms, trifluoromethyl, trifluoromethoxy, hydroxyl, mercapto, nitro, cyano, halogen or amino, |
| $R^3$ | represents hydrogen, or |

represents an amino-protective group customary in β-lactam chemistry,

$R^4$ represents hydrogen, or
represents a carboxyl-protective group customary in β-lactam chemistry, or
represents an ester which can be split off in vivo,
and

$R^5$ represents halogen, cyano, trifluoromethyl, carboxyl, alkoxycarbonyl with up to 8 carbon atoms, alkylsulphonyl or alkylsulphonyloxy with in each case up to 8 carbon atoms, phenylsulphonyl, tolylsulphonyl, phenylsulphonyloxy, tolylsulphonyloxy, sulphamoyl or dialkylsulphamoyl with in each case up to 6 carbon atoms per alkyl group, or
represents cycloalkyl which has 3 to 8 carbon atoms and can be substituted by alkyl, alkoxy or alkoxycarbonyl with in each case up to 6 carbon atoms, carboxyl, carbamoyl, halogen, cyano or phenyl, or
represents cycloalkylalkyl with 3 to 6 ring members and 4 to 10 carbon atoms, or
represents straight-chain or branched alkinyl which has up to 8 carbon atoms and can be substituted by aryl with 6 to 12 carbon atoms, carboxyl or alkoxycarbonyl with up to 8 carbon atoms, or by halogen, or
represents aryl which has 6 to 12 carbon atoms and can be substituted by halogen, cyano, nitro, trifluoromethyl, trifluoromethoxy, trifluoromethylthio or alkyl, alkoxy or alkylthio with in each case up to 6 carbon atoms, or
represents a heterocyclic radical from the series comprising thienyl, furyl, pyridyl, pyridyl oxide, pyrimidyl, pyrazinyl, pyridazinyl, pyrazolyl, imidazolyl, triazolyl, tetrazolyl, oxazolyl, thiazolyl or thiadiazolyl, it being possible for the heterocyclic radicals to be substituted by alkyl or alkoxy with in each case up to 6 carbon atoms, halogen, nitro or cyano, or represents a group of the formula $-NR^8R^9$, $-CH_2-R^{10}$ or $-S-R^{11}$,
wherein

$R^8$ and $R^9$ are identical or different and denote hydrogen, alkyl with up to 6 carbon atoms, aryl with 6 to 12 carbon atoms, aralkyl with 7 to 14 carbon atoms, acyl with up to 7 carbon atoms or an amino-protective group, or wherein

$R^8$ and $R^9$, together with the nitrogen atom, form a 5- to 7-membered ring, which can be interrupted by O, S, NH, N-alkyl with up to 4 carbon atoms or N-phenyl,

$R^{10}$ denotes a heterocyclic radical from the series comprising thienyl, furyl, pyridyl, pyridyl oxide, pyrimidyl, pyrazinyl, pyridazinyl, pyrazolyl, imidazolyl, triazolyl, tetrazolyl, oxazolyl, thiazolyl or thiadiazolyl, it being possible for the heterocyclic radicals to be substituted by alkyl, alkoxy or alkylthio with in each case up to 6 carbon atoms, halogen, nitro, cyano, trifluoromethyl or trifluoromethoxy,
and

$R^{11}$ denotes a heterocyclic radical from the series comprising thienyl, furyl, pyridyl, pyridyl oxide, pyrimidyl, imidazolyl, triazolyl, oxazolyl and thiadiazolyl, it being possible for the heterocyclic radicals to be substituted by alkyl or alkoxy with in each case up to 6 carbon atoms, halogen, trifluoromethyl, trifluoromethoxy, nitro or cyano,

and salts thereof, characterized in that

[A] substituted cephalosporin compounds of the general formula (II)

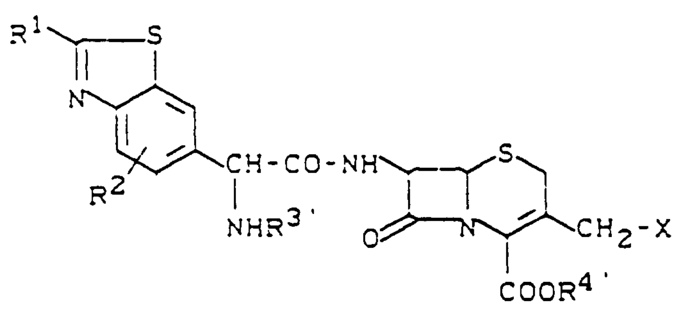

(II)

in which

$R^1$ and $R^2$ have the abovementioned meaning,
$R^{3'}$ represents an amino-protective group customary in $\beta$-lactam chemistry,
$R^{4'}$ represents a carboxyl-protective group customary in $\beta$-lactam chemistry and
X represents a group of the formula

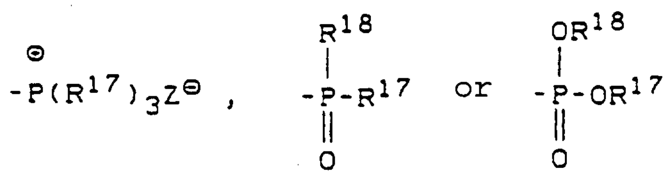

wherein
$R^{17}$ and $R^{18}$ are identical or different and denote alkyl, phenyl or tolyl and
$Z^{\ominus}$ denotes a halide anion, preferably chloride, bromide or iodide,
are reacted with aldehydes of the general formula (III)

$R^5$-CHO (III)

in which
$R^5$ has the abovementioned meaning,
in inert solvents in the presence of bases,
or in that
[B] phosphonium compounds of the general formula (IV)

$R^5-CH_2-X$ (IV)

in which
$R^5$ has the abovementioned meaning and
X represents a group of the formula

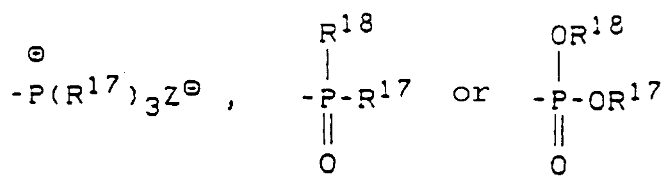

wherein
$R^{17}$ and $A^{18}$ are identical or different and denote alkyl, phenyl or tolyl and
$Z^{\ominus}$ denotes a halide anion, preferably chloride, bromide or iodide,
are reacted with cephalosporin aldehydes of the general formula (V)

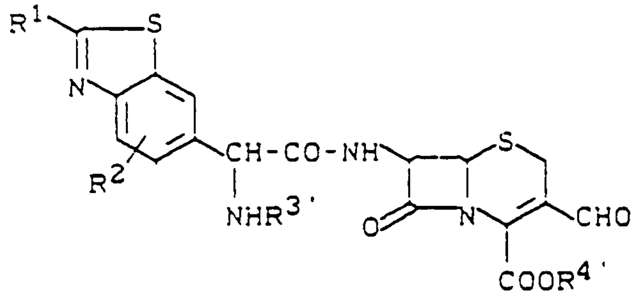

(V)

in which

$R^1$ and $R^2$ have the abovementioned meaning,

$R^{3'}$ represents an amino-protective group customary in β-lactam chemistry and

$R^{4'}$ represents a carboxyl-protective group customary in β-lactam chemistry, in inert solvents in the presence of bases,

or in that

[C] carboxylic acids of the general formula (VI)

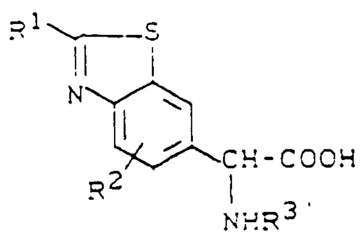

(VI)

in which

$R^1$ and $R^2$ have the abovementioned meaning and

$R^3$ represents an amino-protective group customary in β-lactam chemistry, after activation of the carboxyl group by conversion into a mixed anhydride, for example with ethyl chloroformate or isobutyl chloroformate or methanesulphonyl chloride, or by conversion into the acid halide, or by conversion into an activated ester, for example with dicyclohexylcarbodiimide (DCC), if appropriate in the presence of N-hydroxybenzotriazole,

are reacted with the vinylcephalosporinamines of the general formula (VII)

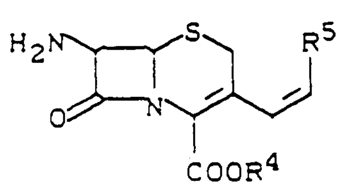

(VII),

in which

$R^4$ and $R^5$ have the abovementioned meaning,

and, if appropriate, the protective groups are split off and the desired salts are prepared or the free acids are prepared from the salts.

**2.** Use of compounds of the formula (I) according to Claim 1 for the preparation of medicaments.

**Revendications**

**Revendications pour les Etats contractants suivants : AT, BE, CH, DE, FR, GB, IT, LI, NL, SE**

**1.** Composés de β-lactame de formule générale (I)

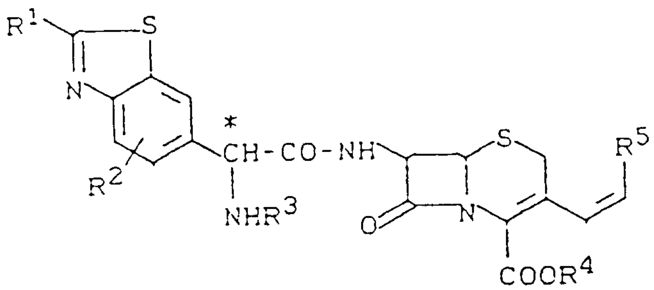

(I),

dans laquelle

$R^1$

- représente l'hydrogène ou
- un groupe alkyle ou alcényle à chaîne droite, à chaîne ramifiée ou cyclique ayant jusqu'à 10 atomes de carbone, qui peut être substitué par un halogène, un radical hydroxy, alkoxy ayant jusqu'à 6 atomes de carbone, cyano, sulfo, alkylsulfonyle ayant jusqu'à 6 atomes de carbone ou par un groupe de formule $-NR^6R^7$,

dans laquelle

$R^6$, $R^7$

- sont identiques ou différents et représentent
- l'hydrogène, un groupe alkyle ayant jusqu'à 8 atomes de carbone, aryle de 6 à 12 atomes de carbone, aralkyle de 7 à 14 atomes de carbone, acyle ayant jusqu'à 7 atomes de carbone ou un groupe protégeant la fonction amino, ou bien
- un groupe aryle ayant 6 à 12 atomes de carbone, qui peut être substitué par un radical halogéno, cyano, nitro, alkyle, alkoxy, alkylthio, chacun ayant jusqu'à 6 atomes de carbone, un radical trifluorométhyle ou trifluorométhoxy, ou bien
- un halogène, un groupe alkoxy, alkylthio, alkylsulfonyle, chacun ayant jusqu'à 8 atomes de carbone, un radical mercapto, hydroxy, phénylthio, phényloxy, benzylthio, benzyloxy, sulfo, sulfamoyle, $-PO(OH)_2$, $-NHNH_2$, -NHOH, guanidino, amidino, hétérocyclyle, hétérocyclylthio ou hétérocyclyloxy avec des hétérocycles de la série pyrrolyle, pyrrolidinyle, pyrazolyle, imidazolyle, pyridyle, pyridazinyle, pyrimidyle, pyrazinyle, quinolyle, isoquinolyle, indolyle, quinoxazolyle, quinazolyle, pipéridinyle, morpholinyle, pipérazinyle, thiomorpholinyle, furyle, thiényle, oxazolyle, thiazolyle, isoxazolyle, thiadiazolyle, triazolyle ou tétrazolyle, qui peuvent être substitués quant à eux par un radical méthyle, méthoxy ou halogéno,
- ou bien un groupe de formule $-NR^6R^7$,

dans laquelle

$R^6$, $R^7$

- ont la définition indiquée ci-dessus,

$R^2$

- représente l'hydrogène, un groupe alkyle, alkoxy, alkylthio, chacun ayant jusqu'à 8 atomes de carbone, un groupe trifluorométhyle, trifluorométhoxy, hydroxy, mercapto, nitro, cyano, un halogène ou un groupe amino,

$R^3$

- représente l'hydrogène ou
- un groupe protégeant la fonction amino classique dans la chimie des β-lactames,

$R^4$

- représente l'hydrogène,
- un groupe protégeant la fonction carboxy classique dans la chimie des β-lactames, ou bien

un ester pouvant être scindé in vivo
et

$R^5$

- représente un halogène, un groupe cyano, trifluorométhyle, carboxy, alkoxycarbonyle ayant jusqu'à 8 atomes de carbone, alkylsulfonyle, alkylsulfonyloxy chacun avec jusqu'à 8 atomes de carbone, phénylsulfonyle, tolylsulfonyle, phénylsulfonyloxy, tolylsulfonyloxy, sulfamoyle, dialkylsulfamoyle, chacun avec jusqu'à 6 atomes de carbone par groupe alkyle, ou bien
- un groupe cycloalkyle ayant 3 à 8 atomes de carbone, qui peut être substitué par un radical alkyle, alkoxy, alkoxycarbonyle, chacun avec jusqu'à 6 atomes de carbone, un radical carboxy, carbamoyle, halogéno, cyano ou phényle, ou bien
- un groupe cycloalkylalkyle ayant 3 à 6 chaînons dans le noyau et 4 à 10 atomes de carbone, ou bien
- un groupe alcynyle à chaîne droite ou ramifiée ayant jusqu'à 8 atomes de carbone, qui peut être substitué par un radical aryle ayant 6 à 12 atomes de carbone, carboxy, alkoxycarbonyle ayant jusqu'à 8 atomes de carbone, ou par un halogène, ou bien
- un groupe aryle ayant 6 à 12 atomes de carbone, qui peut être substitué par un radical halogéno, cyano, nitro, trifluorométhyle, trifluorométhoxy, trifluorométhylthio, alkyle, alkoxy ou alkylthio, chacun avec jusqu'à 6 atomes de carbone, ou bien
- un reste hétérocyclique de la série thiényle, furyle, pyridyle, pyridyloxyde, pyrimidyle, pyrazinyle, pyridazinyle, pyrazolyle, imidazolyle, triazolyle, tétrazolyle, oxazolyle, thiazolyle ou thiadiazolyle, les restes hétérocycliques pouvant être substitués par un radical alkyle, alkoxy, chacun avec jusqu'à 6 atomes de carbone, halogéno, nitro ou cyano, ou bien
- un groupe de formule $-NR^8R^9$, $-CH_2R^{10}$ ou $-S-R^{11}$,

dans laquelle

$R^8$, $R^9$

- sont identiques ou différents et représentent
- l'hydrogène, un groupe alkyle ayant jusqu'à 6 atomes de carbone, aryle de 6 à 12 atomes de carbone, aralkyle de 7 à 14 atomes de carbone, acyle ayant jusqu'à 7 atomes de carbone ou un groupe protégeant la fonction amino, ou dans laquelle

$R^8$, $R^9$

- forment conjointement avec l'atome d'azote un noyau pentagonal à heptagonal qui peut être interrompu par O, S, NH, N-alkyle avec jusqu'à 4 atomes de carbone ou N-phényle,

$R^{10}$

- est un reste hétérocyclique de la série thiényle, furyle, pyridyle, pyridyloxyde, pyrimidyle, pyrazinyle, pyridazinyle, pyrazolyle, imidazolyle, triazolyle, tétrazolyle, oxazolyle, thiazolyle ou thiadiazolyle, les restes hétérocycliques pouvant être substitués par un radical alkyle, alkoxy, alkylthio, chacun avec jusqu'à 6 atomes de carbone, un radical halogéno, nitro, cyano, trifluorométhyle, trifluorométhoxy,

et

$R^{11}$

- représente un reste hétérocyclique de la série thiényle, furyle, pyridyle, pyridyloxyde, pyrimidyle, imidazolyle, triazolyle, oxazolyle ou thiadiazolyle, les restes hétérocycliques pouvant être substitués par un radical alkyle, alkoxy, chacun allant jusqu'à 6 atomes de carbone, halogéno, trifluorométhyle, trifluorométhoxy, nitro ou cyano,

et leurs sels.

**2.** Composés de formule générale (I) suivant la revendication 1, dans laquelle

$R^1$

- représente l'hydrogène ou
- un groupe alkyle à chaîne droite, à chaîne ramifiée ou cyclique, qui peut être substitué par du fluor, du chlore, du brome, un radical hydroxy, alkoxy ou cyano ou par un groupe de formule

$-NR^6R^7$,

dans laquelle

$R^6$, $R^7$

- sont identiques ou différents et représentent
- l'hydrogène, un groupe alkyle ayant jusqu'à 6 atomes de carbone, phényle, benzyle, ou un groupe protégeant la fonction amino, de la série 4-méthoxyphényle, 3,4-diméthoxyphényle, 4-méthoxyméthyloxyphényle, 4-[(2-méthoxyéthoxy)-méthyloxy]phényle, benzyle, 4-méthoxybenzyle, 2,4-diméthoxybenzyle, 3,4-diméthoxybenzyle, 2,4,6-triméthoxybenzyle, méthoxy-(4-méthoxyphényl)méthyle, triméthylsilyle, triéthylsilyle, tertio-butyl-diméthyl-silyle, tertio-butyl-diphénylsilyle, méthoxycarbonylméthyle, tertio-butoxycarbonylméthyle ou 2-hydroxy-2-phényléthyle,

ou bien

- un groupe phényle, éventuellement substitué par un radical fluoro, chloro, bromo, alkyle, alkoxy ayant jusqu'à 6 atomes de carbone, trifluorométhyle, trifluorométhoxy ou cyano, ou bien
- le fluor, le chlore, le brome, un groupe alkoxy, alkylthio, alkylsulfonyle, chacun avec jusqu'à 6 atomes de carbone, un groupe mercapto, hydroxy, phénylthio, phényloxy, sulfamoyle, ou bien
- un groupe de formule $-NR^6R^7$,

dans laquelle

$R^6$, $R^7$

- ont la définition indiquée ci-dessus,

$R^2$

- représente l'hydrogène, un groupe méthyle, méthoxy, trifluorométhyle, trifluorométhoxy, cyano, le fluor, le chlore ou le brome,

$R^3$

- représente l'hydrogène ou
- un groupe protégeant la fonction amino, de la série : 4-méthoxyphényle, 3,4-diméthoxyphényle, 4-méthoxyméthyloxyphényle, 4-[(2-méthoxyéthoxy)méthyloxy]-phényle, benzyle, 4-méthoxybenzyle, 2,4-diméthoxybenzyle, 3,4-diméthoxybenzyle, 2,4,6-triméthoxybenzyle, méthoxy-(4-méthoxyphényl)méthyle, triméthylsilyle, triéthylsilyle, tertio-butyl-diméthylsilyle, tertio-butyl-diphénylsilyle, méthoxycarbonylméthyle, tertio-butoxycarbonylméthyle, 2-hydroxy-2-phényléthyle, tertio-butyloxycarbonyle, 1-méthyl-2-benzoyl-vinyle ou 1-méthyl-2-méthoxy-vinyle,

$R^4$

- est l'hydrogène ou
- un groupe méthyle, éthyle, tertio-butyle, 2-chloréthyle, 2,2,2-trichloréthyle, cyanéthyle, diphénylméthyle, triphénylméthyle, acétoxyméthyle, allyle, benzyle, 4-méthoxybenzyle, 2,4-diméthoxybenzyle, 1-phénoxyéthyle, 2-méthyl-2-propényle, 4-nitrobenzyle, 2-nitrobenzyle, triméthylsilyléthyle ou tertio-butyl-diméthylsilyléthyle, ou bien

- un reste de formule

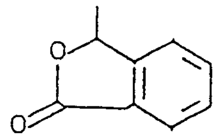 , 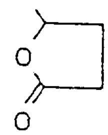 , 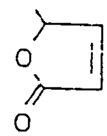 ,

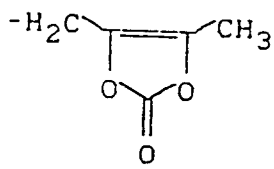 ,

$-CH_2-OCO-C(CH_3)_3$, $-CH(CH_3)-OCOOC_2H_5$ ou $-CH_2-OCOCH_3$ et

$R^5$

- représente le fluor, le chlore, le brome, un groupe cyano, trifluorométhyle, carboxy, alkoxycarbonyle ayant jusqu'à 6 atomes de carbone, alkylsulfonyle, alkylsulfonyloxy avec chacun jusqu'à 6 atomes de carbone, phénylsulfonyle, tolylsulfonyle, phénylsulfonyle, tolylsulfonyloxy ou bien
- les groupes cyclopropyle, cyclopentyle ou cyclohexyle qui peuvent être substitués par un radical alkyle, alkoxy, alkoxycarbonyle avec chacun jusqu'à 4 atomes de carbone, fluoro, chloro, bromo ou phényle, ou bien
- un groupe cyclopropylméthyle, cyclopentylméthyle ou cyclohexylméthyle, ou bien
- un groupe alcynyle à chaîne droite ou ramifiée ayant jusqu'à 6 atomes de carbone, qui peut être substitué par un radical phényle, carboxy, alkoxycarbonyle avec jusqu'à 6 atomes de carbone ou par du fluor, du chlore, du brome, ou bien
- un groupe phényle qui peut être substitué par du fluor, du chlore, du brome, un radical cyano, nitro, trifluorométhyle, trifluorométhoxy, alkyle ou alkoxy avec jusqu'à 4 atomes de carbone, ou bien
- un reste hétérocyclique de la série thiényle, furyle, pyridyle, pyridyloxyde, pyrimidyle, pyrazolyle, triazolyle, tétrazolyle, oxazolyle, thiazolyle ou thiadiazolyle, les restes hétérocycliques pouvant être substitués par un radical alkyle, alkoxy, avec chacun jusqu'à 4 atomes de carbone, fluoro, chloro, bromo ou nitro, ou bien
- un groupe de formule $-NR^8R^9$, $-CH_2-R^{10}$
  ou
  $-S-R^{11}$,

dans laquelle

$R^8$, $R^9$

- sont identiques ou différents et représentent
- l'hydrogène, un groupe alkyle ayant jusqu'à 4 atomes de carbone, phényle, benzyle ou un groupe protégeant la fonction amino, de la série 4-méthoxyphényle, 3,4-diméthoxyphényle, 4-méthoxyméthyloxyphényle, 4-[(2-méthoxyéthoxy)méthyloxy]-phényle, benzyle, 4-méthoxybenzyle, 2,4-diméthoxybenzyle, 3,4-diméthoxybenzyle, 2,4,6-triméthoxybenzyle, méthoxy(4-méthoxyphényl)méthyle, triméthylsilyle, triéthylsilyle, tertio-butyldiméthylsilyle, tertiobutyldiphénylsilyle, méthoxycarbonylméthyle, tertio-butoxycarbonylméthyle ou 2-hydroxy-2-phényléthyle, ou dans laquelle

$R^8$, $R^9$

- forment, conjointement avec l'atome d'azote, un noyau de pyrrolidine, de pipéridine, de morpholine, de thiomorpholine, de pipérazine, de 4-méthyl- ou de 4-phénylpipérazine,

$R^{10}$

- représente un noyau hétérocyclique de la série thiényle, furyle, pyridyle, pyridyloxyde, pyrimidyle, pyrazolyle, triazolyle, tétrazolyle, oxazolyle, thiazolyle ou thiadiazolyle, les restes hétérocycliques pouvant être substitués par un radical alkyle, alkoxy, chacun avec jusqu'à 4 atomes de carbone, fluoro, chloro, bromo ou nitro,

et

$R^{11}$

- est un noyau hétérocyclique de la série thiényle, furyle, pyridyle, pyridyloxyde, pyrimidyle, pyrazolyle, oxazolyle ou thiadiazolyle, les restes hétérocycliques pouvant être substitués par un radical alkyle, alkoxy, chacun avec jusqu'à 4 atomes de carbone, fluoro, chloro, bromo ou nitro,

et leurs sels.

**3.** Composés de formule générale (I) suivant la revendication 1, dans laquelle

$R^1$ est l'hydrogène ou le groupe cyclopropyle, ou bien
- un groupe alkyle à chaîne droite ou ramifié ayant jusqu'à 6 atomes de carbone, qui peut être substitué par un radical fluoro, chloro, méthoxy, cyano, diméthylamino ou diéthylamino, ou bien
- un groupe phényle qui peut être substitué par un radical fluoro, chloro, méthyle, méthoxy, trifluorométhyle, amino ou trifluorométhoxy, ou bien
- du chlore, un groupe alkoxy, alkylsulfonyle avec chacun jusqu'à 4 atomes de carbone, mercapto, hydroxy ou un groupe de formule $-NHR^6$,

dans laquelle

$R^6$

- représente l'hydrogène, un groupe alkyle ayant jusqu'à 4 atomes de carbone, phényle, benzyle ou un groupe protégeant la fonction amino, de la série 4-méthoxyphényle, 4-méthoxybenzyle, 2,4-diméthoxybenzyle, 3,4-diméthoxybenzyle ou tertio-butyl-diméthylsilyle,

$R^2$

- représente l'hydrogène

$R^3$

- représente l'hydrogène ou
- un groupe 4-méthoxyphényle, 4-méthoxybenzyle, 2,4-diméthoxybenzyle, 3,4-diméthoxybenzyle, tertio-butyldiméthylsilyle, 1-méthyl-2-méthoxy-vinyle (MMV) ou tertio-butyloxycarbonyle (Boc), ou bien

$R^4$

- représente l'hydrogène, ou bien
- un groupe méthyle, éthyle, tertio-butyle, diphénylméthyle, 2,2,2-trichloréthyle, allyle, acétoxyméthyle, 4-nitrobenzyle, 2-nitrobenzyle, 4-méthoxybenzyle, benzyle ou triméthylsilyléthyle, ou bien
- un reste de formule

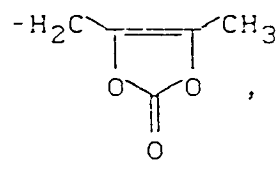,

$-CH(CH_3)-OCOOC_2H_5$ ou
$-CH_2-OCO-C(CH_3)_3$

et

$R^5$

- représente du chlore, du brome, un groupe trifluorométhyle, carboxy, alkoxycarbonyle, alkylsulfonyloxy, chacun avec jusqu'à 4 atomes de carbone, phénylsulfonyloxy, tolylsulfonyloxy, cyclopropyle, cyclopentyle, cyclohexyle, cyclopropylméthyle, cyclopentylméthyle, ou bien

- un groupe alcynyle ayant jusqu'à 4 atomes de carbone, qui peut être substitué par un radical phényle, carboxy ou alkoxycarbonyle avec jusqu'à 4 atomes de carbone, ou bien
- un groupe phényle, ou bien
- un reste de formule

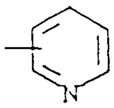 , 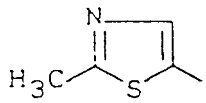 , 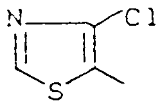 , 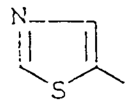 ,

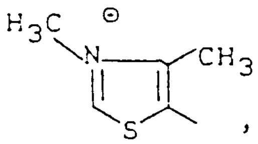 , 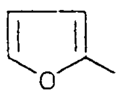 , 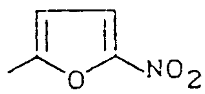 , 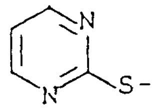 ,

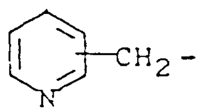 , 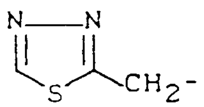 , 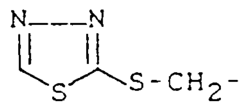 ,

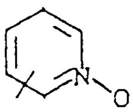 , 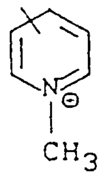 , 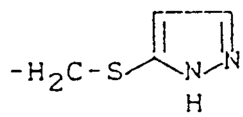

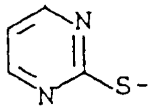 ou 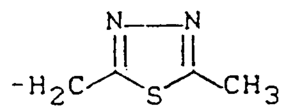

et leurs sels.

**4.** Procédé de production de composés de $\beta$-lactame de formule générale (I)

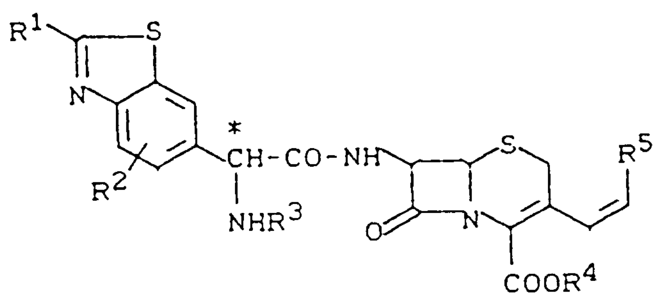 (I),

suivant la revendication 1, et de leurs sels, caractérisé en ce qu'on fait réagir

[A] des composés substitués de céphalosphorine de formule générale (II)

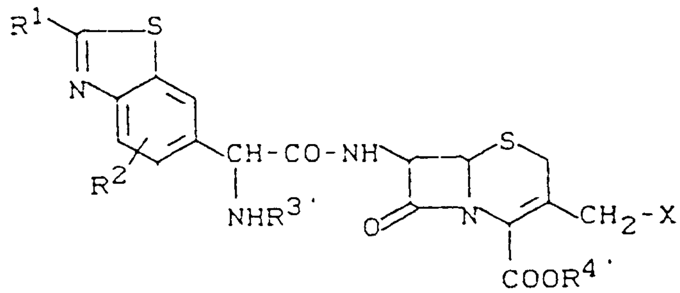

(II)

dans laquelle

$R^1$ et $R^2$ ont la définition indiquée ci-dessus,

$R^{3'}$ est un groupe protégeant la fonction amino classique dans la chimie des $\beta$-lactames,

$R^4$ est un groupe protégeant la fonction carboxy classique dans la chimie des $\beta$-lactames

et

X

- est un groupe de formule

$-\overset{\ominus}{P}(R^{17})_3Z^{\ominus}$ , 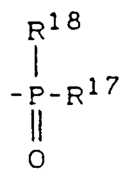 ou 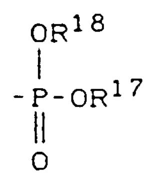

où

$R^{17}$ et $R^{18}$ sont identiques ou différents et représentent un groupe alkyle, phényle ou tolyle, et

$Z^{\ominus}$

- est un anion halogénure, de préférence chlorure, bromure ou iodure,

dans des solvants inertes en présence de bases, avec des aldéhydes de formule générale (III)

$R^5$-CHO (III)

dans laquelle

$R^5$ a la définition indiquée ci-dessus, ou bien

[B] des composés de phosphonium de formule générale (IV)

$R^5$-$CH_2$-X (IV)

dans laquelle

$R_5$ a la définition indiquée ci-dessus et

X

- est un groupe de formule

$-\overset{\ominus}{P}(R^{17})_3Z^{\ominus}$ , 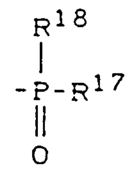 ou 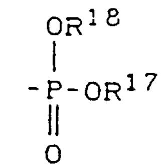

où

R¹⁷ et R¹⁸ sont identiques ou différents et représentent un groupe alkyle, phényle ou tolyle, et

$Z^{\ominus}$ est un anion halogénure, de préférence chlorure, bromure ou iodure,

dans des solvants inertes, en présence de bases, avec des aldéhydes de céphalosporine de formule générale (V)

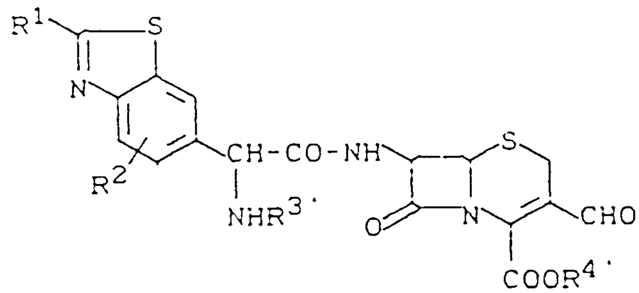

(V)

dans laquelle

$R^1$ et $R^2$ ont la définition indiquée ci-dessus,

$R^{3'}$ représente un groupe protégeant la fonction amino classique dans la chimie des β-lactames,

$R^4$ est un groupe protégeant la fonction carboxy classique dans la chimie des β-lactames,

ou bien

[C] des acides carboxyliques de formule générale (VI)

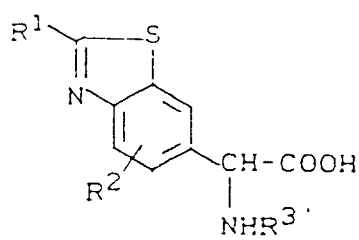

(VI)

dans laquelle

$R^1$ et $R^2$ ont la définition indiquée ci-dessus, et

$R^{3'}$ est un groupe protégeant la fonction amino classique dans la chimie des β-lactames,

après activation du groupe carboxyle par transformation en un anhydride mixte, par exemple avec l'ester éthylique d'acide chloroformique ou l'ester isobutylique d'acide chloroformique ou le chlorure d'acide méthanesulfonique, ou par transformation en l'halogénure d'acide, ou par transformation en un ester activé, par exemple avec le dicyclohexylcarbodiimide (DCC), le cas échéant en présence de N-hydroxybenzotriazole, avec des amines de vinylcéphalosporine de formule générale (VII)

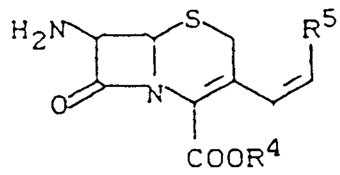

(VII),

dans laquelle

$R^4$ et $R^5$ ont la définition indiquée ci-dessus,

puis on élimine éventuellement les groupes protecteurs et on prépare les sels désirés, ou les acides libres à partir des sels.

**5.** Composés de $\beta$-lactame de formule générale (I) suivant la revendication 1 et leurs sels, destinés à être utilisés dans un procédé de traitement thérapeutique du corps humain ou animal.

**6.** Utilisation de composés de formule (I) suivant la revendication 1 pour la préparation de médicaments.

**7.** Médicaments contenant des composés de $\beta$-lactame de formule générale (I) suivant la revendication 1 et leurs sels.

**8.** Aliments pour le bétail et additifs pour aliments de ce type, contenant des composés de formule (I) suivant la revendication 1.

**Revendications pour les Etats contractants suivants : ES, GR**

**1.** Procédé de production de composés de $\beta$-lactame de formule générale (I)

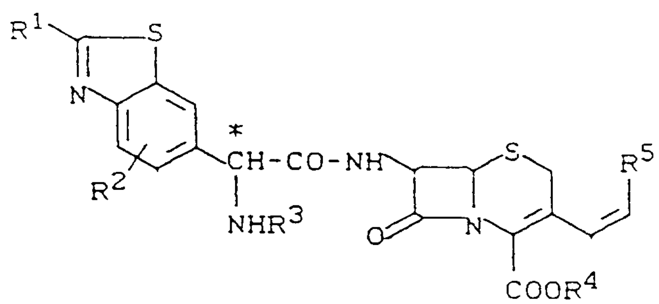

(I),

dans laquelle

$R^1$

- représente l'hydrogène ou
- un groupe alkyle ou alcényle à chaîne droite, à chaîne ramifiée ou cyclique ayant jusqu'à 10 atomes de carbone, qui peut être substitué par un halogène, un radical hydroxy, alkoxy ayant jusqu'à 6 atomes de carbone, cyano, sulfo, alkylsulfonyle ayant jusqu'à 6 atomes de carbone ou par un groupe de formule $-NR^6R^7$,

dans laquelle

$R^6$, $R^7$

- ont identiques ou différents et représentent
- l'hydrogène, un groupe alkyle ayant jusqu'à 8 atomes de carbone, aryle de 6 à 12 atomes de carbone, aralkyle de 7 à 14 atomes de carbone, acyle ayant jusqu'à 7 atomes de carbone ou un groupe protégeant la fonction amino,

  ou bien
- un groupe aryle ayant 6 à 12 atomes de carbone, qui peut être substitué par un radical halogéno, cyano, nitro, alkyle, alkoxy, alkylthio, chacun ayant jusqu'à 6 atomes de carbone, un radical trifluorométhyle ou trifluorométhoxy, ou bien

- un halogène, un groupe alkoxy, alkylthio, alkylsulfonyle, chacun ayant jusqu'à 8 atomes de carbone, un radical mercapto, hydroxy, phénylthio, phényloxy, benzylthio, benzyloxy, sulfo, sulfamoyle, $-PO(OH)_2$, $-NHNH_2$, -NHOH, guanidino, amidino, hétérocyclyle, hétérocyclylthio ou hétérocyclyloxy avec des hétérocycles de la série pyrrolyle, pyrrolidinyle, pyrazolyle, imidazolyle, pyridyle, pyridazinyle, pyrimidyle, pyrazinyle, quinolyle, isoquinolyle, indolyle, quinoxazolyle, quinazolyle, pipéridinyle, morpholinyle, pipérazinyle, thiomorpholinyle, furyle, thiényle, oxazolyle, thiazolyle, isoxazolyle, thiadiazolyle, triazolyle ou tétrazolyle, qui peuvent être substitués quant à eux par un radical méthyle, méthoxy ou halogéno,
- ou bien un groupe de formule $-NR^6R^7$,

dans laquelle

$R^6$, $R^7$

- ont la définition indiquée ci-dessus,

$R^2$

- représente l'hydrogène, un groupe alkyle, alkoxy, alkylthio, chacun ayant jusqu'à 8 atomes de carbone, un groupe trifluorométhyle, trifluorométhoxy, hydroxy, mercapto, nitro, cyano, un halogène ou un groupe amino,

$R^3$

- représente l'hydrogène ou
- un groupe protégeant la fonction amino classique dans la chimie des $\beta$-lactames,

$R^4$

- représente l'hydrogène,
- un groupe protégeant la fonction carboxy classique dans la chimie des $\beta$-lactames, ou bien

un ester pouvant être scindé in vivo

et

$R^5$

- représente un halogène, un groupe cyano, trifluorométhyle, carboxy, alkoxycarbonyle ayant jusqu'à 8 atomes de carbone, alkylsulfonyle, alkylsulfonyloxy chacun avec jusqu'à 8 atomes de carbone, phénylsulfonyle, tolylsulfonyle, phénylsulfonyloxy, tolylsulfonyloxy, sulfamoyle, dialkylsulfamoyle, chacun avec jusqu'à 6 atomes de carbone par groupe alkyle, ou bien
- un groupe cycloalkyle ayant 3 à 8 atomes de carbone, qui peut être substitué par un radical alkyle, alkoxy, alkoxycarbonyle, chacun avec jusqu'à 6 atomes de carbone, un radical carboxy, carbamoyle, halogéno, cyano ou phényle, ou bien
- un groupe cycloalkylalkyle ayant 3 à 6 chaînons dans le noyau et 4 à 10 atomes de carbone, ou bien
- un groupe alcynyle à chaîne droite ou ramifiée ayant jusqu'à 8 atomes de carbone, qui peut être substitué par un radical aryle ayant 6 à 12 atomes de carbone, carboxy, alkoxycarbonyle ayant jusqu'à 8 atomes de carbone, ou par un halogène, ou bien
- un groupe aryle ayant 6 à 12 atomes de carbone, qui peut être substitué par un radical halogéno, cyano, nitro, trifluorométhyle, trifluorométhoxy, trifluorométhylthio, alkyle, alkoxy ou alkylthio, chacun avec jusqu'à 6 atomes de carbone, ou bien
- un reste hétérocyclique de la série thiényle, furyle, pyridyle, pyridyloxyde, pyrimidyle, pyrazinyle, pyridazinyle, pyrazolyle, imidazolyle, triazolyle, tétrazolyle, oxazolyle, thiazolyle ou thiadiazolyle, les restes hétérocycliques pouvant être substitués par un radical alkyle, alkoxy, chacun avec jusqu'à 6 atomes de carbone, halogéno, nitro ou cyano, ou bien
- un groupe de formule $-NR^8R^9$, $-CH_2R^{10}$ ou $-S-R^{11}$,

dans laquelle

$R^8$, $R^9$

- sont identiques ou différents et représentent
- l'hydrogène, un groupe alkyle ayant jusqu'à 6 atomes de carbone, aryle de 6 à 12 atomes de carbone, aralkyle de 7 à 14 atomes de carbone, acyle ayant jusqu'à 7 atomes de carbone ou un groupe protégeant la fonction amino, ou dans laquelle

$R^8$, $R^9$

- forment conjointement avec l'atome d'azote un noyau pentagonal à heptagonal qui peut être interrompu par O, S, NH, N-alkyle avec jusqu'à 4 atomes de carbone ou N-phényle,

$R^{10}$

- est un reste hétérocyclique de la série thiényle, furyle, pyridyle, pyridyloxyde, pyrimidyle, pyrazinyle, pyridazinyle, pyrazolyle, imidazolyle, triazolyle, tétrazolyle, oxazolyle, thiazolyle ou thiadiazolyle, les restes hétérocycliques pouvant être substitués par un radical alkyle, alkoxy, alkylthio, chacun avec jusqu'à 6 atomes de carbone, un radical halogéno, nitro, cyano, trifluorométhyle, trifluorométhoxy,

et

$R^{11}$

- représente un reste hétérocyclique de la série thiényle, furyle, pyridyle, pyridyloxyde, pyrimidyle, imidazolyle, triazolyle, oxazolyle ou thiadiazolyle, les restes hétérocycliques pouvant être substitués par un radical alkyle, alkoxy, chacun allant jusqu'à 6 atomes de carbone, halogéno, trifluorométhyle, trifluorométhoxy, nitro ou cyano,

et de leurs sels,
caractérisé en ce qu'on fait réagir
[A] des composés substitués de céphalosporine de formule générale (II)

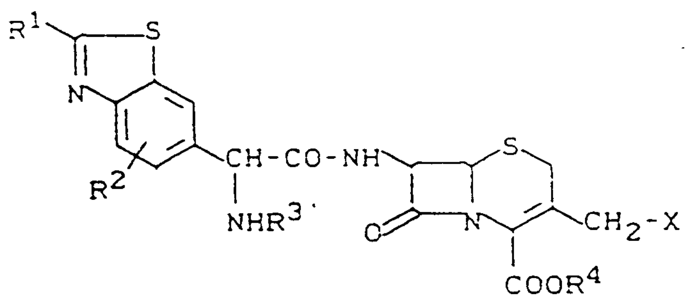

(II)

dans laquelle

- $R^1$ et $R^2$ ont la définition indiquée ci-dessus,
- $R^{3'}$ est un groupe protégeant la fonction amino classique dans la chimie des $\beta$-lactames,
- $R^4$ est un groupe protégeant la fonction carboxy classique dans la chimie des $\beta$-lactames et
- X
  - est un groupe de formule

$$-\overset{\ominus}{P}(R^{17})_3Z^{\ominus}$$ ,

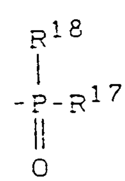

ou

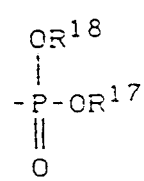

où

- $R^{17}$ et $R^{18}$ sont identiques ou différents et représentent un groupe alkyle, phényle ou tolyle, et
- $Z^{\ominus}$
  - est un anion halogénure, de préférence chlorure, bromure ou iodure,

dans des solvants inertes en présence de bases, avec des aldéhydes de formule générale (III)

$R^5$-CHO (III)

dans laquelle

$R^5$ a la définition indiquée ci-dessus, ou bien

[B] des composés de phosphonium de formule générale (IV)

$R^5$-$CH_2$-X (IV)

dans laquelle

$R_5$ a la définition indiquée ci-dessus et

X

- est un groupe de formule

$-\overset{\ominus}{P}(R^{17})_3Z^{\ominus}$ , 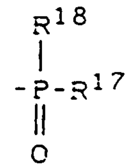 ou 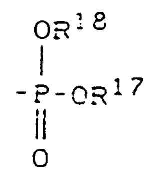

où

$R^{17}$ et $R^{18}$ sont identiques ou différents et représentent un groupe alkyle, phényle ou tolyle, et

$Z^{\ominus}$ est un anion halogénure, de préférence chlorure, bromure ou iodure,

dans des solvants inertes, en présence de bases, avec des aldéhydes de céphalosporine de formule générale (V)

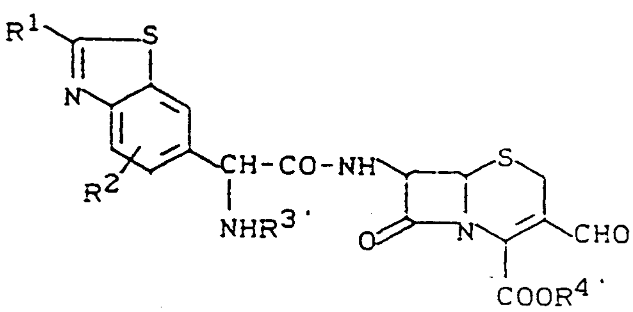

(V)

dans laquelle

$R^1$ et $R^2$ ont la définition indiquée ci-dessus,

$R^{3'}$ représente un groupe protégeant la fonction amino classique dans la chimie des $\beta$-lactames,

$R^4$ est un groupe protégeant la fonction carboxy classique dans la chimie des $\beta$-lactames,

ou bien

[C] des acides carboxyliques de formule générale (VI)

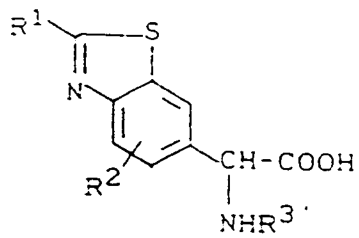

(VI)

dans laquelle

$R^1$ et $R^2$ ont la définition indiquée ci-dessus,

et

$R^{3'}$ est un groupe protégeant la fonction amino classique dans la chimie des β-lactames, après activation du groupe carboxyle par transformation en un anhydride mixte, par exemple avec l'ester éthylique d'acide chloroformique ou l'ester isobutylique d'acide chloroformique ou le chlorure d'acide méthanesulfonique, ou par transformation en l'halogénure d'acide, ou par transformation en un ester activé, par exemple avec le dicyclohexylcarbodiimide (DCC), le cas échéant en présence de N-hydroxybenzotriazole, avec des amines de vinylcéphalosporine de formule générale (VII)

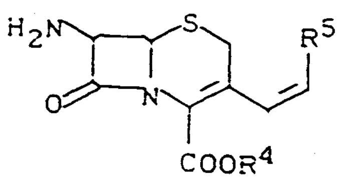

(VII),

dans laquelle

$R^4$ et $R^5$ ont la définition indiquée ci-dessus,

puis on élimine éventuellement les groupes protecteurs et on prépare les sels désirés, ou les acides libres à partir des sels.

**2.** Utilisation de composés de formule (I) suivant la revendication 1 pour la préparation de médicaments.